# EUROPEAN PATENT APPLICATION

(11) **EP 1 364 676 A1**
(43) Date of publication of application: **26.11.2003**
(21) Application number: 02711246.5
(22) Date of filing: 31.01.2002
(51) Int. Cl.: A61M 29/02, A61F 2/06

(54) **STENT**

(30) Priority: 01.02.2001 JP 2001026045; 01.02.2001 JP 2001026044; 13.02.2001 JP 2001034838; 21.05.2001 JP 2001151546; 25.05.2001 JP 2001157188; 19.09.2001 JP 2001285622
(71) Applicant: Kaneka Corporation, Osaka-shi, Osaka 530-0005 (JP)
(72) Inventor: NAKANO, Ryoji, Settsu-shi, Osaka 566-0072 (JP); MAEDA, Hiromi, Uji-shi, Kyoto 611-0011 (JP); MIKI, Shogo, Suita-shi, Osaka 565-0824 (JP)
(74) Representative: Gille Hrabal Struck Neidlein Prop Roos
(86) International application number: JP0200749
(87) International publication number: WO02060521

(57) **Abstract**

The present invention provides a stent which is flexible in the axial direction prior to the expansion of the stent, which shows no contraction in the axial length of the stent during expansion of the stent, which has an extremely large resistance to forces exerted by contracting blood vessels after expansion, which can cause uniform expansion of the struts of the stent, and in which there is no problem of the end portions of the stent warping to a greater diameter than the central portion of the stent at the time of expansion, furthermore a stent in which the size of the basic cells that constitute the stent is reduced so that bulging of endothelial cells of vascular tissues into the inside of the stent is suppressed, furthermore a stent which allows Y stenting into branched blood vessels while maintaining a high radial force and superior scaffold properties.

## Description

### TECHNICAL FIELD

The present invention relates to a stent which is used for placement or transplantation in vascular tissues inside the body; for example, a stent which is used for placement or transplantation in a blood vessel in order to prevent a constricted or blocked site in this blood vessel from becoming re-constricted after this site has been expanded.

### BACKGROUND ART

A stent is a therapeutic device which is placed in a blood vessel or other vascular cavity in the body in order expand a constricted or blocked site in this blood vessel or other vascular cavity, and to maintain the size of this vascular cavity, as a means of treating various disorders caused by such constriction or blockage. Such stents include coil-form stents formed from a metal or polymer material in the form of a single wire, stents that are formed by using a laser to cut out a metal tape, stents that are formed by using laser welding to assemble linear members, stents that are formed by weaving together a plurality of linear metal members, and the like. Such stents are disclosed in Japanese Patent KOKAI Publication No. H8-332230.

Such stents may be classified as stents that are expanded by a balloon on which the stent is mounted, and stents that expand themselves when a member that restricts expansion from the outside is removed. Among these stents, stents that are expanded by means of a balloon are used with the expansion pressure adjusted according to the state of the vascular tissue that is to be expanded, and the mechanical strength of the stent.

In recent years, such stents have been especially widely used in blood vessel shaping techniques for the heart and cervical arteries. Such stents are packaged as products by fixing (pre-mounting) the stent in a state in which the stent is contracted in a balloon catheter (stent delivery catheter). At the time of actual use, the stent delivery catheter is introduced as far as the constricted site of the blood vessel with the stent in the pre-mounted state, and the stent is expanded and placed in the constricted site of the blood vessel by expanding the balloon. Then, the stent delivery catheter is pulled out of the body. Such pre-mounting is disclosed in Japanese Patent KOKAI Publication No. 2000-189520 and the like.

The linear elements that constitute the smallest units that make up a stent are called struts. Stent designs can be broadly classified into two types, i. e., open type and closed type, according to the pattern formed by these struts.

In the open type, as is disclosed in Japanese Patent No. 2645203, a plurality of single segments in which a wave (sine wave) form is repeated in the circumferential direction are repeated in the axial direction, and these segments are connected by connecting segments in one to three places around the circumference.

On the other hand, in the closed type, as is disclosed in Japanese Patent KOKOKU Publication No. S40-6377, Japanese Patent KOKAI Publication No. H10-137345 and Japanese Patent KOHYO Publication No. H10-503676, polygonal shapes are formed (closed) by struts, these polygonal shapes have sides in common, and the polygonal shapes are repeated in the circumferential direction and axial direction. In some stents, the flexibility of the stent is improved by locally forming small curved parts or wave-form parts called flexible links (such as those indicated in the abovementioned laid-open patents) among the abovementioned polygonal shapes.

A stent in which the constituent elements have connected lozenge shapes following expansion is described in Japanese Patent KOKOKU Publication No. H4-6377. This stent offers the advantage of having an extremely large resistance to forces that tend to cause a contraction of the blood vessel. However, this stent lacks flexibility in the axial direction when not expanded; accordingly, it is extremely difficult to insert this stent into bent blood vessels, and there is a possibility that the interior of the blood vessel will be damaged. Furthermore, since this stent lacks flexibility in the axial direction following expansion as well, the following problem has been encountered: namely, in cases where the stent is placed in a bent blood vessel, an excessive stimulus is applied to the blood vessel so that re-constriction is promoted. Furthermore, when the stent is expanded, the length of the stent in the axial direction contracts, so that there are problems such as difficulty of expanding the entire constriction of the blood vessel, difficulty in positioning, and the like.

Furthermore, a stent in which a wire is formed in a zigzag shape, and this wire is further wrapped into a spiral shape so that a cylindrical shape is formed is described in Japanese Patent KOKOKU Publication No. H7-24688. This stent has abundant flexibility in the axial direction, and is superior in terms of insertability into bent blood vessels. However, this stent has an extremely small resistance to forces that tent to cause contraction of the blood vessel, so that any pressure that causes the blood vessel to contract tends to cause contraction of the stent.

Generally, furthermore, when conventional stents are expanded to the desired diameter, it is difficult to cause uniform expansion of the struts of the stent, so that portions that are greatly expanded and portions that are not expanded to a great extent are formed even within the same circumference. When such non-uniform expansion occurs, endothelial tissue of the vascular tissue may bulge through the widely opened portions of the struts, and thus become a cause of re-constriction. Furthermore, in cases where the non-uniform expansion is severe, it may become impossible to maintain a true circle in cross section. Methods of folding the balloon on which the stent is mounted have been devised in order to solve this problem; nevertheless, it is difficult to achieve sufficiently uniform expansion. In other methods, devices such as pasting a member that tends to undergo uniform expansion to the surface of the balloon have been tried; in such cases, however, the profile of the balloon is increased, and it becomes difficult to deliver the stent to the desired site.

Furthermore, in most stents that are expanded by means of a balloon, both end portions of the stent are warped to a larger diameter than the central portion when the stent is expanded, so that the problem of locally excessive expansion arises. If both end portions of the stent are excessively expanded, the endothelial cells of the vascular tissues in these areas are stimulated, so that re-constriction may be caused by cell multiplication in some cases.

Furthermore, in conventional stents, the gap parts formed between the stent struts at the time of expansion are generally large, so that the endothelial cells of the vascular tissues may bulge to a considerably extent through these gap parts, thus causing re-constriction. The reason for this is that the size of the basic cells that make up the stent is large. It is necessary to reduce the width of the struts in order to reduce this size; if this is done, however, the radial force that is obtained, i. e., the force that can withstand stress in the direction of diameter that is received from the outer circumference, is reduced.

Next, the advantages and disadvantages of the abovementioned open type and closed type stents will be described.

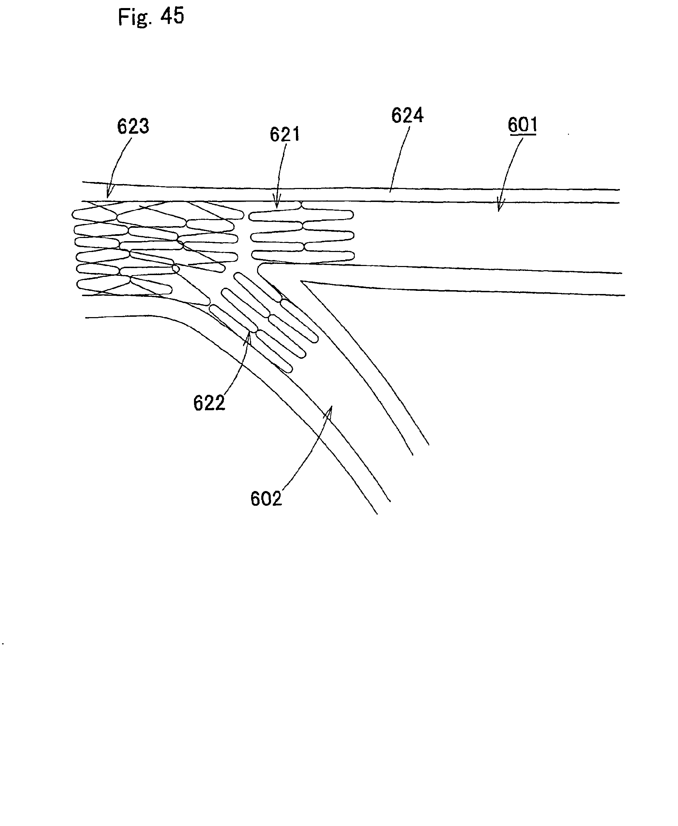

The first advantage of an open type stent is that such a stent has abundant flexibility in the axial direction, and is superior in terms of insertability into bent blood vessels. Furthermore, another great advantage is that a Y stent technique for insertion into branched blood vessels is possible. Such a "Y stent" or "Y stenting" technique is a technique in which one stent is placed in both the main blood vessel and the side-branching blood vessel in cases where there is a constriction in the branched area. Recently, thee has been an increase in the use of this technique, and such performance characteristics have become important among the performance characteristics required in stents. This technique is described in detail in "PTCA Techniques, Toshiaki Mitsufuji", 22. Branched Part Stents (page 203). Specifically, as is shown in Fig. 45, a first stent 621 is placed so that this stent extends from the proximal portion 623 of the main blood vessel 1 to the distal portion 624 of the main blood vessel 601, and a second stent 622 is placed so that this stent extends from the proximal portion 623 of the main blood vessel 601 to the side branch 602. Accordingly, the two stents overlap in the proximal portion 623 of the main blood vessel 601. In this technique, the first stent is first placed in the main blood vessel; then, the delivery catheter on which the second stent is pre-mounted is delivered toward the side branch by being passed through an opening part (gap) between the struts of the first stent, and the balloon is expanded in a state in which this balloon is positioned in an area extending from the proximal portion of the main blood vessel, through the opening part (gap) between the struts of the first stent, and into the entry portion of the side branch. As a result, the stent is placed in an area that extends from the proximal portion of the main blood vessel, through the opening part (gap) between the struts of the first stent, and into the entry portion of the side branch.

The reason for this is as follows: in the case of an open type stent, connecting segments are present in only one to three locations around the circumference, so that the peripheral length of the gap parts formed between the struts is long. Accordingly, when the balloon of the stent delivery catheter is passed through the gap part between the struts and expanded, these struts are deformed, and the gap part between the struts is simultaneously deformed, so that the opening area is increased; consequently, access to the side branch becomes possible.

However, the following problem has been encountered as a disadvantage: namely, the radial force is extremely small, so that the blood vessel is easily contracted by a pressure that tends to cause contraction. Furthermore, since the length of the circumferential portions of the gap parts formed between the abovementioned struts is great, the gap part positioned on the outside of the bend is widely opened when the stent is expanded and placed in a bent blood vessel, so that the endothelial tissue of the blood vessel may bulge considerably into the interior of the stent, thus causing re-constriction. The performance value that indicates how small the opening area of this gap part is is referred to as "scaffold properties".

Meanwhile, as for the advantages of closed type stents, such stents are advantageous in that the struts form polygonal shapes, and these polygonal shapes are repeated in the circumferential direction and axial direction while sharing sides in common, so that the abovementioned radial force is extremely large. At the same time, since polygonal shapes with a limited peripheral length are present, even if the stent is disposed in a bent blood vessel, the polygonal shape constituting the gap part that is positioned on the outside of the bend does not open beyond the area of this polygonal shape; accordingly, the abovementioned scaffold properties are good.

However, such stents suffer from the following disadvantage: namely, Y stents cannot be inserted into the abovementioned branched blood vessels. Specifically, when a Y stent is installed, even if the balloon of another stent delivery catheter is passed through the polygonal shape constituting the gap part between the struts of the closed type stent, and this balloon is expanded, the polygonal shape does not open any further, since the peripheral length of this polygonal shape is limited; accordingly, access to the side branch is impossible. It might be thought that this problem could be solved by increasing the area o the polygonal shapes; if this is done, however, the scaffold properties are sacrificed in the same manner as in open type stents.

### DISCLOSURE OF THE INVENTION

In order to achieve the abovementioned object, the first invention of the present application provides a stent which is flexible in the axial direction prior to the expansion of the stent, which shows no contraction in the axial length of the stent when the stent is expanded, which has an extremely large resistance to forces exerted by contracting blood vessel following expansion, which makes it possible to expand the struts of the stent in a uniform manner, and in which there is no problem of the two end portions of the stent warping to a greater diameter than the central portion when the stent is expanded.

The present invention provides a stent which is formed as a substantially tubular body, and which can be expanded outward in the radial direction of this substantially tubular body, this stent being characterized in that the stent 101 comprises circumferentially extendable substantially wave-form constituent elements 102 and axially extendable substantially wave-form constituent elements 103; a plurality of the circumferentially extendable substantially wave-form constituent elements 102 are disposed substantially in the circumferential direction of the stent 101 without being directly connected to each other; a plurality of the axially extendable substantially wave-form constituent elements 103 are disposed substantially in the circumferential direction of the stent 101 without being directly connected to each other; and these constituent elements 102 and 103 are arranged alternately in periodic series in the axial direction of the stent.

Furthermore, the present invention also provides a stent which is formed as a substantially tubular body, and which can be expanded outward in the radial direction of this substantially tubular body, wherein the stent 101 comprises circumferentially extendable substantially wave-form constituent elements 102 and axially extendable substantially wave-form constituent elements 103; a connecting part 127 on one end of the circumferentially extendable substantially wave-form constituent element 102 is connected with a connecting part 131 on one end of the axially extendable substantially wave-form constituent element 103, and a connecting part 139 on the remaining end of the axially extendable substantially wave-form constituent element 103 is connected with a connecting part 121 on the opposite end from the connecting part 127 of another the circumferentially extendable substantially wave-form constituent element 102, so that the circumferentially extendable substantially wave-form constituent elements 102 and the axially extendable substantially wave-form constituent elements 103 are connected periodically; and, furthermore, a connecting part 123 on a peak or valley protruding part of the circumferentially extendable substantially wave-form constituent element 102 is connected with a connecting part 131 on one end of the axially extendable substantially wave-form constituent element 103, and a connecting part 139 on the remaining end of the axially extendable substantially wave-form constituent element 103 is connected with a connecting part 125 on the peak or valley protruding part present on the opposite side from the connecting part 123 of another the circumferentially extendable substantially wave-form constituent elements 102, whereby the circumferentially extendable substantially wave-form constituent elements 102 and the axially extendable substantially wave-form constituent elements 103 are formed in periodic series.

As a result of combining circumferentially extendable elements of the stent and axially extendable elements, the stent 101 makes it possible to reduced the contraction of the stent in the axial direction when the stent is expanded. Furthermore, as a result of the appropriate disposition of the substantially wave-form constituent elements, the stent is flexible in the axial direction, expands uniformly at the time of expansion, and shows a large resistance force to forces that tend to cause contraction of the blood vessel, so that the abovementioned object is achieved.

Furthermore, the present invention also provides a stent of the abovementioned type which is characterized in that the stent is formed with a plurality of directly connected circumferentially extendable substantially wave-form constituent elements 102 arranged along the circumference of the stent only at the axially opposite ends of the stent 101. As a result, the opposite ends of the stent show an increased resistance to forces that tend to cause contraction of the blood vessel compared to the central portion of the stent, and the warping of both end portions of the stent to a greater diameter than the central portion at the time of expansion is reduced, so that the abovementioned object is achieved.

Furthermore, the present invention provides a stent 201 which is formed as a substantially tubular body, and which can be expanded outward in the radial direction of this substantially tubular body, this stent being characterized in that the stent 201 comprises circumferentially extendable substantially wave-form constituent elements 202, circumferentially extendable substantially wave-form constituent elements 203 and axially extendable substantially wave-form constituent elements 204; a plurality of the circumferentially extendable substantially wave-form constituent elements 202 are disposed substantially in the circumferential direction of the stent without being directly connected to each other, a plurality of the circumferentially extendable substantially wave-form constituent elements 203 are disposed substantially in the circumferential direction of the stent without being directly connected to each other, a plurality of the axially extendable substantially wave-form constituent elements 204 are disposed substantially in the circumferential direction of the stent without being directly connected to each other, and these elements are arranged alternately in periodic series in the axial direction of the stent.

Furthermore, the present invention also provides a stent which is formed as a substantially tubular body, and which can be expanded outward in the radial direction of this substantially tubular body, wherein the stent comprises circumferentially extendable substantially wave-form constituent elements 202, circumferentially extendable substantially wave-form constituent elements 203, and axially extendable substantially wave-form constituent elements 204; a connecting part 227 on one end of the circumferentially extendable substantially wave-form constituent element 202 is connected with a connecting part 241 on one end of the axially extendable substantially wave-form constituent element 204, a connecting part 249 on the remaining end of the axially extendable substantially wave-form constituent element 204 is connected with a connecting part 233 on a peak or valley protruding part of the circumferentially extendable substantially wave-form constituent element 203, a connecting part 235 on a peak or valley protruding part present on the opposite side from the connecting part 233 of the circumferentially extendable substantially wave-form constituent element 203 is connected with the connecting part 241 on one end of another circumferentially extendable substantially wave-form constituent element 204, and the connecting part 249 on the remaining end of the axially extendable substantially wave-form constituent element 204 is connected with a connecting part 221 on the opposite end from the connecting part 227 of another circumferentially extendable substantially wave-form constituent element 202, so that the circumferentially extendable substantially wave-form constituent elements 202, the circumferentially extendable substantially wave-form constituent elements 203 and the axially extendable substantially wave-form constituent elements 204 are arranged in periodic series; furthermore, a connecting part 223 on a peak or valley protruding part of the circumferentially extendable substantially wave-form constituent element 202 is connected with the connecting part 241 on one end of the axially extendable substantially wave-form constituent elements 204, the connecting part 249 on the remaining end of the axially extendable substantially wave-form constituent element 204 is connected with a connecting part 237 on one end of the circumferentially extendable substantially wave-form constituent element 203, a connecting part 231 on the opposite end from the connecting part 237 of the circumferentially extendable substantially wave-form constituent element 203 is connected with the connecting part 241 on one end of another axially extendable substantially wave-form constituent element 204, and the connecting part 249 on the remaining end of the axially extendable substantially wave-form constituent element 204 is connected with a connecting part 225 on a peak or valley protruding part on the opposite side from the connecting part 223 of another circumferentially extendable substantially wave-form constituent elements 202, so that the circumferentially extendable substantially wave-form constituent elements 202, the circumferentially extendable substantially wave-form constituent elements 203 and the axially extendable substantially wave-form constituent elements 204 are arranged in periodic series; and whereby a stent is formed in which the circumferentially extendable substantially wave-form constituent elements 202, the circumferentially extendable substantially wave-form constituent elements 203 and the axially extendable substantially wave-form constituent elements 204 are arranged in periodic series.

As a result of using circumferentially extendable elements of the stent and axially extendable elements of the stent in combination, the abovementioned stent 201 makes it possible to reduce the contraction of the stent in the axial direction at the time of expansion. Furthermore, as a result of the appropriate disposition of substantially wave-form constituent elements, the stent is flexible in the axial direction, expands uniformly at the time of expansion, and shows a large resistance force to forces that tend to cause contraction of the blood vessel, so that the abovementioned object is achieved.

Furthermore, the present invention also provides the abovementioned stent which is characterized in that only the axially opposite ends of the stent 201 are formed by arranging along the circumference of the stent a plurality of directly connected circumferentially extendable substantially wave-form constituent elements 202 or 203 (or both). As a result, the opposite ends of the stent have a larger resistance to forces that tend to cause contraction of the blood vessel than the central portion of the stent. Furthermore, the warping of both end portions of the stent to a greater diameter than the central portion during expansion is reduced, so that the abovementioned object is achieved.

The present invention also provides a stent which is formed as a substantially tubular body, and which can be expanded in the radial direction from the inside of the substantially tubular body, this stent being characterized in that the basic elements that constitute the abovementioned stent 301, 303 or 305 are substantially shaped as parallelograms.

Furthermore, the present invention provides a stent which is characterized in that substantially parallelogram-shaped elements 302, 304 and 306 are combined together in an alternately oriented fashion.

Furthermore, the present invention provides a stent which is characterized in that substantially parallelogram-shaped elements 302, 304, 306 are combined together in an alternately oriented fashion, the respective sides of the substantially parallelogram-shaped elements are substantially parallel to the axial direction of the stent prior to the expansion of the stent, and the respective sides of the substantially parallelogram-shaped elements 302, 304, 306 form an angle with respect to the axial direction of the stent following the expansion of the stent.

'Furthermore, the present invention provides a stent in which the substantially parallelogram-shaped elements 304 and 306 are constructed from substantially linear struts 321 and 331 and substantially wave-form struts 322 and 332.

Furthermore, the present invention provides a stent in which substantially parallelogram-shaped elements with different areas are disposed in the axial direction of the stent.

Furthermore, the present invention provides a stent in which different strut widths or different strut thicknesses, or both, are combined in the axial direction of the stent.

The present invention provides a stent which is formed as a substantially tubular body, and which can be expanded outward in the radial direction of this substantially tubular body, characterized in that: the stent 401, 402 comprises annular first substantially wave-form elements 411, 451 that can be expanded in the radial direction, link part elements 413, 453 that can be extended in the axial direction, and branch-form elements 412, 452 that extend from the first substantially wave-form elements 411, 451; one end of the link part element 413, 453 is connected to the first substantially wave-form element 411, 451, the other end of the link part element 413, 453 is connected to one end of the branch-form element 412, 452, and the other end of the branch-form element 412, 452 is connected to the first substantially wave-form element 411, 451.

The present invention provides a stent which is formed as a substantially tubular body, and which can be expanded outward in the radial direction of the substantially tubular body, this stent being characterized in that the abovementioned stent 403 comprises annular first substantially wave-form elements 481 which can be expanded in the radial direction, link part elements 483 which can be extended in the axial direction, branch-form elements 482 which extend from the abovementioned first substantially wave-form elements 481, and substantially N-shaped elements 485.

The present invention provides a stent in which both end portions of the stent comprise annular second substantially wave-form elements 414, 454, 484 that can be expanded in the radial direction.

The present invention provides a stent which can be uniformly expanded in substantially the same shape except for both end portions.

Next, the second invention of the present application provides a stent which is uniformly expanded and in which excessive expansion is suppressed, while at the same time the size of the basic cells that constitute the stent is reduced so that the bulging of endothelial cells of vascular tissues into the interior of the stent is suppressed.

The present invention provides a stent 501, 502 or 503 for placement in vascular tissues inside body cavities, which is formed as a substantially tubular body, and which can be expanded outward in the radial direction of the substantially tubular body, this stent being characterized in that the stent has a structure that prevents excessive expansion to a diameter greater than the desired diameter.

Furthermore, the present invention also provides the abovementioned stent 501, 502 or 503, which has a structure in which the basic cells 511 that constitute the stent comprise main struts 514 which are disposed so that the length of these struts is oriented in the axial direction of the stent when the stent has not yet been expanded, and sub-struts 515 which are folded between these main struts 514, and which support the main struts 514 in the circumferential direction when the stent is expanded, the main struts 514 and sub-struts 515 form annular substantially polygonal shapes that comprise three or more sides when the stent is expanded, a plurality of these basic cells 511 are connected in the circumferential direction so that band parts 512 are formed, and a plurality of these band parts 512 are connected in the longitudinal direction via link parts 513.

Furthermore, it is desirable that the stent 501, 502 or 503 of the present invention satisfy the relationships π × D = 0.5 × A × sin θ × B and 60° ≤ θ < 90°, where A is the overall length of the sub-struts 515 in one basic cell 511 folded between the main struts 514, B is the number of basic cells 511 within one band part 512 formed by the series of a plurality of basic cells 511 in the circumferential direction, and D is the desired expanded diameter of the stent, and that the stent further satisfy the relationship L ≤ A < 2 × L, here L is the length of the main struts 514 (in the longitudinal direction) within the basic cells 511 when the stent has not yet been expanded. Furthermore, it is even more desirable that the stent satisfy the relationship 0.5 × W ≤ T ≤ 3 × W, where W is the width of the wire material that constitutes the main struts 514 and sub-struts 515, and T is the thickness of this wire material.

In the abovementioned stent 501, 502 or 503, it is desirable that the shape of the basic cells 511 at the time of expansion of the stent be substantially triangular, substantially square or substantially trapezoidal, that the link parts 513 that connect the band parts 512 formed by a plurality of these basic cells 511 being connected in the circumferential direction have a structure that allows expansion and contraction in the longitudinal direction, and that this stent satisfy the relationship 0.3 × L ≤ C ≤ 2L, where C is the length of the link parts in the axial direction of the stent when the stent has not yet been expanded.

In the abovementioned stents 501, 502 and 503, it is desirable that at least the main struts 514 and sub-struts 515 comprise one or more materials selected from a set comprising stainless steel, super-elastic metals, polymer materials with a bending elastic modulus of 1 GPa or greater, and biodegradable polymer materials.

Furthermore, the stents 501, 502 and 503 of the present invention are also provided as structures in which a tubular thin polymer film is formed on the outer circumferential surface of the abovementioned stents.

Furthermore, it is desirable that the abovementioned stents 501, 502 and 503 have an X-ray-impermeable marker that allows confirmation of the position of the stent in X-ray imaging. Furthermore, a drug or therapeutic gene which is used to prevent re-constriction or to suppress the formation of thrombi may be added or applied as a surface treatment.

Furthermore, the third invention of the present application provides a closed type stent which eliminates the disadvantages of a closed type stent while retaining the advantages of such a stent, and which at the same time retains only the advantages of an open type stent without suffering from any of the disadvantages of such an open type stent. In more concrete terms, the third invention makes it possible to install a Y stent in branched blood vessels (the impossibility of such installation being a shortcoming of closed types stents) while maintaining the high radial force and superior scaffold properties that are the advantages of closed type stents.

The present invention is a closed type stent 603. Here, more or less polygonal shape patterns 605 which are surrounded by struts 604 constituting linear elements are endowed with a linear peripheral length and opening part area that are required in order to maintain superior scaffold properties and a high radial force. At the same time, a relatively large number of local folded portions 607 that can undergo elongating deformation are disposed in the abovementioned polygonal shape patterns 605, so that the opening parts of the abovementioned polygonal shape patterns can be spread (caused to undergo an expanding deformation) to a size that is sufficient to allow access to side branches by pushing and spreading the abovementioned polygonal shape patterns 605 from the inside toward the outside.

In other words, the stent 603 of the present invention is a stent which is formed as a substantially tubular body, and which can be expanded outward in the radial direction of the substantially tubular body, wherein a plurality of patterns 605 with more or less polygonal shapes surrounded by struts 604 constituting linear elements are lined up in the circumferential direction and axial direction, this stent being characterized in that the abovementioned polygonal shape patterns 605 have a linear peripheral length 606, 609 (original linear peripheral length), and these polygonal shape patterns 605 have three or more local folded portions 607, 610 per polygonal shape pattern 605, which are capable of elongating deformation so that the peripheral length 608 following expansion by pushing is extended to 1.3 to 2.0 times the original linear peripheral length 606, 609. Furthermore, it is desirable that the number of local folded parts 607, 610 per polygonal shape pattern 605 that are capable of elongating deformation be the same as the number of sides of the abovementioned polygons.

Furthermore, the present invention provides a stent 603 which is formed as a substantially tubular body, and which can be expanded outward in the radial direction of the substantially tubular body, wherein a plurality of patterns 605 with more or less polygonal shapes surrounded by struts 604 constituting linear elements are lined up in the circumferential direction and axial direction, this stent being characterized in that the abovementioned polygonal shape patterns 605 have a linear peripheral length 606, 609, the abovementioned polygonal shape patterns 605 have parts 607, 610 that are capable of elongating deformation in the polygonal shape patterns 605 so that the peripheral length of the polygonal shape patterns 605 can be expanded to a value that is 1.3 times to 2.0 times the abovementioned linear peripheral length 606, 609 by pushing the polygonal shape patterns open from the inside toward the outside, and these parts that are capable of elongating deformation are formed so that the total of the linear lengths of these parts that are capable of elongating deformation in the directions of the sides of the abovementioned polygonal shapes extends from 1/3 to 1 time the linear length of the abovementioned polygonal shape patterns 605.

As a result of having a plurality of polygonal shape patterns 605 in the circumferential direction and axial direction, the abovementioned stent 603 can maintain a high radial force, and at the same time has superior scaffold properties. Furthermore, since local folded parts 607, 610 that are capable of elongating deformation are present in relatively large numbers in the respective polygonal shape patterns 605 as described above, the peripheral length of the polygonal shape patterns 605 can be greatly deformed by expanding the balloon of another stent delivery catheter placed in the side branch via these polygonal shape patterns 605; accordingly, access to side-branch vessels and Y stenting are possible.

In the techniques described in Japanese Patent KOKAI Publication No. H10-137345 and Japanese Patent KOHYO Publication No. H10-503676, two U-shaped parts are present facing each other in the lozenge-shaped parts formed by the struts. However, these parts are used to endow the stent with flexibility in the axial direction; these U-shaped parts are not parts that are caused to undergo elongating deformation in order to allow Y stenting, and there is no description of any such action. In actuality, furthermore, in a case where there are only two U-shaped parts per lozenge shape as described in the patent specifications, a sufficient opening area to allow side branch access cannot be obtained by pushing the shape open by the expansion of the balloon of the abovementioned stent delivery catheter.

### BRIEF DESCRIPTION OF THE DRAWINGS

Fig. 1 is a development view which shows a stent 101 constituting a first embodiment of the first invention of the present application;
Fig. 2 is an explanatory diagram which shows one of the circumferentially extendable substantially wave-form constituent elements 102 of the stent 101 shown in Fig. 1;
Fig. 3 is an explanatory diagram which shows one of the axially extendable substantially wave-form constituent elements 103 of the stent 101 shown in Fig. 1;
Fig. 4 is a development view of the stent 101 shown in Fig. 1 prior to expansion;
Fig. 5 is a development view of the stent 101 showing a modification of the first embodiment;
Fig. 6 is a development view which shows a stent 201 constituting a second embodiment of the first invention of the present application;
Fig. 7 is an explanatory diagram which shows one of the circumferentially extendable substantially wave-form constituent elements 202 of the stent 201 shown in Fig. 6;
Fig. 8 is an explanatory diagram which shows one of the circumferentially extendable substantially wave-form constituent elements 203 of the stent 201 shown in Fig. 6;
Fig. 9 is an explanatory diagram which shows one of the axially extendable substantially wave-form constituent elements constituent elements 204 of the stent 201 shown in Fig. 6;
Fig. 10 is a development view of the stent 201 shown in Fig. 6 prior to expansion;
Fig. 11 is a development view of the stent 201 shown in Fig. 6 following expansion;
Fig. 12 is a development view of the stent 201 showing a modification of the second embodiment;
Fig. 13 is a development view which shows a stent 301 constituting a third embodiment of the first invention of the present application;
Fig. 14 is a development view of a stent 303 which shows a modification of the third embodiment;
Fig. 15 is a development view of a stent 305 following expansion of the stent, which shows another modification of the third embodiment;
Fig. 16 is a development view of the stent 305 shown in Fig. 15 prior to expansion;
Fig. 17 is a development view which shows a stent 401 constituting a fourth embodiment of the first invention of the present application;
Fig. 18 is a development view of the central portion of the stent 401 shown in Fig. 17;
Fig. 19 is a development view of both end portions of the stent 401 shown in Fig. 17;
Fig. 20 is development view of the stent 401 shown in Fig. 17 prior to expansion;
Fig. 21 is a development view of a stent 402 which shows a modification of the fourth embodiment;
Fig. 22 is a development view of the central portion of the stent 402 shown in Fig. 21;
Fig. 23 is a development view of both end portions of the stent 402 shown in Fig. 21;
Fig. 24 is a development view of a stent 403 which shows another modification of the fourth embodiment;
Fig. 25 is a development view of the central portion of the stent 403 shown in Fig. 24;
Fig. 26 is a development view of both end portions of the stent 3 shown in Fig. 24;
Fig. 27 is a development view which shows a stent 501 constituting an embodiment of the second invention of the present application;
Fig. 28 is a partial enlarged view which shows a basic cell of the stent 501 shown in Fig. 27 prior to expansion;
Fig. 29 is a partial enlarged view which shows a basic cell of the stent 501 shown in Fig. 27 following expansion;
Fig. 30 is an explanatory diagram which shows the struts of the basic cell of the stent 501 shown in Fig. 27 prior to expansion, as modeled by line segments;
Fig. 31 is a development view of the stent 501 shown in Fig. 27 following expansion;
Fig. 32 is a partially cut-away enlarged perspective view of the stent 501 shown in Fig. 27;
Fig. 33 is a partial enlarged view which shows an example of a variation in the shape of the portion where plastic deformation occurs during the expansion of the stent;
Fig. 34 is an enlarged sectional view of the portion with a varied shape shown in Fig. 33;
Fig. 35 is a development view of a stent 502 constituting another embodiment of the second invention prior to expansion;
Fig. 36 is a development view of a stent 503 constituting still another embodiment of the second invention prior to expansion;
Fig. 37 is a development view of the stent 502 shown in Fig. 35 following expansion;
Fig. 38 is a development view of the stent 503 shown in Fig. 36 following expansion;
Fig. 39 is a partial enlarged view which shows one example of the link part 513 of the stent of the second invention;
Fig. 40 is a partial enlarged view which shows the link part 513 of the stent of the second invention, and shows an example in which this link part is endowed with flexibility with respect to forces that are perpendicular to the direction of length;
Fig. 41 is a partial enlarged view which shows the link part 513 of the stent of the second invention, and shows another example in which this link part is endowed with flexibility with respect to forces that are perpendicular to the direction of length;
Fig. 42 is a partial enlarged view which shows the link part 513 of the stent of the second invention, and shows another example in which this link part is endowed with flexibility with respect to forces that are perpendicular to the direction of length;
Fig. 43 is a partial enlarged view which shows the link part 513 of the stent of the second invention, and shows another example in which this link part is endowed with flexibility with respect to forces that are perpendicular to the direction of length;
Fig. 44 is a partial enlarged view which shows the band part 512 and link part 513 of the stent 501 of the second invention;
Fig. 45 is an explanatory diagram which shows a Y stent or Y stenting;
Fig. 46 is a development view which shows an embodiment of the stent 603 o the third invention of the present application;
Fig. 47 is a partial enlarged view of a polygonal shape pattern 605 showing the original linear peripheral length 606 in the stent 603 shown in Fig. 46;
Fig. 48 is a development view which shows the peripheral length 608 after the opening parts of the specified polygonal shape pattern 605 have been pushed open;
Fig. 49 is a partial enlarge view of the polygonal shape pattern 605 of the stent 603 showing another embodiment of the third invention; and
Fig. 50 shows the embodiment shown in Fig. 49, and is a partial enlarged view of the polygonal shape pattern 605 showing the liner length in the direction of the side of the polygonal shape in a local portion that can be extended and deformed.

### BEST MODE FOR CARRYING OUT THE INVENTION

Embodiments of the stent of the present invention will be described below with reference to the attached figures; however, the present invention is not limited to these embodiments.

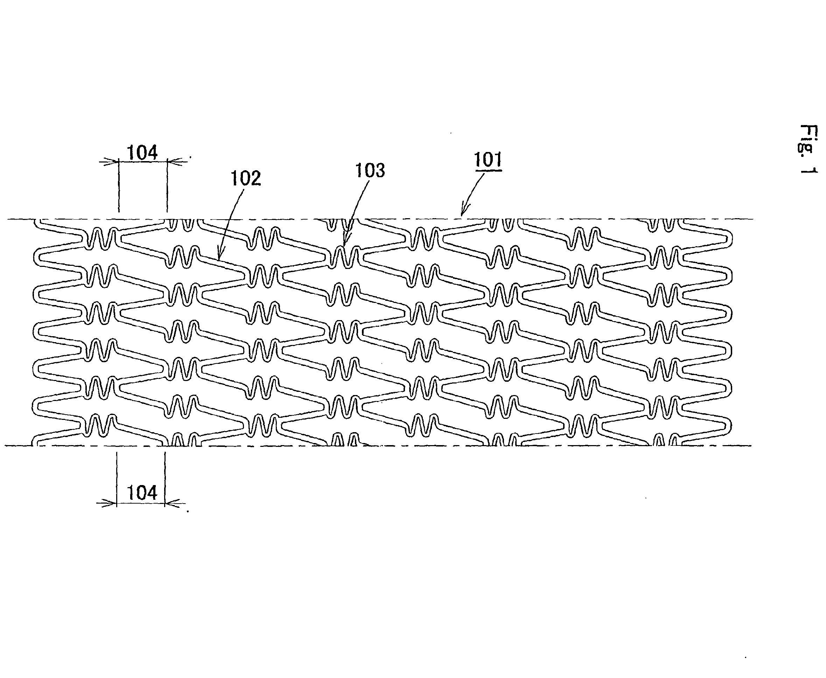

Figs. 1 through 5 show a stent constituting a first embodiment of the first invention of the present application. Fig. 1 is a development view of the stent 101. The stent 101 is a stent which is formed in a substantially tubular shape, and which can be expanded outward in the radial direction of this tubular shape. This stent comprises circumferentially extendable substantially wave-form constituent elements 102, and axially extendable substantially wave-form constituent elements 103. Three of the abovementioned circumferentially extendable substantially wave-form constituent elements 102 are disposed substantially in the circumferential direction of the stent without being directly connected to each other, and six of the abovementioned axially extendable substantially wave-form constituent elements 103 are disposed in the circumferential direction of the stent without being directly connected to each other. These elements are alternately and periodically connected to each other, thus forming the stent. The numbers of the abovementioned circumferentially extendable substantially wave-form constituent elements 102 and axially extendable substantially wave-form constituent elements 103 which are present in one circumference of the stent are determined in accordance with the length and external diameter of the stent that is manufactured; these numbers are not limited to three circumferentially extendable substantially wave-form constituent elements 102 and six axially extendable substantially wave-form constituent elements 103. As a result of using circumferentially extendable elements of the stent and axially extendable elements of the stent in combination, the stent 101 can be expanded outward in the radial direction, and can reduce contraction of the stent in the axial direction at the time of this expansion. Furthermore, as a result of the abovementioned substantially wave-form constituent elements 102 and circumferentially extendable substantially wave-form constituent elements 103 or axial direction being disposed substantially in the circumferential direction of the stent without being directly connected to each other, the stent can be endowed with flexibility.

Here, the term "circumferentially extendable constituent elements or axial direction" refers respectively to elements with a structure that allows elongation in the circumferential direction of the tubular stent, or in the axial direction (direction of length) of the stent. However, it is desirable that this structure be a structure that also allows contraction. For example, in cases where the stent is placed in a straight blood vessel, there is basically no problem in the case of deformation comprising only extension. However, in cases where the stent is placed in a bent blood vessel, an extra extension because of the disposition in the shape of the blood vessel is generated besides the extension generated at the time of expansion on the outside of the bent portion. In this case, if contractile deformation is possible on the inside of the bent portion, the excessive increase in the spacing between the stent struts caused by excessive extension on the outside of he bent blood vessel can be reduced. The substantially wave-form constituent elements 102 and circumferentially extendable substantially wave-form constituent elements 103 and axial direction shown in the stent 101 have respective structures that also allow contraction.

Furthermore, as long as the substantially wave-form constituent elements 102 and substantially wave-form constituent elements 103 have respective structures that allow extension in the circumferential direction and axial direction, these elements may have various types of shapes other than those shown in Fig. 1. For example, in the case of the circumferentially extendable substantially wave-form constituent elements 102, shape alterations such as adjustment of the angle or the like or formation of the entire element with a curved surface or the like, may be made in order to adjust the required dimension at the time of extension or the expanding force. However, it is desirable that the abovementioned circumferentially extendable substantially wave-form constituent elements 102 have a structure in which the combined number of peak and valley vertices is 2 or greater. If this number is smaller than 2, it is difficult to maintain the number of connection locations while obtaining a capacity for expansion. A structure with 4 peak and valley vertices is even more desirable. The circumferentially extendable substantially wave-form constituent elements 102 of the stent 101 shown in Fig. 1 have peak and valley vertices on the end portions, and the combined number of peak and valley vertices including the end portions is 4. Furthermore, in the case of the axially extendable substantially wave-form constituent elements 103, various shapes with respect to (for example) the number and angle of the bent locations and the like may be used in order to adjust the required dimension at the time of extension or the expanding force. However, it is desirable that the axially extendable substantially wave-form constituent elements 103 have a structure in which the combined number of peak and valley vertices is 1 or greater. If there is not even a single vertex, it is difficult to obtain a capacity for extension. A structure which has 2 or 4 vertices is preferable, and a structure which has 4 vertices is even more preferable. In the axially extendable substantially wave-form constituent elements 103 of the stent 101 shown in Fig. 1, the combined number of peaks and valleys is 4.

Furthermore, in the case of the circumferential elements 104 that are constructed by disposing a plurality of the abovementioned circumferentially extendable substantially wave-form constituent elements 102 (which are not directly connected to each other) substantially in the circumferential direction of the stent, it is desirable that the length of these elements 104 in the axial direction be short, since this allows smooth bending of the stent, thus preventing damage to the blood vessel walls, in cases where the stent is inserted into bent blood vessels. Preferably, the stent has 5 or more circumferential elements 104 per 10 mm of the stent.

If the numbers of the abovementioned circumferentially extendable substantially wave-form constituent elements 102 and axially extendable substantially wave-form constituent elements 103 (per single circumference) are small, a large blood vessel retaining force cannot be expected. Preferably, per single circumference, the number of the abovementioned circumferentially extendable substantially wave-form constituent elements 102 is 3 or greater, and the number of the abovementioned axially extendable substantially wave-form constituent elements 103 is 6 or greater. In this case, a high blood vessel retaining force can be manifested.

At the opposite ends of the stent 101, the abovementioned substantially wave-form constituent elements 102 that can be extended in the circumferential are directly and continuous connected, so that a substantially wave-form shape that can be extended in the circumferential is formed around the circumferential of the stent. As a result, both ends o the stent have a greater resistance to forces that tend to cause contraction of the blood vessel than the central portion of the stent, and the problem of both end portions o the stent warping to a greater diameter than the central portion of the stent when the stent is expanded does not arise.

Furthermore, the length (in the axial direction of the stent) of the circumferentially extendable substantially wave-form constituent elements 102 which make up the axially opposite ends of the stent may be set so that this length is shorter than the length (in the axial direction of the stent) of the substantially wave-form (circumferentially extendable) constituent elements 102 which make up the portions of the stent other than the axially opposite ends of the stent. In this case, the resistance to forces that tend to cause contraction of the blood vessel is even greater than in cases where such a structure is not used; furthermore, the problem of both end portions of the stent warping to a greater diameter than the central portion at the time of expansion does not occur.

Furthermore, it is also possible to make the width of the struts of the circumferentially extendable substantially wave-form constituent elements 102 wider only at the axially opposite ends of the stent (compared to locations other than the axially opposite ends of the stent). In this case, the resistance to forces that tend to cause contraction of the blood vessel is even greater than in cases where such a structure is not used; furthermore, the problem of both end portions of the stent warping to a greater diameter than the central portion at the time of expansion does not occur. Here, the term "strut" refers to the wire-form members that constitute the stent.

Furthermore, it is also possible to make the thickness of the struts of the circumferentially extendable substantially wave-form constituent elements 102 thicker only at the axially opposite ends of the stent (compared to locations other than the axially opposite ends of the stent). In this case, the resistance to forces that tend to cause contraction of the blood vessel is even greater than in cases where such a structure is not used; furthermore, the problem of both end portions of the stent warping to a greater diameter than the central portion at the time of expansion does not occur.

Furthermore, it is also possible to use a combined structure in which the length (with respect to the axial direction of the stent) of the circumferentially extendable substantially wave-form constituent elements 102 is made shorter, the width of the struts is made wider, and the thickness of the struts is made thicker only at the axially opposite ends of the stent compared to locations other than the axially opposite ends of the stent. Furthermore, it is also possible to make the length (with respect to the axial direction of the stent) of the circumferentially extendable substantially wave-form constituent elements 102 or the axially extendable substantially wave-form constituent elements 103, or both, shorter in steps as the position of these elements shifts from the central portion of the stent (with respect to the axial direction) to the end portions. In this case, the resistance to forces that tend to cause contraction of the blood vessel is even greater than in cases where such a structure is not used, and the problem of both end portions of the stent warping to a greater diameter than the central portion at the time of expansion does not occur; furthermore, the flexibility in the axial direction of the stent can be varied in steps without causing any great variation in this flexibility.

Furthermore, it is also possible to increase the width of the struts of the abovementioned circumferentially extendable substantially wave-form constituent elements 102 or the abovementioned axially extendable substantially wave-form constituent elements 103, or both, in steps as the positions of these elements shift from the central portion of the stent (with respect to the axial direction) to the end portions. In this case, the resistance to forces that tend to cause contraction of the blood vessel is even greater than in cases where such a structure is not used, and the problem of both end portions of the stent warping to a greater diameter than the central portion at the time of expansion does not occur; furthermore, the flexibility in the axial direction of the stent can be varied in steps without causing any great variation in this flexibility.

Furthermore, it is also possible to increase the thickness of the struts of the abovementioned circumferentially extendable substantially wave-form constituent elements 102 or the abovementioned axially extendable substantially wave-form constituent elements 103, or both, in steps as the positions of these elements shift from the central portion of the stent (with respect to the axial direction) to the end portions. In this case, the resistance to forces that tend to cause contraction of the blood vessel is even greater than in cases where such a structure is not used, and the problem of both end portions of the stent warping to a greater diameter than the central portion at the time of expansion does not occur; furthermore, the flexibility in the axial direction of the stent can be varied in steps without causing any great variation in this flexibility.

Furthermore, it is also possible to use a structure in which the length (in the axial direction of the stent) of the circumferentially extendable substantially wave-form constituent elements 102 or the axially extendable substantially wave-form constituent elements 103, or both, is increased in steps, the width of the struts is increased in steps and the thickness of the struts is increased in steps as the positions of these elements shift from the central portion of the stent (with respect to the axial direction) to both end portions of the stent.

The stent 101 shown in Fig. 1 has a construction in which 9 rows of the abovementioned circumferentially extendable substantially wave-form constituent elements 102 (including both end) and 8 rows of the abovementioned axially extendable substantially wave-form constituent elements 103 are alternately connected. These 9 rows of the abovementioned circumferentially extendable substantially wave-form constituent elements 102 (including both ends) and 8 rows of the abovementioned axially extendable substantially wave-form constituent elements 103 are determined in accordance with the length and external diameter of the stent that is manufactured; the number of the abovementioned circumferentially extendable substantially wave-form constituent elements 102 (including both ends) is not limited to 9 rows, and the number of the abovementioned axially extendable substantially wave-form constituent elements 103 is not limited to 8 rows.

As is shown in Fig. 1, the abovementioned circumferentially extendable substantially wave-form constituent elements 102 are lined up in the circumferential direction of the stent, and are not directly connected to each other. Furthermore, the abovementioned axially extendable substantially wave-form constituent elements 103 are also lined up in the circumferential direction of the stent, and are likewise not directly connected to each other.

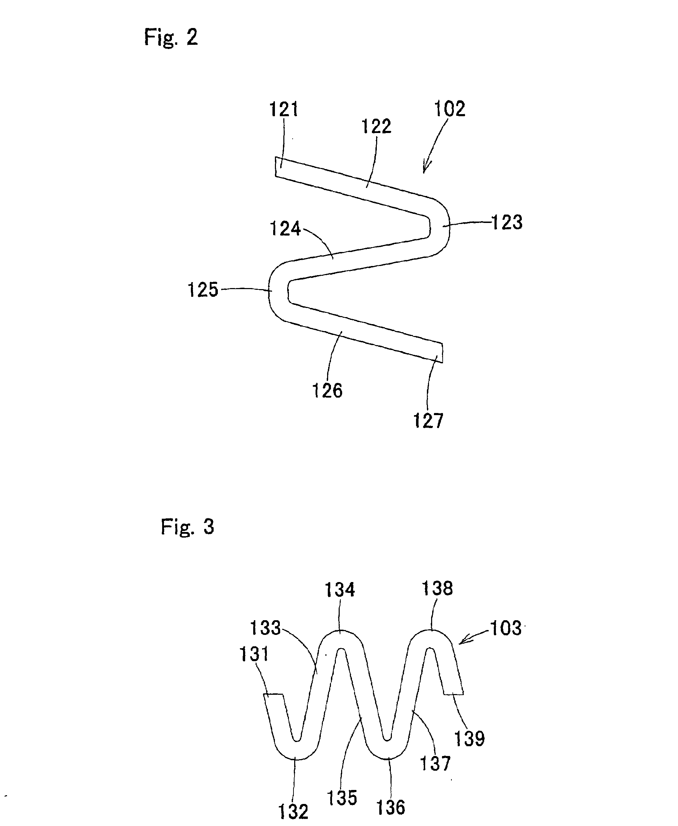

One aspect of the circumferentially extendable substantially wave-form constituent elements 102 is shown in Fig. 2, and one aspect of the axially extendable substantially wave-form constituent elements 103 is shown in Fig. 3. The abovementioned circumferentially extendable substantially wave-form constituent elements 102 are constructed from linear parts 122, 124, 126 and connecting parts 121, 123, 125, 127, and the abovementioned axially extendable substantially wave-form constituent elements 103 are constructed from linear parts 133, 135, 137, connecting parts 131, 139 and bent parts 132, 134, 136, 138. All of the connecting parts 121, 123, 125, 127 are respectively connected to one of the connecting parts 131 or 139; accordingly, when the stent is expanded, the force is uniformly transmitted to the circumferentially extendable substantially wave-form constituent elements 102 and the axially extendable substantially wave-form constituent elements 103, so that the stent struts can be uniformly expanded.

In regard to the linear parts 122, 124, 126 of the circumferentially extendable substantially wave-form constituent elements 102 and the linear parts 133, 135, 137 of the axially extendable substantially wave-form constituent elements 103, the flexibility in the axial direction of the stent is lost if the width and thickness of the struts are increased; conversely, the force that withstands radial stresses applied from the outer circumference is reduced if the width and thickness of the struts are reduced. Accordingly, in order to appropriately satisfy requirements for both flexibility in the axial direction of the stent and a force that can withstand radial stresses applied from the outer circumference, it is desirable that the linear parts 122, 124, 126 of the abovementioned circumferentially extendable substantially wave-form constituent elements 102 have a width of 80 µm to 150 µm and a thickness of 70 µm to 150 µm; furthermore, a width of 120 µm to 140 µm and a thickness of 100 µm to 120 µm are even more desirable. Furthermore, it is desirable that the linear parts 133, 135, 137 of the abovementioned axially extendable substantially wave-form constituent elements 103 have a width of 50 µm to 100 µm and a thickness of 50 µm to 150 µm; moreover, a width of 60 µm to 80 µm and a thickness of 80 µm to 120 µm are even more desirable. However, both the circumferentially extendable substantially wave-form constituent elements 102 and the substantially wave-form axially extendable constituent elements 103 can be adjusted to various sizes other than the abovementioned dimensions in accordance with the material that forms the stent and the positions in which the elements are used.

If the length (in the axial direction) of the abovementioned circumferentially extendable substantially wave-form constituent elements 102 is long, the stent cannot be smoothly bent, and the corners of the struts tend to stand out, when the stent is inserted into a bent blood vessel. Conversely, if the axial length of the abovementioned circumferentially extendable substantially wave-form constituent elements 102 is short, then the stent cannot be expanded to the required stent diameter at the time of expansion. Furthermore, if the axial length of the abovementioned axially extendable substantially wave-form constituent elements 103 is long, then the gaps that are formed between the stent struts at the time of expansion are large, so that endothelial cells of vascular tissues may bulge to considerable extent through these gap areas, thus causing re-constriction in some cases. Conversely, if the axial length of the abovementioned axially extendable substantially wave-form constituent elements 103 is short, then the flexibility of the stent in the axial direction is lost. Accordingly, it is desirable that the axial length of the abovementioned circumferentially extendable substantially wave-form constituent elements 102 be 0.8 mm to 1.8 mm, and a length of 1.0 mm to 1.4 mm is even more desirable. Furthermore, it is desirable that the axial length of the abovementioned axially extendable substantially wave-form constituent elements 103 be 0.5 mm to 1.5 mm, and a length of 0.7 mm to 1.0 mm is even more desirable. However, both the substantially wave-form constituent elements 102 and 103 an be adjusted to various sizes other than the abovementioned dimensions in accordance with the material that forms the stent and the position in which the stent is used.

The stent of the present invention can be manufactured using a metal which has an appropriate rigidity and elasticity such as stainless steel, an Ni-Ti alloy, a Cu-Al-Mn alloy or the like, or a polymer material which has an appropriate rigidity and elasticity.

The stent of the present invention may also be finished by plating the stent with a protective material, impregnating the stent with drugs, or covering the stent with materials.

Furthermore, laser working methods, discharge working methods, mechanical cutting methods, etching methods and the like can be used as stent forming methods.

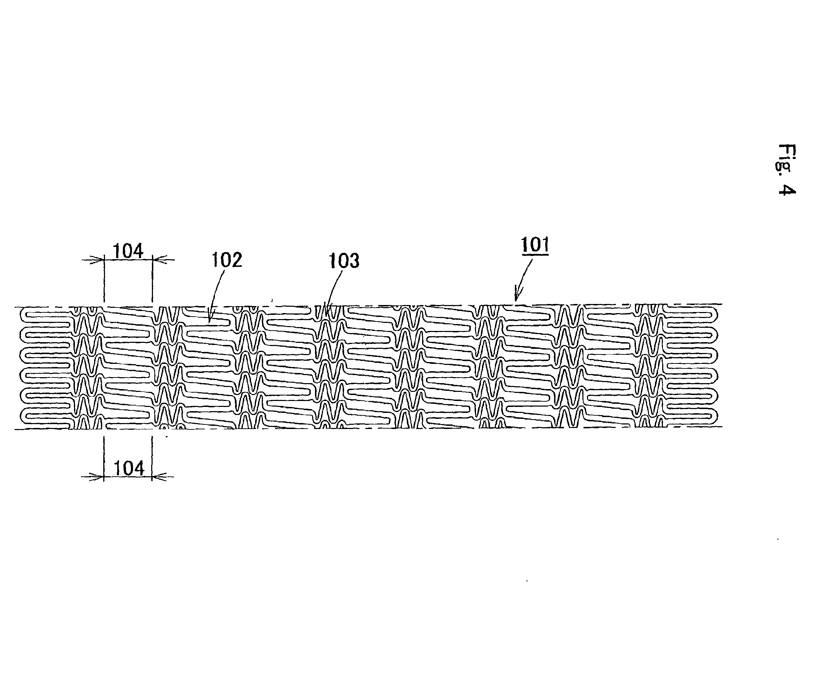

Fig. 4 shows a development view of the stent of the present invention mounted on a balloon catheter. As is shown in Fig. 4, even when the stent is mounted on a balloon catheter, the abovementioned circumferentially extendable substantially wave-form constituent elements 102 are lined up in the circumferential direction of the stent, the abovementioned axially extendable substantially wave-form constituent elements 103 are also lined up in the circumferential direction of the stent, and circumferential elements 104 in which the abovementioned circumferentially extendable substantially wave-form constituent elements 102 are lined up in the circumferential direction and circumferential elements in which the abovementioned axially extendable substantially wave-form constituent elements 103 are lined up in the circumferential direction are alternately connected in the axial direction of the stent. As result, when the stent is expanded, even if the circumferentially extendable substantially wave-form constituent elements 102 contract in the axial direction, the axially extendable substantially wave-form constituent elements 103 are expanded in the axial direction, so that the overall length of the stent can be maintained at the same length before and after expansion of the stent.

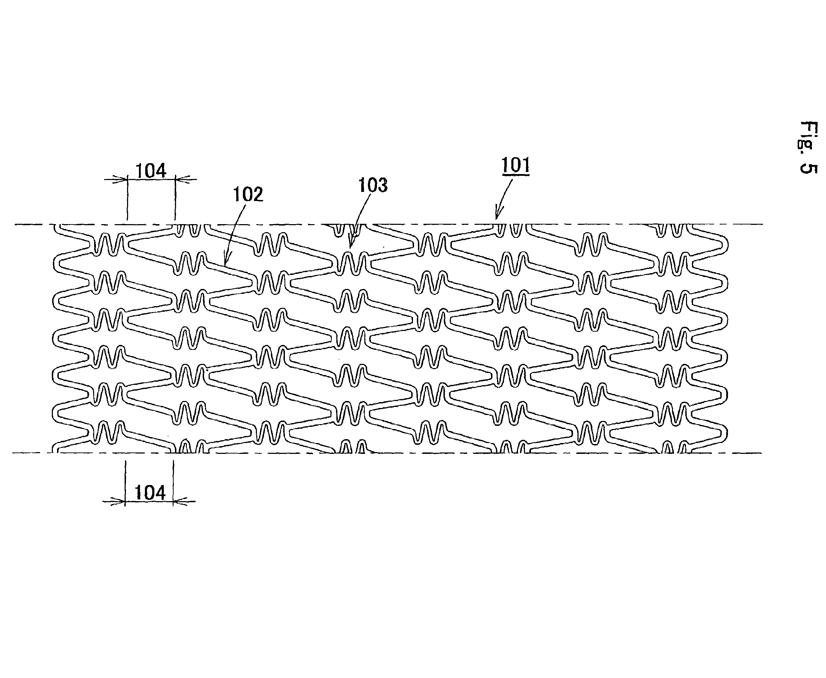

Fig. 5 shows a modification of the first embodiment of the first invention. Specifically, in this embodiment, only the two ends of the stent in the axial direction are formed by disposing in the circumferential direction of the stent a plurality of directly connected circumferentially extendable substantially wave-form constituent elements 102. Furthermore, the axial length of these circumferentially extendable substantially wave-form constituent elements 102 is shorter than the axial length of the substantially wave-form (circumferentially extendable) constituent elements 102 which make up the portions of the stent other than the axially opposite ends of the stent. As a result, the warping of the struts in the end portions of the stent can be reduced, and the resistance force against forces that tend to cause contraction of the blood vessel in both end portions can be increased. Furthermore, the resistance force against forces that tend to cause contraction of the blood vessel can be increased by increasing the strut width or thickness only in both end portions.

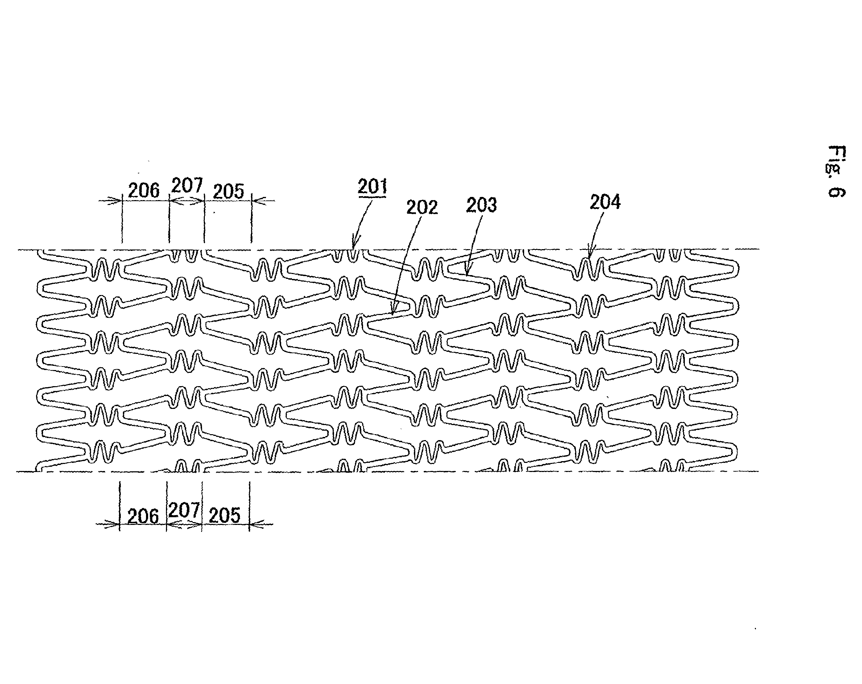

Figs. 6 through 12 show a stent constituting a second embodiment of the first invention. Fig. 6 is a development view of the stent 201 of the present invention. The stent 201 is a stent which is formed as a substantially tubular body, and which can be expanded outward in the radial direction of this tubular body. This stent comprises circumferentially extendable substantially wave-form constituent elements 202, circumferentially extendable substantially wave-form constituent elements 203, and axially extendable substantially wave-form constituent elements 204. Three of the abovementioned circumferentially extendable substantially wave-form constituent elements 202 are disposed substantially in the circumferential direction of the stent without being directly connected to each other, three of the abovementioned circumferentially extendable substantially wave-form constituent elements 203 are disposed substantially in the circumferential direction of the stent without being directly connected to each other, and six of the abovementioned axially extendable substantially wave-form constituent elements 204 are disposed in the circumferential direction of the stent without being directly connected to each other. These elements are alternately and periodically connected to each other in the axial direction of the stent, thus forming the stent. The numbers of the circumferentially extendable substantially wave-form constituent elements 202, circumferentially extendable substantially wave-form constituent elements 203 and abovementioned axially extendable substantially wave-form constituent elements 204 per single circumference of the stent are determined in accordance with the length and external diameter of the stent that is manufactured, and are not limited to three of the abovementioned substantially wave-form constituent elements 202, three of the abovementioned substantially wave-form constituent elements 203 and six of the abovementioned substantially wave-form constituent elements 204. As a result of having a combination of circumferentially extendable elements of the stent and axially extendable elements of the stent, the stent 201 can expand radially outward, and can reduce the contraction in the axial direction of the stent that occurs during this expansion. Furthermore, as a result of the abovementioned circumferentially extendable substantially wave-form constituent elements 202, 203 and 204 or axial direction being disposed substantially in the axial direction of the stent without being directly connected to each other, the stent can be endowed with flexibility.

Here, the term "circumferentially or axially extendable constituent elements" refers respectively to elements with a structure that allows elongation in the circumferential direction of the tubular stent, or in the axial direction (direction of length) of the stent. However, it is desirable that this structure be a structure that also allows contraction. For example, in cases where the stent is placed in a straight blood vessel, there is basically no problem in the case of deformation comprising only extension. However, in cases where the stent is placed in a bent blood vessel, an extra extension because of the disposition in the shape of the blood vessel is generated besides the extension generated at the time of expansion on the outside of the bent portion. In this case, if contractile deformation is possible on the inside of the bent portion, the excessive increase in the spacing between the stent struts caused by excessive extension on the outside of he bent blood vessel can be reduced. The substantially wave-form circumferentially and axially extendable constituent elements 202, 203 and 204 and shown in the stent 201 have respective structures that also allow contraction.

Furthermore, as long as the substantially wave-form constituent elements 202, 203 and 204 have respective structures that allow extension in the circumferential direction and axial direction, these elements may have various types of shapes other than those shown in Fig. 6. For example, in the case of the circumferentially extendable substantially wave-form constituent elements 202 and 203, shape alterations such as adjustment of the angle or the like or formation of the entire element with a curved surface or the like, may be made in order to adjust the required dimension at the time of extension or the expanding force. However, it is desirable that the abovementioned circumferentially extendable substantially wave-form constituent elements 202 and 203 have a structure in which the combined number of peak and valley vertices is 2 or greater. If this number is smaller than 2, it is difficult to maintain the number of connection locations while obtaining a capacity for expansion. A structure with 4 peak and valley vertices is even more desirable. The circumferentially extendable substantially wave-form constituent elements 202 and 203 of the stent 201 shown in Fig. 6 have peak and valley vertices on the end portions, and the combined number of peak and valley vertices including the end portions is 4. Furthermore, in the case of the axially extendable substantially wave-form constituent elements 204, various shapes with respect to (for example) the number and angle of the bent locations and the like may be used in order to adjust the required dimension at the time of extension or the expanding force. However, it is desirable that the axially extendable substantially wave-form constituent elements 204 have a structure in which the combined number of peak and valley vertices is 1 or greater. If there is not even a single vertex, it is difficult to obtain a capacity for extension. A structure which has 2 or 4 vertices is preferable, and a structure which has 4 vertices is even more preferable. In the axially extendable substantially wave-form constituent elements 204 of the stent 201 shown in Fig. 6, the combined number of peaks and valleys is 4.

Furthermore, in the stent 201 shown in Fig. 6, the substantially wave-form constituent elements 202 and circumferentially extendable substantially wave-form constituent elements 203 are in a relationship of shapes that show mutual linear symmetry. In the case of linear-symmetrical shapes, uniform expansion of the struts at the time of expansion of the stent is facilitated.

Furthermore, in the case of the circumferential elements 205 that are constructed by disposing a plurality of the abovementioned circumferentially extendable substantially wave-form constituent elements 202 (which are not directly connected to each other) substantially in the circumferential direction of the stent, and the circumferential elements 206 that are constructed by disposing a plurality of the abovementioned circumferentially extendable substantially wave-form constituent elements 203 (which are not directly connected to each other) substantially in the circumferential direction of the stent, it is desirable that the length of these elements 205 and 206 in the axial direction be short, since this allows smooth bending of the stent, thus preventing damage to the blood vessel walls, in cases where the stent is inserted into bent blood vessels. Preferably, the stent has 5 or more circumferential elements 205 and 206 per 10 mm of the stent.

If the numbers (per single circumference of the stent) the abovementioned circumferentially extendable substantially wave-form constituent elements 202 and 203 and the substantially wave-form axially extendable constituent elements 204 are small, a large blood vessel retaining force cannot be expected. Preferably, per single circumference, the number of the abovementioned circumferentially extendable substantially wave-form constituent elements 202 or circumferentially extendable substantially wave-form constituent elements 203 is 3 or greater, and the number of the abovementioned axially extendable substantially wave-form constituent elements 204 is 6 or greater. In this case, a high blood vessel retaining force can be manifested.

At the opposite ends of the stent 201, the circumferentially extendable substantially wave-form constituent elements 202 or the circumferentially extendable substantially wave-form constituent elements 203 are directly and continuously connected, so that a circumferentially extendable substantially wave-form shape is formed around the circumference of the stent. As a result, both ends of the stent have a greater resistance to forces that tend to cause contraction of the blood vessel than the central portion of the stent; furthermore, the problem of both end portions of the stent warping to a greater diameter than the central portion at the time of expansion does not occur.

Furthermore, the length in the axial direction of the stent) of the substantially wave-form (circumferentially extendable) constituent elements 202 or 203 which make up the axially opposite ends of the stent may be set so that this length is shorter than the length (in the axial direction of the stent) of the substantially wave-form (circumferentially extendable). constituent elements 202 or 203 which make up the portions of the stent other than the axially opposite ends of the stent. In this case, the resistance to forces that tend to cause contraction of the blood vessel is even greater than in cases where such a structure is not used; furthermore, the problem of both end portions of the stent warping to a greater diameter than the central portion at the time of expansion does not occur.

Furthermore, it would also be possible to increase the thickness of the struts of the circumferentially extendable substantially wave-form constituent elements 202 or 203, or of the struts of both elements, only at the axially opposite ends of the stent compared to locations other than the axially opposite ends of the stent. In this case, the resistance to forces that tend to cause contraction of the blood vessel is further increased compared to cases where this is not done, and the problem of both end portions of the stent warping to a diameter that is greater than that of the central portion at the time of expansion of the stent does not occur.

Furthermore, it is also possible to make the thickness of the struts of the circumferentially extendable substantially wave-form constituent elements 202 or 203, or of both of these elements, greater only at the axially opposite ends of the stent (compared to areas other than the axially opposite ends of the stent). In this case, the resistance to forces that tend to cause contraction of the blood vessel is even greater than in cases where such a structure is not used; furthermore, the problem of both end portions of the stent warping to a greater diameter than the central portion at the time of expansion does not occur.

Furthermore, it is also possible to use a combined structure in which the length (with respect to the axial direction of the stent) of the circumferentially extendable substantially wave-form constituent elements 202 or 203, or of both of these elements, is made shorter, the width of the struts is made wider, and the thickness of the struts is made thicker only at the axially opposite ends of the stent compared to locations other than the axially opposite ends of the stent.

Furthermore, it is also possible to make the length (in the axial direction of the stent) of one or more types of elements selected from the circumferentially extendable substantially wave-form constituent elements 202 and 203 and the axially extendable substantially wave-form constituent elements 204 shorter in steps as the positions of these elements shift from the central portion of the stent (with respect to the axial direction) to the end portions of the stent. In this case, the resistance to forces that tend to cause contraction of the blood vessel is even greater than in cases where such a structure is not used, and the problem of both end portions of the stent warping to a greater diameter than the central portion at the time of expansion does not occur; furthermore, the flexibility in the axial direction of the stent can be varied in steps without causing any great variation in this flexibility.

Furthermore, it is also possible to increase in steps the width of the struts of one or more types of elements selected from the circumferentially extendable substantially wave-form constituent elements 202 and 203 and the axially extendable substantially wave-form constituent elements 204 as the positions of these elements shift from the central portion of the stent (with respect to the axial direction) to the end portions of the stent. In this case, the resistance to forces that tend to cause contraction of the blood vessel is even greater than in cases where such a structure is not used, and the problem of both end portions of the stent warping to a greater diameter than the central portion at the time of expansion does not occur; furthermore, the flexibility in the axial direction of the stent can be varied in steps without causing any great variation in this flexibility.

Furthermore, it is also possible to increase in steps the thickness of the struts of one or more types of elements selected from the circumferentially extendable substantially wave-form constituent elements 202 and 203 and the axially extendable substantially wave-form constituent elements 204 as the positions of these elements shift from the central portion of the stent (with respect to the axial direction) to the end portions of the stent. In this case, the resistance to forces that tend to cause contraction of the blood vessel is even greater than in cases where such a structure is not used, and the problem of both end portions of the stent warping to a greater diameter than the central portion at the time of expansion does not occur; furthermore, the flexibility in the axial direction of the stent can be varied in steps without causing any great variation in this flexibility.

Furthermore, it is also possible to use a combined structure in which the length (in the axial direction of the stent) of one or more types of elements selected from the circumferentially extendable substantially wave-form constituent elements 202 and 203 and the axially extendable substantially wave-form constituent elements 204 is shortened in steps, the width of the struts is increased in steps and the thickness of the struts is increased in steps as the positions of these elements shift from the central portion of the stent (with respect to the axial direction) to the end portions of the stent.

As is shown in Fig. 6, the abovementioned circumferentially extendable substantially wave-form constituent elements 202 and 203 are lined up in the circumferential direction of the stent, and are not directly connected to each other. Furthermore, the abovementioned axially extendable substantially wave-form constituent elements 204 are also lined up in the circumferential direction of the stent, and are likewise not directly connected to each other.

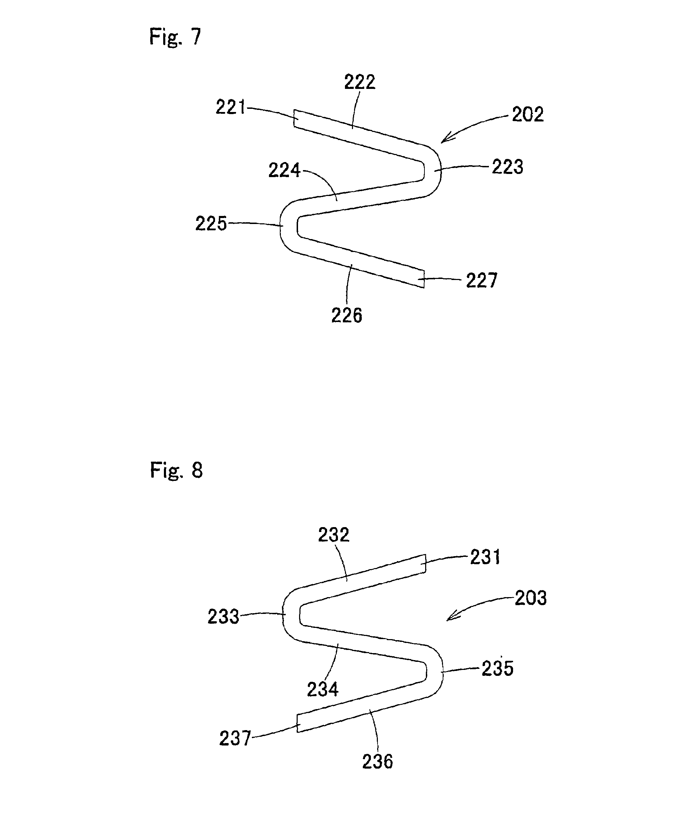

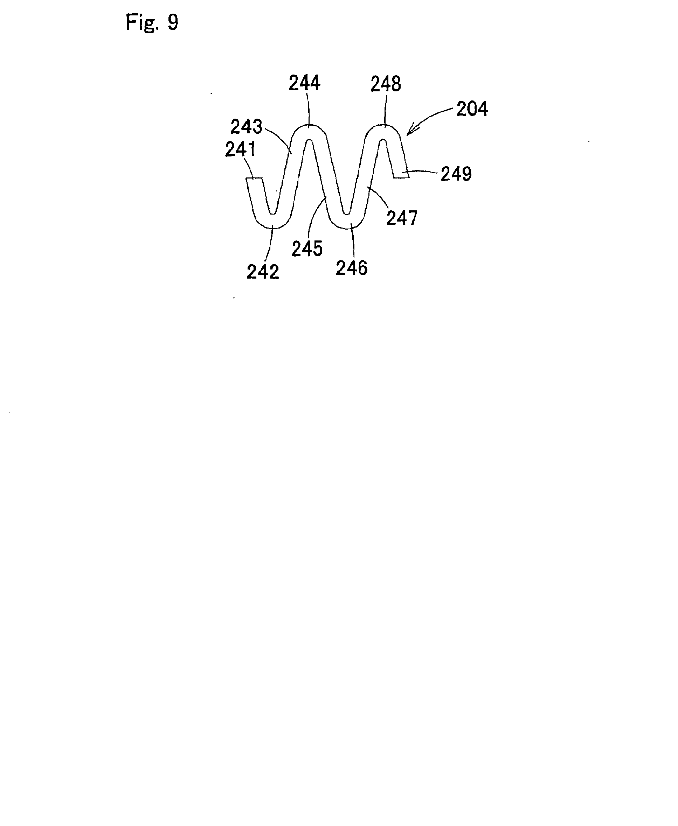

Fig. 7 shows one aspect of the circumferentially extendable substantially wave-form constituent elements 202, Fig. 8 shows one aspect of the circumferentially extendable substantially wave-form constituent elements 203, and Fig. 9 shows one aspect of the axially extendable substantially wave-form constituent elements 204. The abovementioned circumferentially extendable substantially wave-form constituent elements 202 are constructed from linear parts 222, 224, 226 and connecting parts 221, 223, 225, 227, the abovementioned circumferentially extendable substantially wave-form constituent elements 203 are constructed from linear parts 232, 234, 236 and connecting parts 231, 233, 235, 237, and the abovementioned axially extendable substantially wave-form constituent elements 204 are constructed from linear parts 243, 245,247, connecting parts 241, 249 and bent parts 242, 244, 246, 248. All of the connecting parts 221, 223, 225, 227, 231, 233, 235, 237 of the abovementioned circumferentially extendable substantially wave-form constituent elements 202 and 203 are respectively connected to one of the connecting parts 241 or 249 of the abovementioned axially extendable substantially wave-form constituent elements 204; accordingly, when the stent is expanded, the force tends to be transmitted uniformly to the abovementioned substantially wave-form constituent elements 202 and 203 and the abovementioned substantially wave-form constituent elements 204, so that the stent struts can be uniformly expanded.

In regard to the linear parts 222, 224, 226 of the abovementioned circumferentially extendable substantially wave-form constituent elements 202, the linear parts 232, 234, 236 of the abovementioned circumferentially extendable substantially wave-form constituent elements 203, and the linear parts 243, 245, 247 of the abovementioned axially extendable substantially wave-form constituent elements 204, if the width and thickness of the struts are set at large values, the flexibility of the stent in the axial direction is lost. Conversely, if the width and thickness are set at small values, the force that can withstand radial stresses that are applied from the outer circumference is reduced. Accordingly, in order to appropriately satisfy the requirements for both the flexibility of the stent in the axial direction and a force that can withstand radial stresses that are applied from the outer circumference, it is desirable that the linear parts 222, 224, 226, 232, 234, 236 of the abovementioned circumferentially extendable substantially wave-form constituent elements 202 and 203 have a width of 80 µm to 150 µm and a thickness of 70 µm to 150 µm; furthermore, a width of 120 µm to 140 µm and a thickness of 100 µm to 120 µm are even more desirable. Furthermore, it is desirable that the linear parts 243, 245, 247 of the abovementioned axially extendable substantially wave-form constituent elements 204 have a width of 50 µm to 100 µm and a thickness of 50 µm to 150 µm; moreover, a width of 60 µm to 80 µm and a thickness of 80 µm to 120 µm are even more desirable. However, both the circumferentially extendable substantially wave-form constituent elements 202 and 203 and the axially extendable substantially wave-form constituent elements 204 may be adjusted to various sizes other than the abovementioned dimensions in accordance with the material that forms the stent and the position in which the stent is used.

If the axial length of the abovementioned circumferentially extendable substantially wave-form constituent elements 202 and 203 is long, the stent cannot be smoothly bent, and the corners of the struts tend to stand out, when the stent is inserted into a bent blood vessel. Conversely, if the axial length of the abovementioned circumferentially extendable substantially wave-form constituent elements 202 and 203 is short, then the stent cannot be expanded to the required stent diameter at the time of expansion. Furthermore, if the axial length of the abovementioned axially extendable substantially wave-form constituent elements 204 is long, then the gaps that are formed between the stent struts at the time of expansion are large, so that endothelial cells of vascular tissues may bulge to considerable extent through these gap areas, thus causing re-constriction in some cases. Conversely, if the axial length o the abovementioned axially extendable substantially wave-form constituent elements 204 is short, then the flexibility of the stent in the axial direction is lost. Accordingly, it is desirable that the axial length o the abovementioned circumferentially extendable substantially wave-form constituent elements 202 and 203 be 0.8 mm to 1.8 mm, and a length of 1.0 mm to 1.4 mm is even more desirable. Furthermore, it is desirable that the axial length of the abovementioned axially extendable substantially wave-form constituent elements 204 be 0.5 mm to 1.5 mm, and a length of 0.7 mm to 1.0 mm is even more desirable. However, the circumferentially extendable substantially wave-form constituent elements 202, 203 and 204 or the axial direction may be adjusted to various sizes other than the abovementioned dimensions in accordance with the material that forms the stent and the position in which the stent is used.

The stent of the present invention can be manufactured using a metal which has an appropriate rigidity and elasticity such as stainless steel, an Ni-Ti alloy, a Cu-Al-Mn alloy or the like, or a polymer material which has an appropriate rigidity and elasticity.

The stent 201 may also be finished by plating the stent with a protective material, impregnating the stent with drugs, or covering the stent with materials.

Furthermore, laser working methods, discharge working methods, mechanical cutting methods, etching methods and the like can be used as stent forming methods.

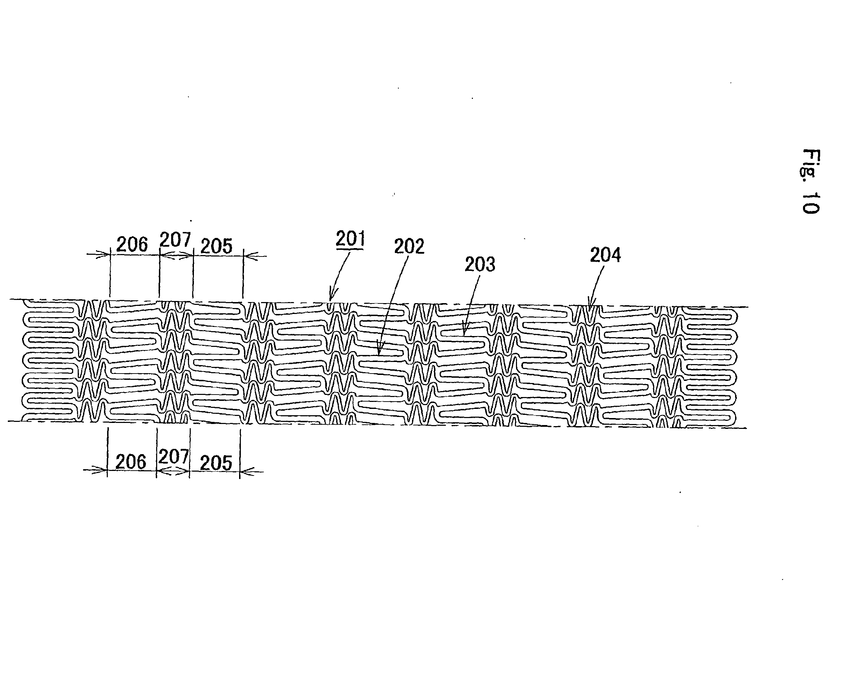

Fig. 10 shows a development view of the stent of the present invention mounted on a balloon catheter. As is shown in Fig. 10, even when the stent is mounted on a balloon catheter, the abovementioned circumferentially extendable substantially wave-form constituent elements 202 are lined up in the circumferential direction of the stent without being directly connected to each other, so that circumferential elements 205 are formed, the circumferentially extendable substantially wave-form constituent elements 203 are also lined up in the circumferential direction of the stent without being directly connected to each other, so that circumferential elements 206 are formed, the abovementioned axially extendable substantially wave-form constituent elements 204 are lined up in the circumferential direction of the stent without being directly connected to each other, so that circumferential elements 207 are formed, and the abovementioned circumferential elements 205, the abovementioned circumferential elements 206 and the abovementioned circumferential elements 207 are periodically connected in the axial direction of the stent in the order circumferential elements 205, circumferential elements 207, circumferential elements 206 and circumferential elements 207. As a result, even if the circumferentially extendable substantially wave-form constituent elements 202 or 203 contract in the axial direction when the stent is expanded, the abovementioned substantially wave-form constituent elements 204 are expanded in the axial direction, so that the overall length of the stent maintains more or less the same value before and after expansion of the stent.

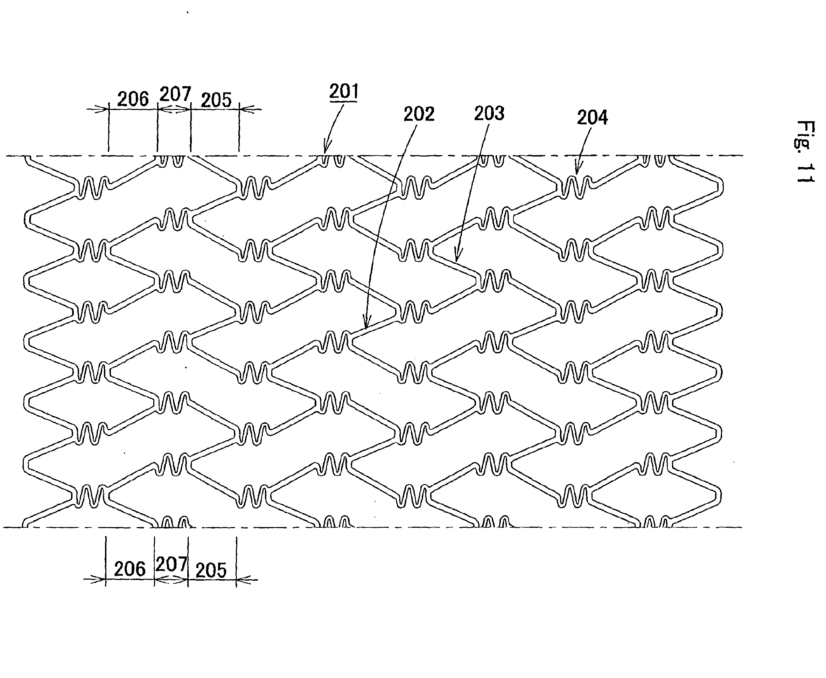

Fig. 11 shows a development view o the stent following expansion. As is shown in Fig. 11, the stent 201 following expansion is formed by combinations of oblong four-sided shapes except for the two end portions of the stent. Furthermore, the abovementioned oblong four-sided shapes include four-sided shapes oriented in two directions which have a fixed angle with respect to the axial direction of the stent. Here, the term "four-sided shapes having a fixed angle with respect to the axial direction of the stent" refers to the fact that the four-sided shapes do not have sides that are parallel to the axial direction of the stent. Thus, as a result of two types of oblong four-sided shapes that form an angle with respect to the axial direction of the stent being disposed with the regularity shown in Fig. 11, a stent which has a large resistance to forces that tend to cause a contraction of the blood vessel, and which has flexibility in the axial direction, can be realized.

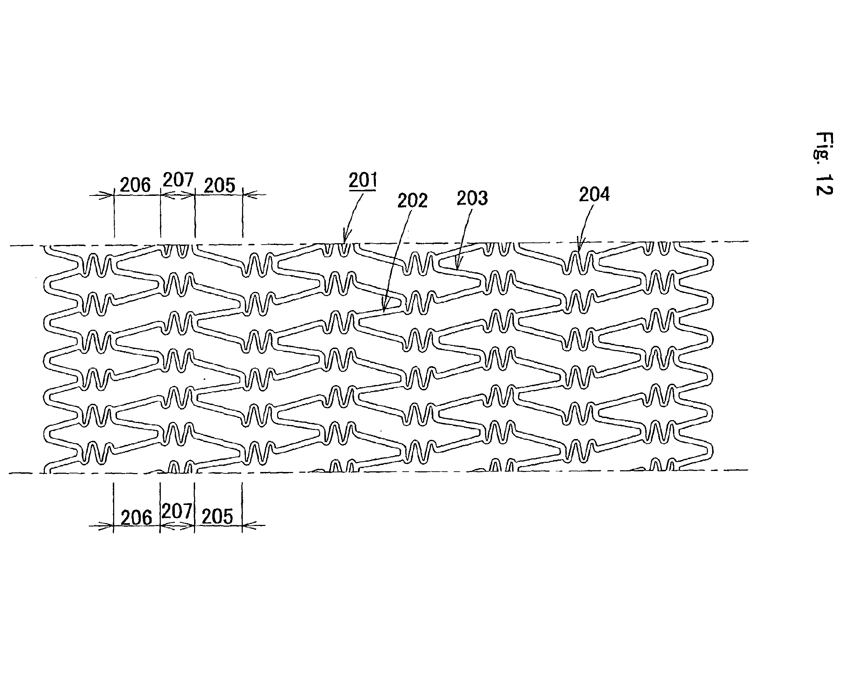

Fig. 12 shows a modification of the second embodiment of the first invention. Specifically, in the present embodiment, only the axially opposite ends of the stent are formed by a plurality of directly connected substantially wave-form constituent elements 202 or substantially wave-form constituent elements 203 that can be extended in the circumferential being disposed in the circumferential direction of the stent. Furthermore, the axial length of these elements located only at the axially opposite ends of the stent is shorter than the axial length of the substantially wave-form (circumferentially extendable) constituent elements 202 or 203 which make up the portions of the stent other than the axially opposite ends of the stent. As a result, warping of the struts on both end portions of the stent can be reduced, and the resistance of both end portions to forces that tend to cause contraction of the blood vessel can be increased. Furthermore, the resistance to forces that tend to cause contraction of the blood vessel can be increased by increasing the width or thickness of the struts only in both end portions of the stent.

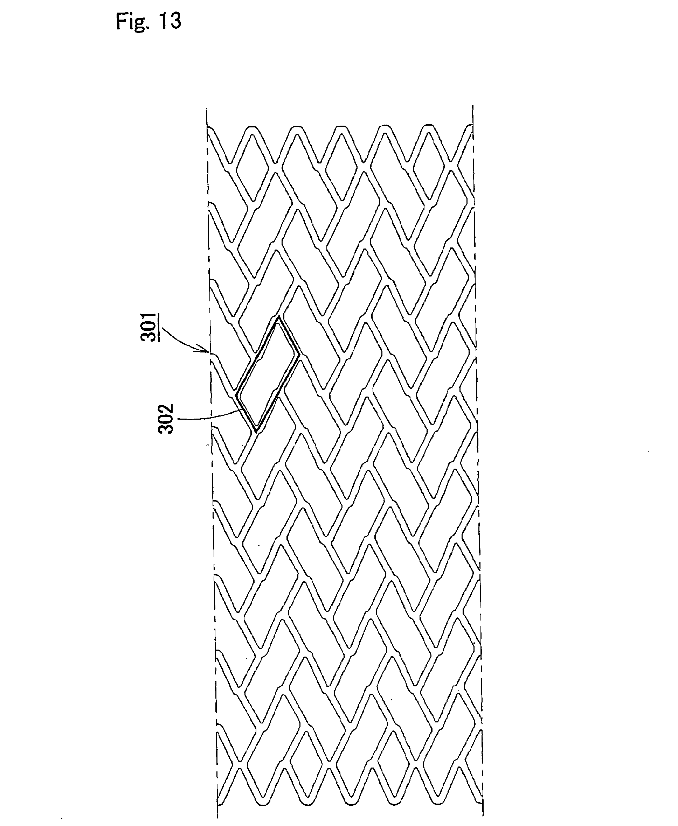

Figs. 13 through 16 show a stent constituting a third embodiment of the first invention. Fig. 13 is a development view of the stent 301 of the present invention. The stent 301 is a stent which is formed as a substantially tubular body, and which can be expanded in the radial direction from the inside of this substantially tubular body. The basic elements that form the abovementioned stent are substantially parallelogram-shaped elements. These parallelogram-shaped elements 302 are combined together in an alternately oriented fashion to form the stent 301. Here, the term "substantially parallelogram-shaped elements 302 combined together in an alternately oriented fashion" refers to the fact that substantially parallelogram-shaped elements 302 that are lined up in mutually different directions (when seen in a development view) are present, and the stent is formed by combining these elements with each other. Elements other than substantially parallelogram-shaped elements may be included only in both end portions of the stent in order to adjust both end portions of the stent to a radial section that is substantially perpendicular to the axial direction of the stent.

By constructing the stent from substantially parallelogram-shaped elements 302, it is possible to reduce the number of locations where connections are made compared to a conventional stent, so that high flexibility can be obtained prior to the expansion of the stent; furthermore, as a result of the substantially parallelogram-shaped elements 302 being combined together in an alternately oriented fashion, a high radial force is obtained following expansion of the stent. Specifically, the abovementioned substantially parallelogram-shaped elements 302 show a variation in the angle formed relative to the axial direction of the stent before and after the expansion of the stent. The angle formed by the substantially parallelogram-shaped elements 302 and the axial direction of the stent prior to expansion is smaller than the angle formed by the substantially parallelogram-shaped elements 302 and the axial direction of the stent following expansion. Accordingly, the number of locations where connections are made can be reduced compared to a conventional stent; furthermore, the following two performance features are obtained: namely, a high flexibility in the axial direction of the stent is obtained prior to the expansion of the stent, and the radial force is high following the expansion of the stent. Here, the term "radial force" refers to the resistance to forces that tend to cause contraction of the blood vessel. When the radial force is low, the resistance to forces that tend to cause contraction of the blood vessel is low, so that the stent is caused to contract by forces that tend to cause contraction of the blood vessel, thus hindering blood flow; in the worst case, this may lead to re-constriction of the blood vessel. Accordingly, a high radial force is required.

Since the substantially parallelogram-shaped elements 302 are uniformly disposed in the stent 301, the struts of the stent can be uniformly expanded.

If the number of substantially parallelogram-shaped elements 302 that are lined up in the axial direction of the stent 301 is reduced, a high flexibility in the axial direction of the stent is obtained, but the radial force is lowered. On the other hand, if the number of the abovementioned substantially parallelogram-shaped elements 302 is increased, a high radial force is obtained, but the flexibility is the axial direction of the stent drops. In order to obtain an appropriate flexibility in the axial direction of the stent and a high radial force . the number of substantially parallelogram-shaped elements 302 lined up in the axial direction of the stent 301 should be at least seven 7 but not more than 11, preferably at least 8 but not more than 10, per 20 mm of the axial length of the stent.

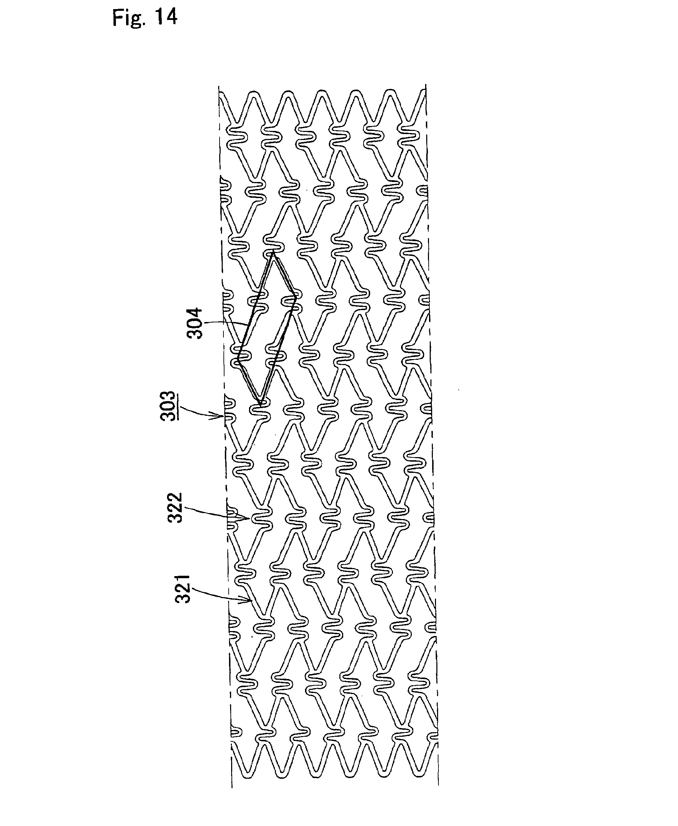

Fig. 14 is a development view of a stent 303 constituting a modification of the second embodiment of the first invention. The stent 303 is a stent which is formed as a substantially tubular body, and which can be expanded in the radial direction from the inside of this substantially tubular body, The basic elements that form the abovementioned stent 303 are substantially parallelogram-shaped elements, and the abovementioned substantially parallelogram-shaped elements 304 are combined together in an alternately oriented fashion to form the stent 303. The substantially parallelogram-shaped elements 304 are constructed from substantially linear struts 321 and substantially wave-form struts 322. Here, the term "substantially parallelogram-shaped elements 304 combined together in an alternately oriented fashion" refers to the fact that there are substantially parallelogram-shaped elements 304 that are lined up in alternate directions when seen in a development view, and the stent is formed by differently combining these elements with each other. Elements other than substantially parallelogram-shaped elements may be included only in both end portions of the stent in order to adjust both end portions of the stent to a radial section that is substantially perpendicular to the axial direction of the stent.

By constructing the stent from substantially parallelogram-shaped elements 304, it is possible to reduce the number of locations where connections are made compared to a conventional stent, so that high flexibility can be obtained prior to the expansion of the stent; furthermore, as a result of the substantially parallelogram-shaped elements 304 being combined together in an alternately oriented fashion, a high radial force is obtained following expansion of the stent. Specifically, the abovementioned substantially parallelogram-shaped elements 304 show a variation in the angle formed relative to the axial direction of the stent before and after the expansion of the stent. The angle formed by the substantially parallelogram-shaped elements 304 and the axial direction of the stent prior to expansion is smaller than the angle formed by the substantially parallelogram-shaped elements 304 and the axial direction of the stent following expansion. Accordingly, the number of locations where connections are made can be reduced compared to a conventional stent; furthermore, the following two performance features are obtained: namely, a high flexibility in the axial direction of the stent is obtained prior to the expansion of the stent, and the radial force is high following the expansion of the stent.

Furthermore, since the substantially parallelogram-shaped elements 304 have substantially wave-form struts 322, a higher flexibility in the axial direction of the stent can be obtained prior to the expansion of the stent; furthermore, as a result of the expansion and contraction of the substantially wave-form struts 322 in the axial direction, contraction of the axial length of the stent at the time of expansion can be prevented.

Since the substantially parallelogram-shaped elements 304 are uniformly disposed in the stent 303, the struts of the stent can be uniformly expanded.

If the number of substantially parallelogram-shaped elements that are lined up in the axial direction of the stent 303 is reduced, a high flexibility in the axial direction of the stent is obtained, but the radial force is lowered. On the other hand, if the number of the abovementioned substantially parallelogram-shaped elements 304 is increased, a high radial force is obtained, but the flexibility is the axial direction of the stent drops. In order to obtain an appropriate flexibility in the axial direction of the stent and a high radial force, the number of substantially parallelogram-shaped elements 304 lined up in the axial direction of the stent 303 should be at least 5 but not more than 9, preferably at least 6 but not more than 8, per 20 mm of the axial length of the stent.

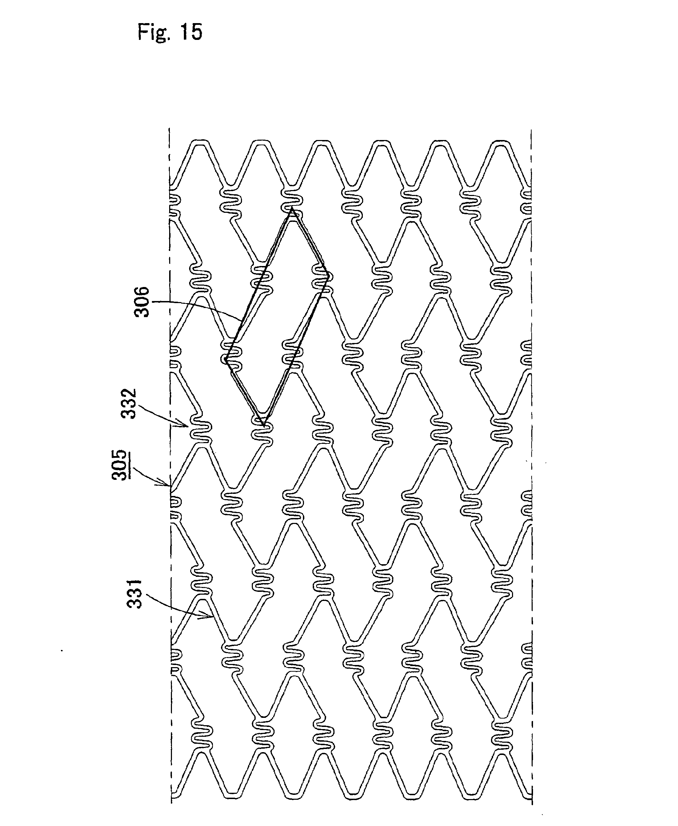

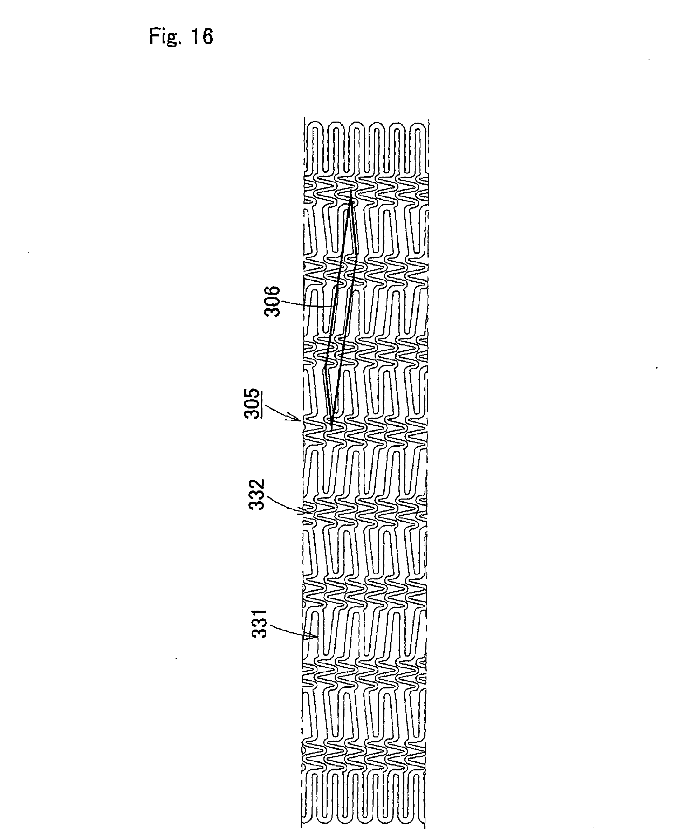

Fig. 15 is a development view of a stent 305 constituting another modification of the third embodiment of the first invention showing the state following expansion of the stent. Fig. 16 is a development view of the stent 305 before expansion thereof. The stent 305 is a stent which is formed as a substantially tubular body, and which can be expanded in the radial direction from the inside of this substantially tubular body. The basic elements that form the abovementioned stent 305 are substantially parallelogram-shaped elements, and the stent 305 is constructed by combining the abovementioned substantially parallelogram-shaped elements 306 differently with each other. The substantially parallelogram-shaped elements 306 are constructed from substantially linear struts 331 and substantially wave-form struts 332. Here, the term "substantially parallelogram-shaped elements 306 combined together in an alternately oriented fashion" refers to the fact that there are substantially parallelogram-shaped elements 306 that are lined up in alternate directions when seen in a development view, and the stent is formed by differently combining these elements with each other. Elements other than substantially parallelogram-shaped elements may be included only in both end portions of the stent in order to adjust both end portions of the stent to a radial section that is substantially perpendicular to the axial direction of the stent. Furthermore, in the stent 305, the respective sides of the substantially parallelogram-shaped elements 306 are substantially parallel to the axial direction of the stent prior to the expansion of the stent (Fig. 16), and the respective sides of the substantially parallelogram-shaped elements 306 form an angle of the stent following the expansion of the stent (Fig. 15).

By constructing the stent from substantially parallelogram-shaped elements 306, it is possible to reduce the number of locations where connections are made compared to a conventional stent, 'so that high flexibility can be obtained prior to the expansion of the stent; furthermore, as a result of the substantially parallelogram-shaped elements 306 being combined together in an alternately oriented fashion, a high radial force is obtained following expansion of the stent. Specifically, the abovementioned substantially parallelogram-shaped elements 306 show a variation in the angle formed relative to the axial direction of the stent before and after the expansion of the stent. Prior to the expansion of the stent, the respective sides of the substantially parallelogram-shaped elements 306 are substantially parallel to the axial direction of the stent (Fig. 16), while following the expansion of the stent, the respective sides of the substantially parallelogram-shaped elements 306 form an angle with respect to the axial direction of the stent. As a result. compared to conventional stents, the following two performance values are obtained: namely, a high flexibility in the axial direction of the stent is obtained prior to the expansion of the stent, and the radial force is high following the expansion of the stent.

Furthermore, since the substantially parallelogram-shaped elements 306 have substantially wave-form struts 332, a higher flexibility in the axial direction of the stent can be obtained prior to the expansion of the stent; furthermore, as a result of the expansion and contraction of the substantially wave-form struts 332 in the axial direction, contraction of the axial length of the stent at the time of expansion can be prevented.

Since the substantially parallelogram-shaped elements 306 are uniformly disposed in the stent 305, the struts of the stent can be uniformly expanded.

If the number of substantially parallelogram-shaped elements 306 that are lined up in the axial direction of the stent 305 is reduced, a high flexibility in the axial direction of the stent is obtained, but the radial force is lowered. On the other hand, if the number of the abovementioned substantially parallelogram-shaped elements 306 is increased, a high radial force is obtained, but the flexibility is the axial direction of the stent drops. In order to obtain an appropriate flexibility in the axial direction of the stent and a high radial force, the number of substantially parallelogram-shaped elements 306 lined up in the axial direction of the stent 305 should be at least 5 but not more than 9, preferably at least 6 but not more than 8, per 20 mm of the axial length of the stent.

In the stent of the present invention, an unusually high flexibility in the axial direction of the stent and a high radial force can be obtained by disposing substantially parallelogram-shaped elements 306 with different areas in the axial direction of the stent. The respective areas of the abovementioned substantially parallelogram-shaped elements 306 can be appropriately selected in accordance with the diameter and length of the stent.

In the stent 305 of the present invention, an unusually high flexibility in the axial direction of the stent and a high radial force can be obtained by combining different strut widths or strut thicknesses (or both) in the axial direction of the stent. The abovementioned strut widths and strut thicknesses can be appropriately selected in accordance with the diameter and length of the stent.

The stents 301, 303 and 305 of the third embodiment can be manufactured using a metal which has an appropriate rigidity and elasticity such as stainless steel, an Ni-Ti alloy, a Cu-Al-Mn alloy or the like, or a polymer material which has an appropriate rigidity and elasticity.

The stents 301, 303 and 305 of the third embodiment may also be finished by plating the stent with a protective material, impregnating the stent with drugs, or covering the stent with materials.

Drugs which can suppress the thickening of arterial walls, or drugs which can suppress the multiplication of vascular smooth muscle cells, anti-platelet drugs (aspiring, heparin, anti-thrombin formulations, dipyramidamole or the like) or anti-inflammatory drugs (steroids or the like) can be caused to adhere or applied as coatings to the stents 301, 303 and 305 of the third embodiment.

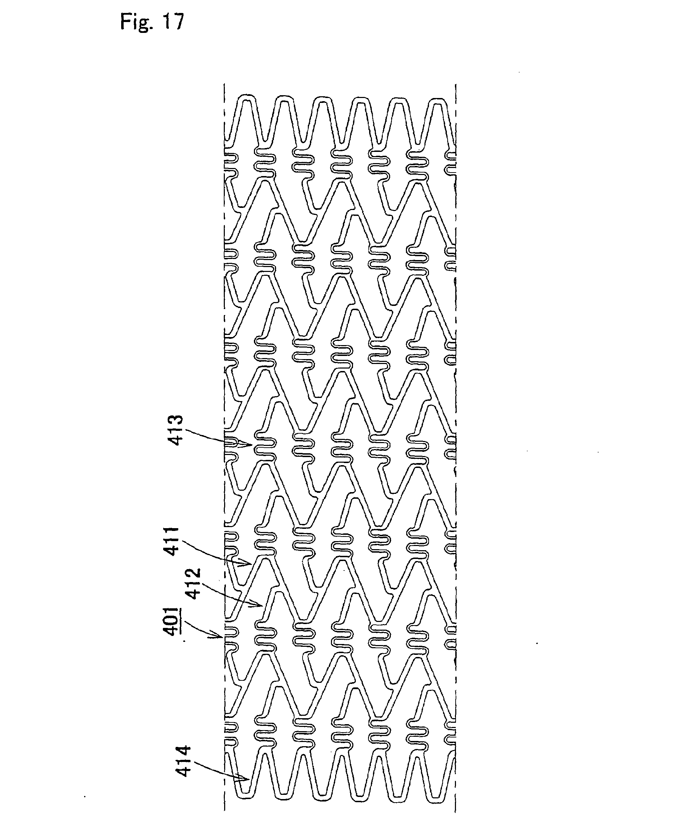

Figs. 17 through 26 show a stent constituting a fourth embodiment of the first invention. Fig. 17 is a development view of the stent 401 of the present invention. The stent 401 is a stent which is formed as a substantially tubular body, and which can be expanded outward in the radial direction of this substantially tubular body. This stent comprises annular first substantially wave-form elements 411 that can be expanded in the radial direction, axially extendable link part elements 413, branch-form elements 412 that extend from the first substantially wave-form elements, and second substantially wave-form elements 414 that form both end portions of the stent. One end of each of the link part elements 413 is connected to one of the vertices of the first substantially wave-form elements 411, the other ends of the link part elements 413 are connected to one end of each of the branch-form elements 412, and the other ends of the branch-form elements 412 are connected to the center points of the first substantially wave-form elements 411, so that these are continuous in the axial direction, and the second substantially wave-form elements 414 are connected to both end portions of the stent via the link part elements 413. As a result of having a combination of elements that can be expanded outward in the radial direction of the stent and axially extendable elements, the stent 401 can be expanded outward in the radial direction, and the contraction of the stent in the axial direction that occurs during this expansion can be reduced. Furthermore, since the stent 401 comprises annular first substantially wave-form elements 411 that can be expanded in the radial direction, axially extendable link part elements 413, branch-form elements 412 that extend from the first substantially wave-form elements 412 and second substantially wave-form elements 414 that form both end portions, the stent 401 is constructed from square shapes in which all four sides have a specified angle with respect to the axial direction of the stent at the time of the expansion of the stent. Furthermore, the abovementioned square shapes are disposed differently with respect to each other, and all four sides of the abovementioned square shapes are substantially parallel to the axial direction of the stent prior to the expansion of the stent. As a result, superior flexibility in the axial direction of the stent can be obtained prior to the expansion of the stent, and a superior strength with respect to the radial direction of the stent can be obtained following the expansion of the stent. Here, the term "radial direction of the stent" refers to a direction that is substantially perpendicular to the axial direction of the stent.

Here, the term "constituent elements that can be expanded in the radial direction or extended in the axial direction" refers to elements which have respective structures that allow expansion in the radial direction of the tubular stent or extension in the axial direction of the stent. However, it is desirable that these structures be structures that also allow contraction. For example, in cases where the stent is placed in a straight blood vessel, there is basically no problem in the case of deformation comprising only extension. However, in cases where the stent is placed in a bent blood vessel, an extra extension because of the disposition in the shape of the blood vessel is generated besides the extension generated at the time of expansion on the outside of the bent portion. In this case, if contractile deformation is possible on the inside of the bent portion, the excessive increase in the spacing between the stent struts caused by excessive extension on the outside of he bent blood vessel can be reduced. The first substantially wave-form elements 411, second substantially wave-form elements 414, and link part elements 413 that can be expanded in the radial direction or extended in the axial direction shown in the stent 401 have respective structures that also allow contraction.

In the stent 401, the annular first substantially wave-form elements 411 that can be expanded in the radial direction are disposed in the same phase in the axial direction. As a result of the abovementioned annular first substantially wave-form elements 411 being disposed in the same phase in the axial direction, uniform expansion of the stent is facilitated when the stent is expanded.

As long as the abovementioned first substantially wave-form elements 411, the abovementioned second substantially wave-form elements 414 and the abovementioned link part elements 413 have respective structures that allow expansion or extension in the radial direction or axial direction, various shapes other than those shown in Fig. 17 may be used. For example, in the case of the first substantially wave-form elements 411 and second substantially wave-form elements 414 that can be expanded in the radial direction, the numbers or angles of the peaks and valleys of the wave-form shape may be adjusted, or the entire elements may be formed with curved surfaces or the like, in order to adjust the required dimensions at the time of expansion or the expanding force.

If the axial length of the abovementioned first substantially wave-form elements 411 that can be expanded in the radial direction is long, the stent cannot be smoothly bent, and the corners of the struts tend to stand out, when the stent is inserted into a bent blood vessel. Conversely, if this length is short, then the stent cannot be expanded to the required stent diameter at the time of expansion. Furthermore, if the axial length of the axially extendable link part elements 413 is long, then the gaps that are formed between the stent struts at the time of expansion are large, so that endothelial cells of vascular tissues may bulge to considerable extent through these gap areas, thus causing re-constriction in some cases. Conversely, if the axial length of the link part elements 413 is short, then the flexibility of the stent in the axial direction is lost. Accordingly, it is desirable that the axial length of the abovementioned first substantially wave-form elements 411 that can be expanded in the radial direction be 1.0 mm to 2.2 mm, preferably 1.4 mm to 1.8 mm. Furthermore, it is desirable that the axial length of the axially extendable link part elements 413 be 0.5 mm to 1.5 mm, preferably 0.8 mm to 1.2 mm.

If the number of waves of the abovementioned annular first substantially wave-form elements 411 per single circumference is small, a large blood vessel retaining force cannot be expected. Preferably, the number of waves of the abovementioned annular first substantially wave-form elements 411 per single circumference is 3 or greater; in this case, a high blood vessel retaining force can be manifested. Here, the "number of waves of the abovementioned annular first substantially wave-form elements 411 per single circumference" refers to a number in which one period including a peak and valley is counted as one wave; in Fig. 17, the number of waves of the abovementioned annular first substantially wave-form elements 411 per single circumference is 3.

The opposite ends of the stent 401 are constructed from the abovementioned annular second substantially wave-form elements 414 which are not continuous with the abovementioned branch-form elements 412. Furthermore, in regard to the number of waves per single circumference, if the number of waves of the abovementioned annular second substantially wave-form elements 414 that form both end portions of the stent is made greater than the number of waves of the abovementioned annular first substantially wave-form elements 411 that form the central portion of the stent, then the opposite ends of the stent will have a greater resistance to forces that tend to cause contraction of the blood vessel than the central portion of the stent; furthermore, the problem of both end portions of the stent being warped to a greater diameter than the central portion of the stent at the time of expansion will not occur.

Furthermore, the axial length of the abovementioned annular second substantially wave-form elements 414 that form both end portions of the stent can be made shorter than the axial length of the abovementioned annular first substantially wave-form elements 411 that form the central portion of the stent. In this case, the resistance to forces that tend to cause contraction of the blood vessel is further increased compared to cases where such a structure is not used; furthermore, the problem of both end portions of the stent warping to a greater diameter than the central portion at the time of expansion does not occur.

Furthermore, it is also possible to make the width of the struts of the abovementioned annular second substantially wave-form elements 414 that form both end portions of the stent greater than the width of the axial struts of the abovementioned annular first substantially wave-form elements 411 that form the central portion of the stent. In this case, the resistance to forces that tend to cause contraction of the blood vessel is further increased compared to cases where such a structure is not used; furthermore, the problem of both end portions of the stent warping to a greater diameter than the central portion at the time of expansion does not occur.

Furthermore, it is also possible to make the thickness of the struts of the abovementioned annular second substantially wave-form elements 414 that form both end portions of the stent greater than the thickness of the axial struts of the abovementioned annular first substantially wave-form elements 411 that form the central portion of the stent. In this case, the resistance to forces that tend to cause contraction of the blood vessel is further increased compared to cases where such a structure is not used; furthermore, the problem of both end portions of the stent warping to a greater diameter than the central portion at the time of expansion does not occur.

Furthermore, it is also possible to use a combined structure in which only the abovementioned annular second substantially wave-form elements 414 that form both end portions of the stent are formed with a shorter length in the axial direction of the stent, a greater strut width and a greater strut thickness than the abovementioned annular first substantially wave-form elements 411 that form the central portion of the stent.

Furthermore, it is also to form the abovementioned annular first substantially wave-form elements 411 that form the central portion of the stent and the abovementioned annular second substantially wave-form elements 414 that form both end portions of the stent so that the length of these elements in the axial direction of the stent is shortened, the strut width is increased and the strut thickness is increased in steps as the positions of these elements shift from the central portion of the stent (with respect to the axial direction) to the end portions of the stent. In this case, the resistance to forces that tend to cause contraction of the blood vessel is further increased compared to cases where such a structure is not used; furthermore, the problem of both end portions of the stent warping to a greater diameter than the central portion at the time of expansion does not occur, and the flexibility in the axial direction of the stent can be varied in steps.

In regard to the abovementioned first substantially wave-form elements 411 and the abovementioned second substantially wave-form elements 414, if the width and thickness of the struts are increased, then the flexibility in the axial direction of the stent is lost. Conversely, if the width and thickness of the struts are reduced, then the force that can withstand radial stresses that are applied from the outer circumference is reduced. Accordingly, in order to appropriately satisfy performance requirements for both flexibility in the axial direction of the stent and a force that can withstand radial stresses that are applied from the outer circumference, it is desirable that the abovementioned first substantially wave-form elements 411 and the abovementioned second substantially wave-form elements 414 have a width of 80 µm to 150 µm and a thickness of 70 µm to 150 µm; furthermore, a width of 120 µm to 140 µm and a thickness of 100 µm to 120 µm are even more desirable.

In regard to the abovementioned link part elements 413, if the width and thickness of the struts are increased, then the flexibility in the axial direction of the stent is lost. Conversely, if the width and thickness are reduced, then there is a danger of breakage when the stent is bent. Accordingly, in order to appropriately satisfy the performance requirements for both flexibility in the axial direction of the stent and strength against breaking during bending of the stent, it is desirable that the abovementioned link part elements 413 have a width of 30 µm to 90 µm and a thickness of 70 µm to 150 µm; furthermore, a width of 50 µm to 70 µm and a thickness of 100 µm to 120 µm are even more desirable.

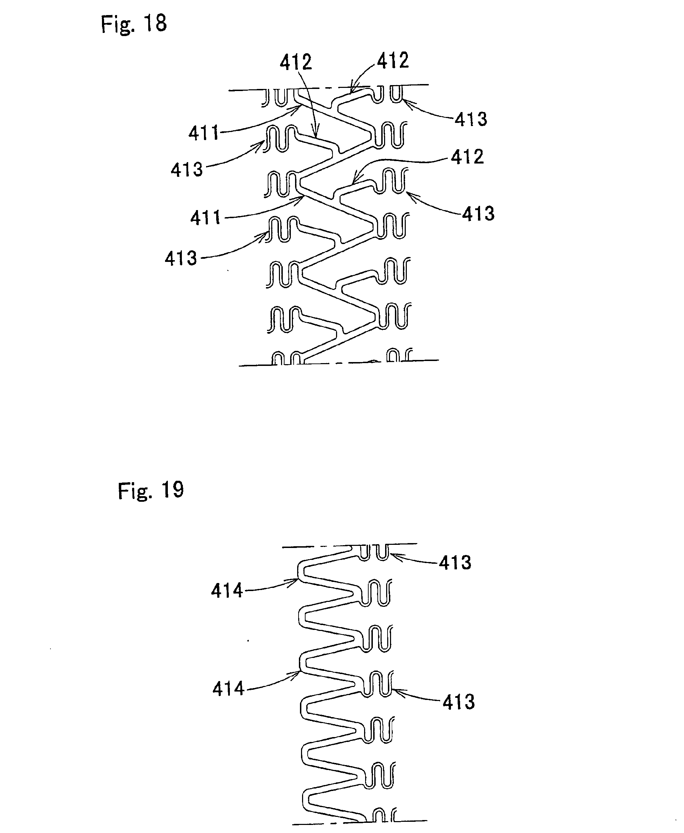

Fig. 18 is a development view of the central portion of the stent 401 of the present invention. This portion is constructed from the abovementioned first substantially wave-form elements 411 and the abovementioned branch-form elements 412. The first substantially wave-form elements 411 form annular bodies that an expand in the radial direction, and one branch-form element 412 is disposed on each substantially linear side of each first substantially wave-form element 411. It is desirable that the abovementioned branch-form elements 412 be disposed on the center point of each substantially linear side of the abovementioned first substantially wave-form elements 411; as a result, the stent can be uniformly expanded.

Fig. 19 is a development view of both end portions of the stent 401 of the present invention. These end portions of the stent comprise the second substantially wave-form elements 414, and are connected to the link part elements 413 in the vicinity of the vertices of the substantially wave-form shapes. The axial length of the abovementioned second substantially wave-form elements 414 can be determined independently from the axial length of the abovementioned substantially wave-form elements 411. It is desirable that the axial length of the abovementioned second substantially wave-form elements 414 be longer than the axial length of the abovementioned branch-form elements 412 and shorter than the axial length of the abovementioned substantially wave-form elements 411. As a result, the flexibility of the stent can be made uniform in the axial direction.

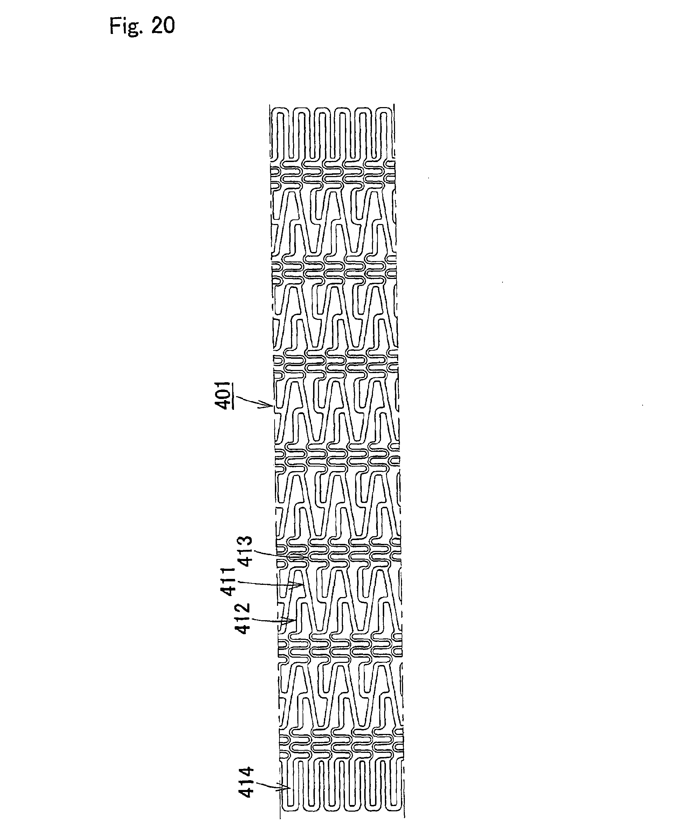

Fig. 20 is a development view of the stent 401 of the present invention prior to expansion. This figure shows a development view of a state in which the stent is mounted on a balloon catheter. When the stent is expanded, even if the first substantially wave-form elements 411 and second substantially wave-form elements 414 that can be expanded in the radial direction contact in the axial direction, the link part elements 413 are expanded in the axial direction, so that the overall length of the stent can be maintained at more or less the same length before and after expansion of the stent.

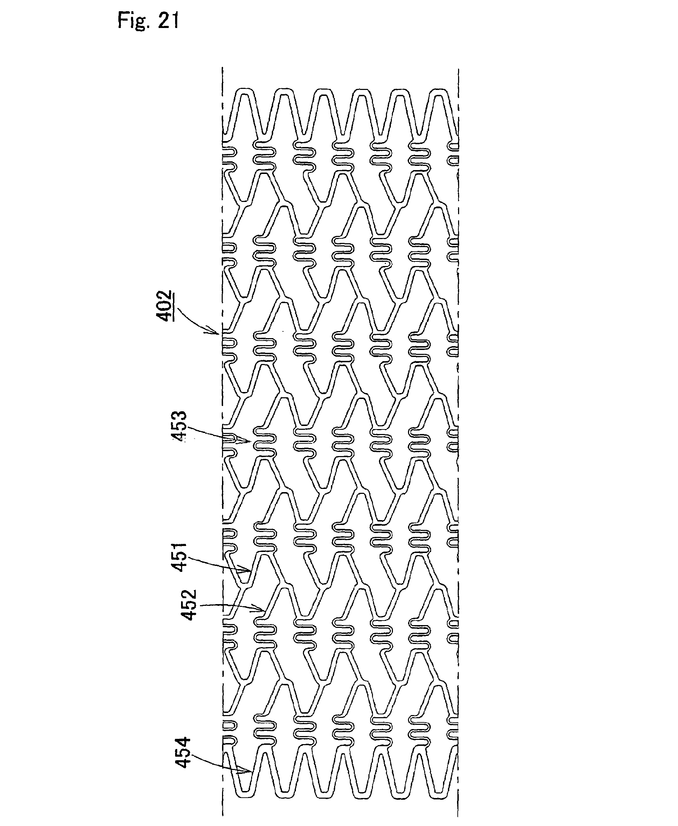

Fig. 21 is a development view of the stent 402 of the present invention. The stent 402 is a stent which is formed as a substantially tubular body, and which can be expanded outward in the radial direction of this substantially tubular body. This stent comprises annular first substantially wave-form elements 451 that can be expanded in the radial direction, axially extendable link part elements 453, branch-form elements 452 that extend from the first substantially wave-form elements 451, and second substantially wave-form elements 454 that form both end portions of the stent. One end of each of the link part elements 453 is connected to one of the vertices of the first substantially wave-form elements 451, the other ends of the link part elements 453 are connected to one end of each of the branch-form elements 452, and the other ends of the branch-form elements 452 are connected to the center points of the first substantially wave-form elements 451, so that these are continuous in the axial direction, and the second substantially wave-form elements 454 are connected to both end portions of the stent via the link part elements 453. As a result of having a combination of elements that can be expanded in the radial direction and axially extendable elements, the stent 402 can be expanded outward in the radial direction, and the contraction of the stent in the axial direction during this expansion can be reduced. Furthermore, since the abovementioned stent 402 comprises annular first substantially wave-form elements 451 that can be expanded in the radial direction, axially extendable link part elements 453, branch-form elements 452 that extend from the first substantially wave-form elements 451 and second substantially wave-form elements 454 that form both end portions of the stent, the stent 402 is constructed from square shapes in which all four sides have a specified angle with respect to the axial direction of the stent prior to the expansion of the stent. Furthermore, the abovementioned square shapes are disposed differently with respect to each other, and all fours sides of the abovementioned square shapes are substantially parallel to the axial direction of the stent prior to the expansion of the stent. As a result, a superior flexibility is obtained in the axial direction of the stent prior to the expansion of the stent, and a superior strength is obtained in the radial direction of the stent following the expansion of the stent.

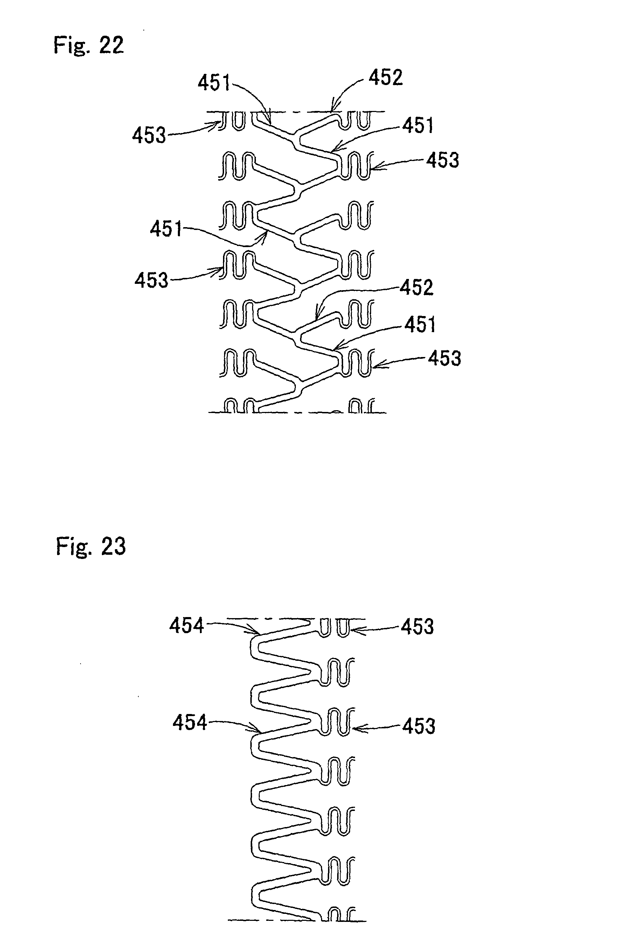

Fig. 22 is a development view of the central portion of the stent 402 of the present invention. This portion is constructed from the abovementioned first substantially wave-form elements 451 and the abovementioned branch-form elements 452. The first substantially wave-form elements 451 form annular bodies that can expand in the radial direction, and one branch-form element 452 is disposed on one substantially linear side of each first substantially wave-form element 451. It is desirable that the abovementioned branch-form elements 452 be disposed on the center point of one substantially linear side of each of the abovementioned first substantially wave-form elements 451; as a result, the stent can be uniformly expanded.

Fig. 23 is a development view of both end portions of the stent 402 of the present invention. Both end portions of the abovementioned stent comprise second substantially wave-form elements 454, and are connected to the link part elements 453 in the vicinity of the vertices of the substantially wave-form shapes. The axial length of the abovementioned second substantially wave-form elements 454 can be determined independently from the axial length of the abovementioned substantially wave-form elements 451. It is desirable that the axial length of the abovementioned second substantially wave-form elements 454 be longer than the axial length of the abovementioned branch-form elements 452 and shorter than the axial length of the abovementioned substantially wave-form elements 451. As a result, the flexibility of the stent can be made uniform in the axial direction.

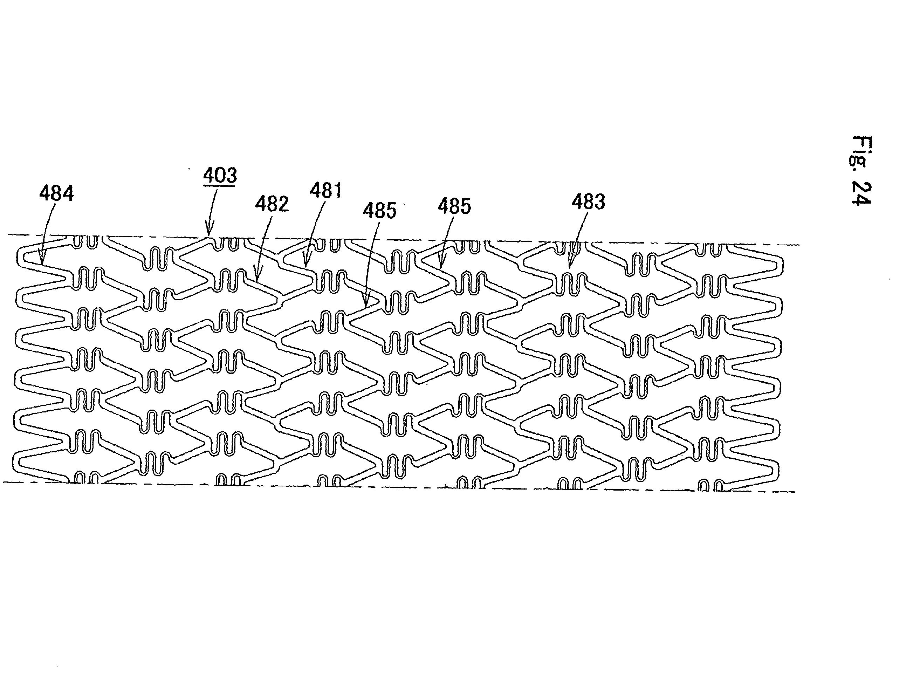

Fig. 24 is a development view of the stent 403 of the present invention. The stent 403 is a stent which is formed as a substantially tubular body, and which can be expanded outward in the radial direction of this substantially tubular body. The abovementioned stent comprises annular first substantially wave-form elements 481 that can be expanded in the radial direction, axially extendable link part elements 483, branch-form elements 482 that extend from the first substantially wave-form elements 481, substantially N-shaped elements 485, and second substantially wave-form elements 484 that form both end portions of the stent. One end of each of the link part elements 483 is connected to one of the vertices of the first substantially wave-form elements 481, the other ends of the link part elements 483 are connected to one end of each of the substantially N-shaped elements 485, one end of each of the branch-form elements 482 extending from the center points of the first substantially wave-form elements 481 is connected to one end of each of the link part elements 483, the other ends of the link part elements 483 are connected to one of the vertices of the substantially N-shaped elements 485, one end of each of the link part elements 483 is connected to the vertex of one substantially N-shaped element 485 out of two substantially N-shaped elements 485 that are adjacent to each other in the axial direction, and the other end of each of the link part elements 483 is connected to one end of the other substantially N-shaped element 485 of the abovementioned pair of adjacent N-shaped elements 485; furthermore, one end of each link part element 483 is connected to the other end of the first substantially N-shaped element 485, and the other end of this link part element 483 is connected to the vertex of the other substantially N-shaped element 485.

As a result of having a combination of elements that can be expanded outward in the radial direction of the stent and axially extendable elements, the stent 403 can be expanded outward in the radial direction, and the contraction of the stent in the axial direction during this expansion can be reduced. Furthermore, as a result of the inclusion of N-shaped elements 485, the flexibility in the axial direction of the stent can be increased. Furthermore, since the cells that include the N-shaped elements 485 have four link part elements 483 per single cell circumference, this part can be opened widely using a balloon catheter in cases where this is necessary, so that deeply located areas of pathological changes can be treated via this opening. Furthermore, since the abovementioned stent 403 comprises annular first substantially wave-form elements 481 that can be expanded in the radial direction, axially extendable link part elements 483, branch-form elements 482 that extend from the first substantially wave-form elements 481, substantially N-shaped elements 485 and second substantially wave-form elements 484 that form both end portions of the stent, the stent 403 is constructed from square shapes in which all four sides have a specified angle with respect to the axial direction of the stent prior to the expansion of the stent. Furthermore, the abovementioned square shapes are disposed differently with respect to each other, and all four sides of the abovementioned square shapes are substantially parallel to the axial direction of the stent prior to the expansion of the stent. As result, a superior flexibility is obtained in the axial direction of the stent prior to the expansion of the stent, and a superior strength in the radial direction of the stent is obtained following the expansion of the stent.

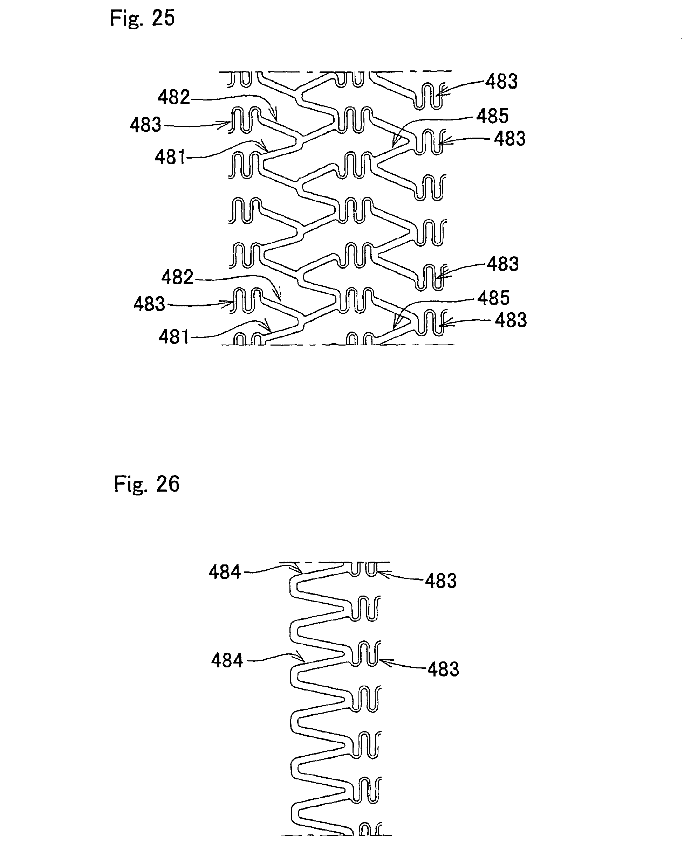

Fig. 25 is a development view of the central portion of the stent 403 of the present invention. This portion is constructed from annular first substantially wave-form elements 481 that can be expanded in the radial direction, axially extendable link part elements 483, branch-form elements 482 that extend from the first substantially wave-form elements 481, and substantially N-shaped elements 485. The first substantially wave-form elements 481 form annular bodies that can expand in the radial direction, and one branch-form element 482 is disposed on one substantially linear side of each first substantially wave-form element 481. It is desirable that the abovementioned branch-form elements 482 be disposed on the center point of one substantially linear side of each of the abovementioned first substantially wave-form elements 481; as a result, the stent can be uniformly expanded.

Fig. 26 is a development view of both end portions of the stent 403 of the present invention. Both end portions of the abovementioned stent comprise second substantially wave-form elements 484, and are connected to the link part elements 483 in the vicinity of the vertices of the substantially wave-form shapes. The axial length of the abovementioned second substantially wave-form elements 484 can be determined independently from the axial length of the abovementioned substantially wave-form elements 481. It is desirable that the axial length of the abovementioned second substantially wave-form elements 484 be longer than the axial length of the abovementioned branch-form elements 482, but shorter than the axial length of the abovementioned substantially wave-form elements 481. As a result, the flexibility of the stent can be made uniform in the axial direction.

The stents 401, 402 and 403 of the present invention can be manufactured using a metal which has an appropriate rigidity and elasticity such as stainless steel, an Ni-Ti alloy, a Cu-Al-Mn alloy or the like, or a polymer material which has an appropriate rigidity and elasticity.

The stents 401, 402 and 403 of the present invention may also be finished by plating the stent with a protective material, impregnating the stent with drugs, or covering the stent with materials.

Furthermore, laser working methods, discharge working methods, mechanical cutting methods, etching methods and the like can be used as stent forming methods.

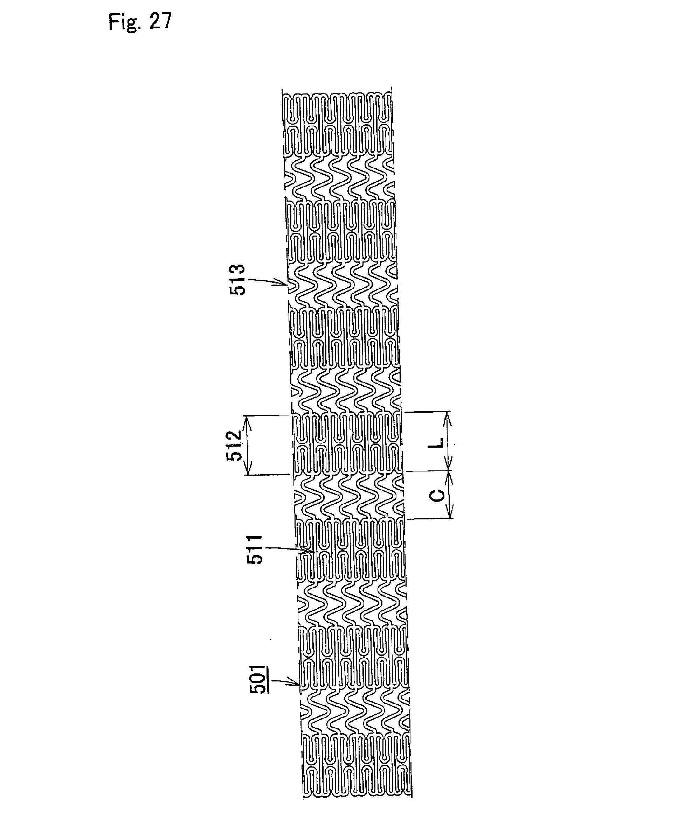

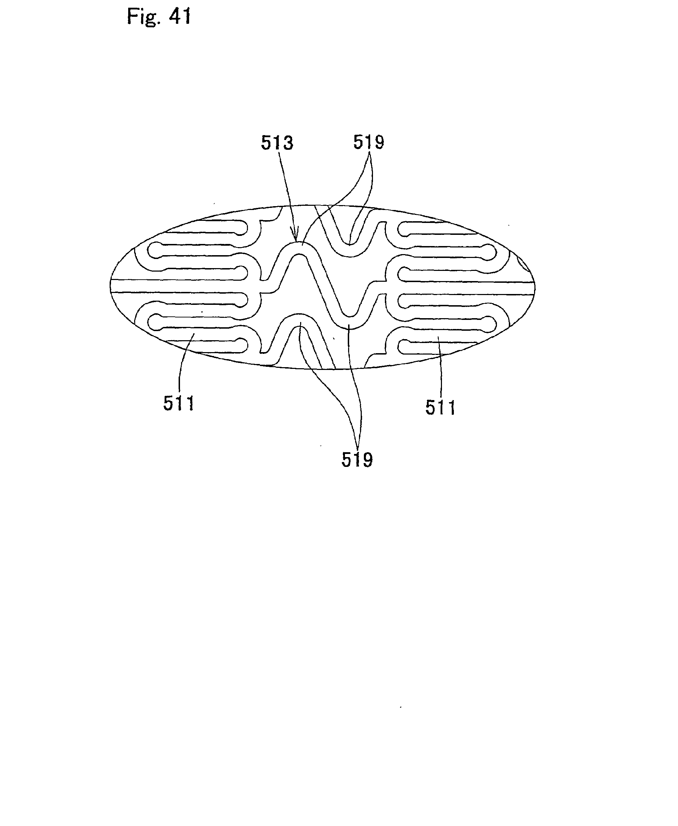

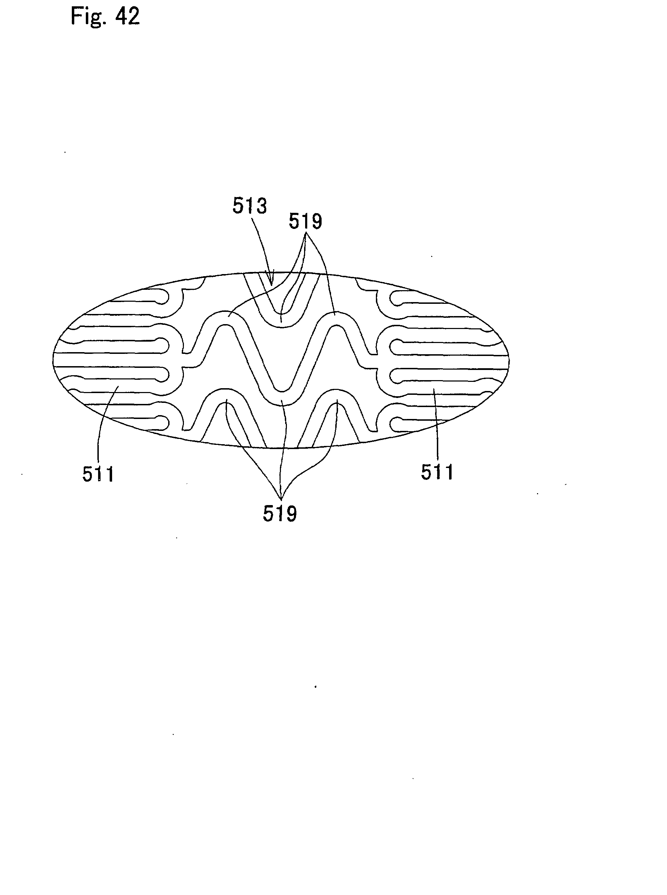

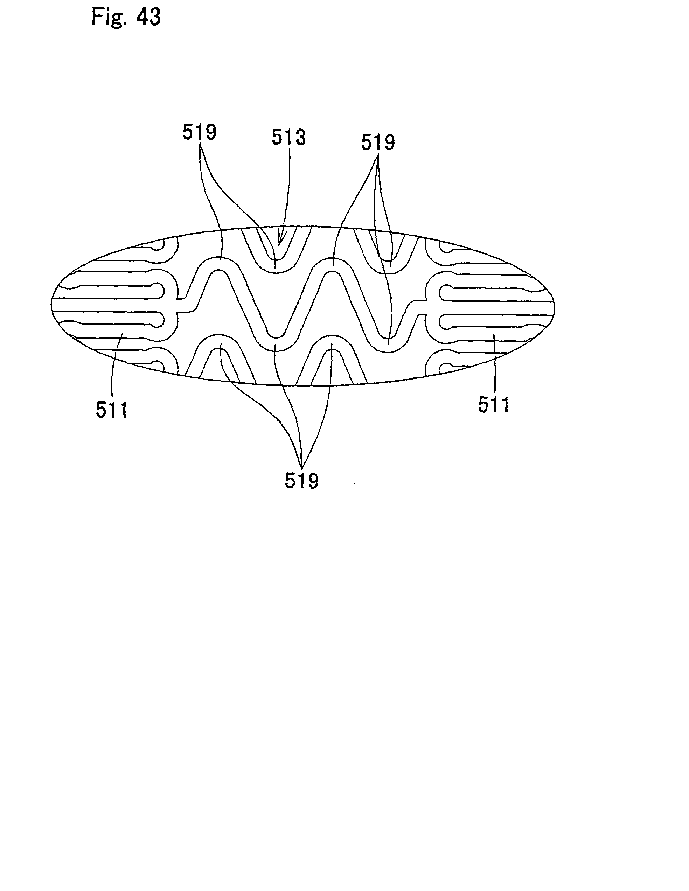

Figs. 27 through 44 show a stent constituting the second invention of the present application. Fig. 27 is a development view of the stent 501 of the present invention in a state in which the stent has not yet been expanded. The stent 501 is a stent which is formed as a substantially tubular body, and which can be expanded outward in the radial direction of this tubular body. A plurality of circumferentially extendable basic cells 511 of the stent are continuous in the circumferential direction of the stent, thus forming band parts 512 which generate a force that against forces that tend to cause contraction of the blood vessel at the time of expansion, i. e., a radial force. The respective band parts 512 are connected by link parts 513 in order to impart flexibility with respect to forces from a direction perpendicular to the direction of length of the stent 501 (resulting from the stent advancing through bent tubular cavities such as blood vessels or the like). The band pats 512 and link parts 513 are continuous in the longitudinal direction for the required length of the stent.

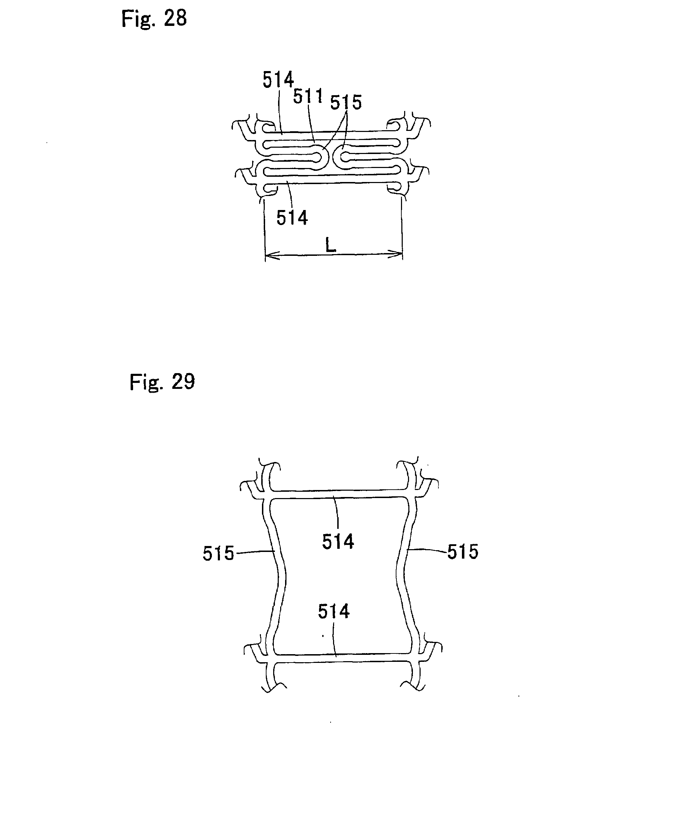

Fig. 28 shows an enlargement of the structure of the basic cells 511 of this stent 501. The basic cells 511 comprise main struts 514 which are disposed with the length of the struts oriented in the axial direction of the stent, and sub-struts 515 which are folded between the main struts 514, and which support the main struts 514 in the circumferential direction when the stent is expanded. These sub-struts 515 have the functions of supporting the main struts 514 in the circumferential direction, and regulating the diameter to which the stent is expanded when the stent 501 is expanded in the radial direction. Fig. 29 shows the state of the basic cells 511 when the stent 501 is in an expanded state. The main struts 514 and sub-struts 515 are continuous so that an annular shape is formed, thus forming substantially square shapes.

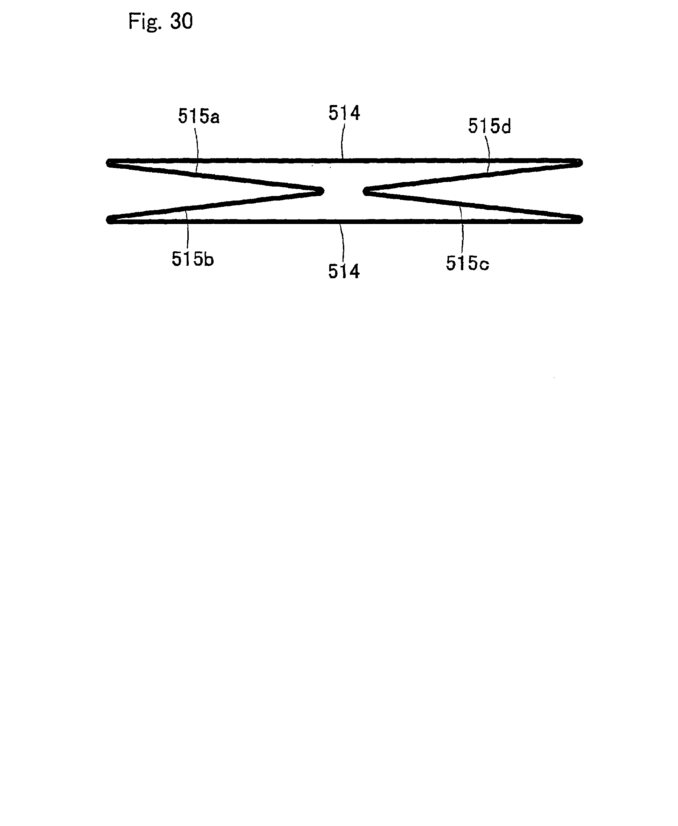

Fig. 30 shows this basic cell 511 modeled by line segments. The respective dimensions used in the present invention refer to dimensions used in the case of modeling by line segments. A dimensional ratio is used which satisfies the relationships π × D = 0.5 × A × sin θ × B and 60° ≤ θ < 90°, where L is the length of the basic cells 511 in the longitudinal direction, i. e., the length of the main struts 514, A is the total length of the sub-struts 515 within one basic cell 511 folded between the main struts 514, i. e., the length corresponding to 515a + 515b + 515c + 515d, B is the number of basic cells 511 that are continuous in the circumferential direction within one band part 512 of the stent 501, and D is the desired expanded diameter of the stent. Here, π is the circumference ratio. Furthermore, θ is the angle on the side of the inside angle of the substantially polygonal shape (of the angles formed by line segments used to model the sub-struts 515 and the axial direction of the stent at the time of expansion), i. e., the angle that is smaller than 90°. In order to ensure that the respective basic cells 511 that are continuous in the circumferential direction are caused to undergo a uniform expansion, it is important that θ be 60° or greater. If θ is smaller than this, a sufficiently uniform expansion cannot be obtained. Furthermore, the value of this θ is theoretically smaller than θ = 90°, which is the state of maximum expansion. In the present invention, the fact that these sub-struts 515 support the main struts 514 at a large angle is the most important special feature; as a result, it is possible to obtain a large radial force, and to suppress excessive expansion of the stent. If the radial force can be strengthened, then the width W of the wire material that forms the main struts 514 and sub-struts 515 constituting the stent can be reduced, so that the number of basic cells formed inside one band can be increased. As a result, the size of the basic cells can be reduced, so that the opening area of the basic cells at the time of expansion can be reduced; accordingly, the bulging of endothelial cells of vascular tissues into the stent can be suppressed to a minimum. From the standpoints of the radial force and suppression of excessive expansion, it is further desirable that the angle formed by the sub-struts 515 and the axial direction of the stent be such that 70° ≤ θ ≤ 80°.

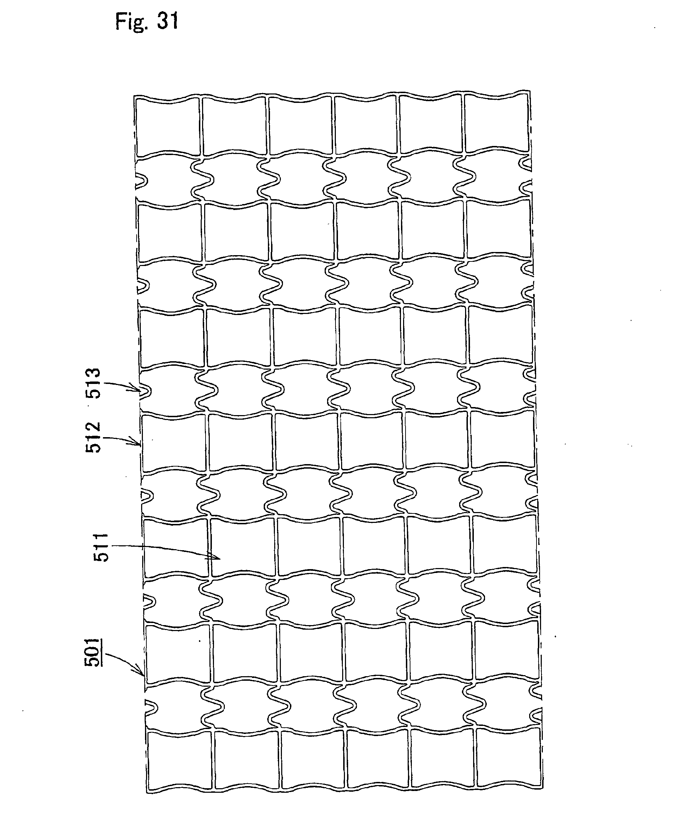

Furthermore, in order to suppress the bulging of endothelial cells of vascular tissues through the centers of the substantially square shapes formed by the main struts 514 and sub-struts 515 following the expansion of the stent, it is desirable that the relationship between the length L of he main struts 514 and the overall length A of the sub-struts 515 inside one basic cell be L ≤ A < 2 × L. If L > A, the basic cells 511 at the time of expansion will form substantially oblong shapes that are long in the longitudinal direction; under such conditions, a radial force cannot be sufficiently manifested in the vicinity of the central portions of the main struts 514. Conversely, in cases where A ≥ 2 2 × L, the sub-struts 515 that are folded between the main struts 514 overlap with each other in the longitudinal direction, and the number B of basic cells that are continuous in the circumferential direction cannot be increased. Under such conditions, the size of the substantially square shapes formed by the main struts 514 and sub-struts 515 following expansion is increased, so that endothelial cells of vascular tissues tend to bulge v into the- stent. Furthermore, the problem of a weakening of, the radial force in the circumferential direction also arises. The most ideal state is a state in which the total length A of the sub-struts 515 within one basic cell is smaller than 2 × L, so that the sub-struts 515 inside one basic cell do not overlap with each other, and A is the maximum length. In this case, the substantially square shapes formed by the main struts 514 and sub-struts 515 are shapes that are close to a true square. Fig. 31 is a development view of this stent 501 following expansion.

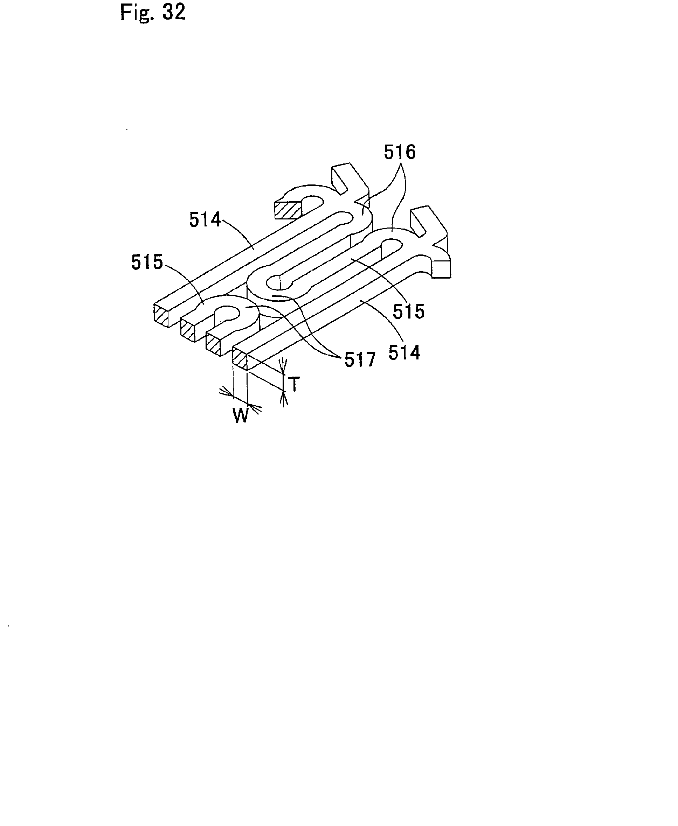

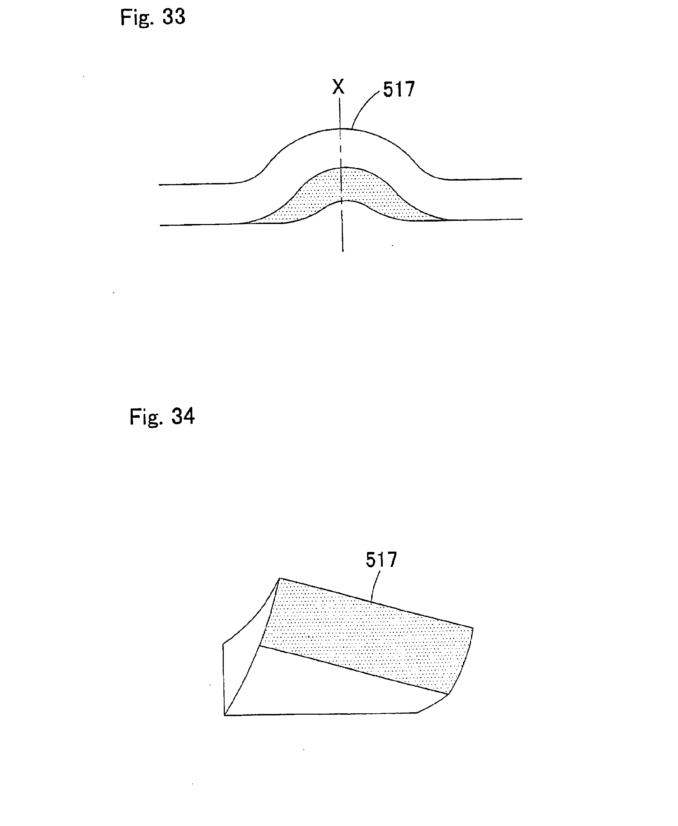

Fig. 32 is a diagram showing a portion of the section of the stent 501. In this diagram, it is desirable that the condition 0.5 × W ≤ T ≤ 3 × W be satisfied, where W is the width of the wire material that forms the main struts 514 and sub-struts 515, and T is the thickness of this wire material. In cases where the thickness T is smaller than 0.5 x W, portions that show great plastic deformation at the time of expansion of the stent, i. e., the portions 516 that connect the main struts 514 and sub-struts 515, and the folded tip end portions 517 of the sub-struts 515, peel upward as shown in Fig. 33 when this plastic deformation occurs, and these portions bite into the inside surfaces of the vascular tissues. Fig. 34 shows a section along line X-X of the peeled-up portion 517 shown in Fig. 33. In order to suppress such deformation, it is desirable that the thickness T of the respective struts be 0.5 × W or greater, and a thickness of 0.7 × W or greater is even more desirable. Such a condition also applies in the case of self-expanding stents made of super-elastic metals or some polymer materials. Portions that show great elastic deformation when folded into shape prior the expansion of the stent, i. e., the portions 516 that connect the main struts 514 and sub-struts 515, and the folded tip end portions 517 of the sub-struts 515 folded inside the main struts 514, are deformed as shown in Fig. 33. In the case of self-expanding stents, a system is generally used in which the stent is inserted in state in which the stent is folded inside a sheath that prevents expansion of the stent while the stent is transported into the desired vascular tissues inside the body; then, while the stent is transported by the catheter inside the stent and fixed in the desired position so that the position of the stent does not shift, the outside sheath is pulled away and the stent is allowed to undergo self-expansion. If deformed portions such as those shown in Fig. 33 are formed when the stent is folded into shape prior to expansion, uniform insertion of the folded stent into the sheath becomes difficult, and when the sheath is pulled away and the stent is expanded, the abovementioned peeled-up portions catch on the sheath, so that in the worst case, it may be impossible to release the stent from the sheath. On the other hand, if the thickness T of the stent is greater than 3 × W, the working precision that is obtained when the pattern of the stent is worked by a laser drops.

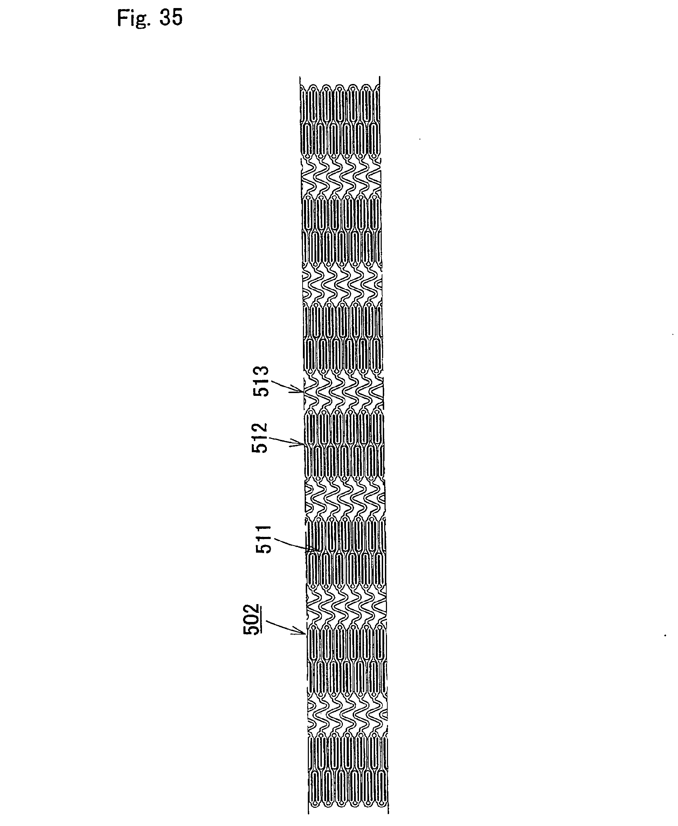

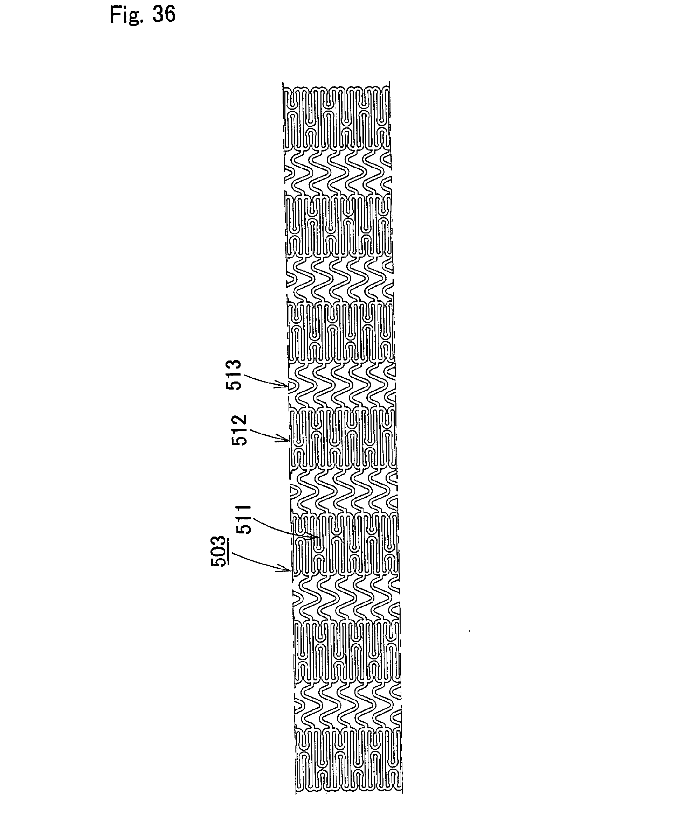

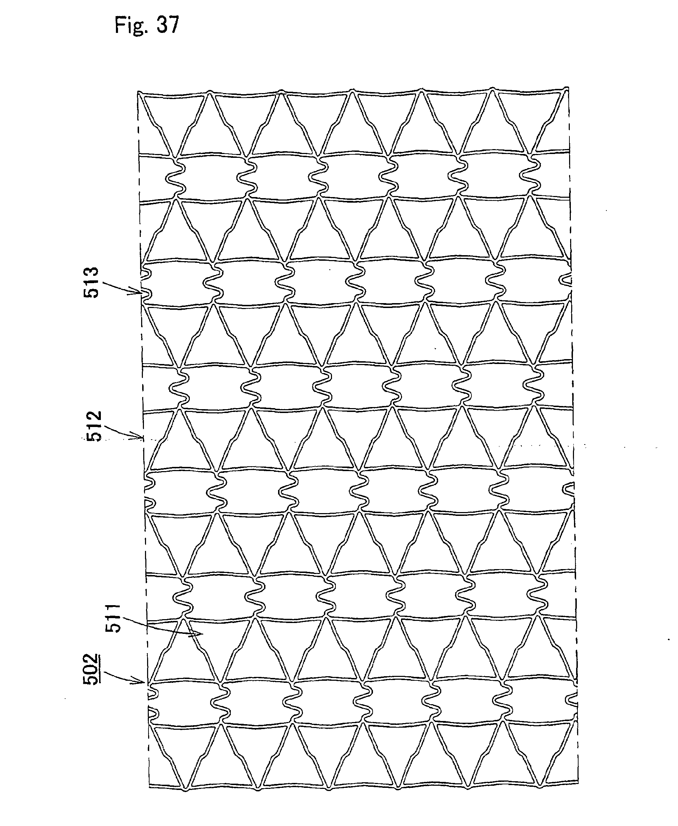

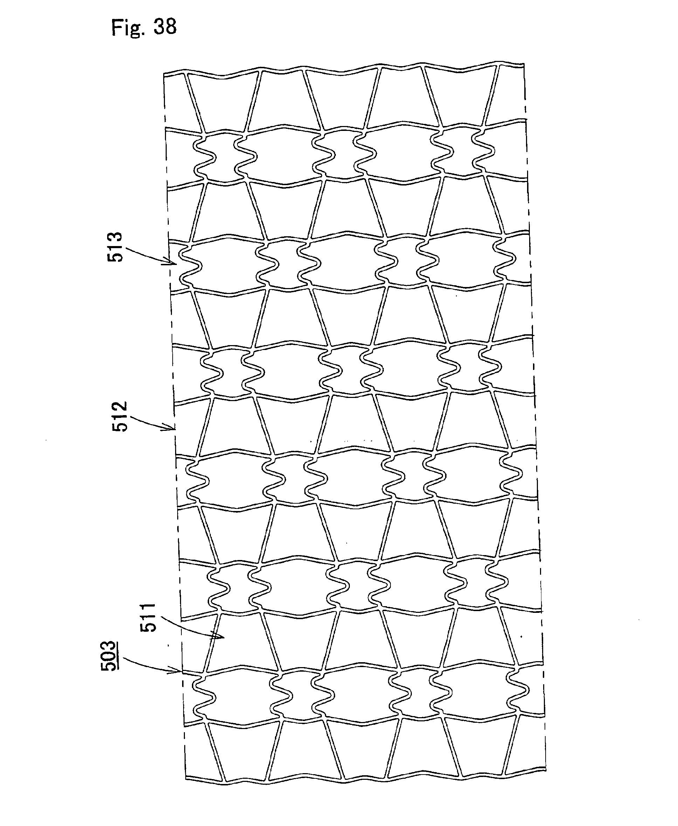

Figs. 35 and 36 show stents 502 and 503 of other embodiments; here, the basic cells 511 at the time of expansion respectively form substantially triangular shapes and substantially trapezoidal shapes. Figs. 37 and 38 are development views of these stents 502 and 503 in the expanded state. In these examples, in cases where the respective basic cells 511 are continuously connected in the circumferential direction, it is necessary to continuously connect the basic cells so that desired shapes alternate with respect to the axial direction of the stent, i. e., so that the vertices and bottom sides of the substantially triangular shapes alternate in cases where the basic cells at the time of expansion form substantially triangular shapes (Figs. 35 and 37), or so that the upper bottoms and lower bottoms of the substantially trapezoidal shapes alternate in cases where the basic cells at the time of expansion form substantially trapezoidal shapes (Figs. 36 and 38). Accordingly, the number of basic cells in one circumference, i. e., the number of basic cells present within one band part, must be an even number. In this case, this can be realized by balancing the desired diameter of the stent at the time of expansion and the lengths of the respective struts of the basic cells within the abovementioned conditions. Furthermore, in the case of stents in which the basic cells 511 at the time of expansion thus form substantially triangular shapes or substantially trapezoidal shapes, the contract of the stent in the axial direction at the time of expansion of the stent will be increased if the angle formed by the main struts 514 with the axial direction of the stent is large, so that positioning and the like at the time of placement of the stent becomes difficult. In order to reduce the contraction of the stent in the axial direction at the time of expansion of the stent, it is important that the angel formed by the main struts 514 and the axial direction of the stent be reduced to a small angle; in practical terms, it is desirable that this angle be 30° or less. In cases where the angle formed by the main struts 514 and the axial direction of the stent is 0°, the contraction in the longitudinal direction at the time of expansion of the stent is zero; in this case, the shape of the basic cells 511 is a substantially square shape. In cases where the shape of the basic cells 511 at the time of expansion of the stent is a substantially triangular shape, it is difficult to balance the radial force and the contraction in the longitudinal direction at the time of expansion of the stent; accordingly, it is most desirable that the shape of the basic cells at the time of expansion of the stent be close to a true triangular shape. In this case, the rate of contraction of the basic cells 511 in the longitudinal direction is approximately 15%.

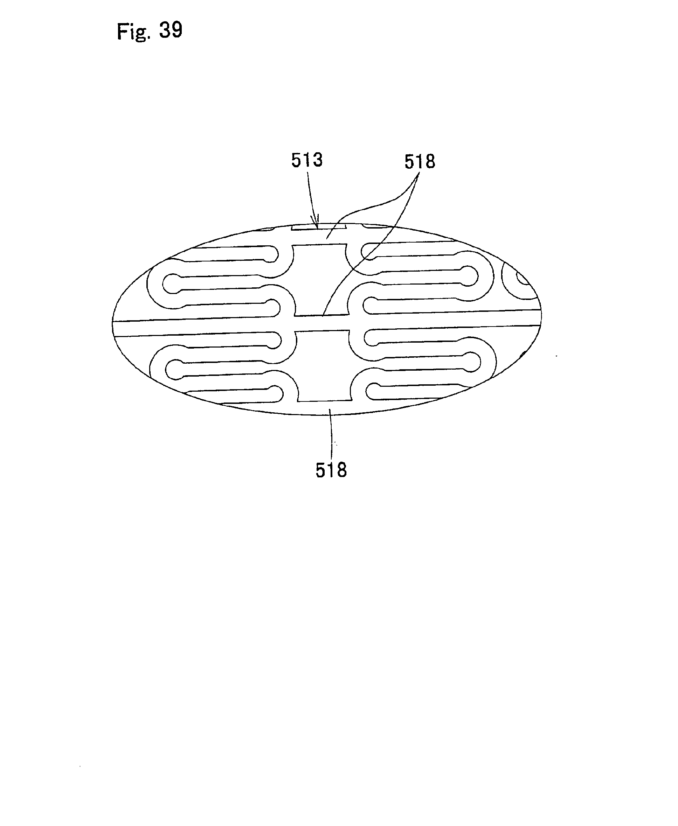

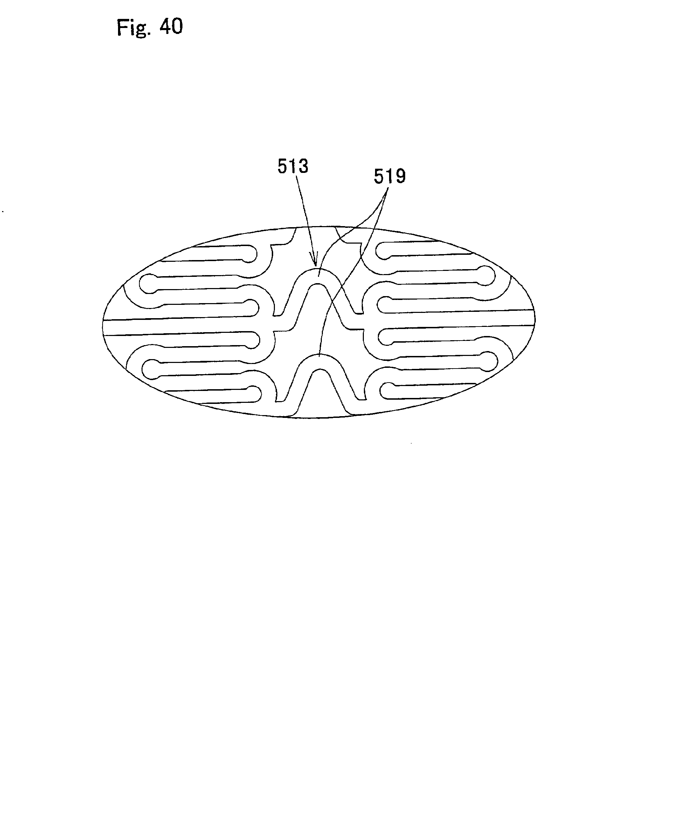

Fig. 39 is a diagram which sows the links 518 inside the link parts 513 that continuously connect the band parts 512 formed by the continuous connection of the basic cells 511. It is desirable that these links 518 have the function of expanding or contracting in the longitudinal direction in order to conform to bent vascular tissues when the stent is transported to a desired position through such bent vascular tissues. Figs. 40 through 43 show examples in which there is at least one bent part 519 within each link 518, thus endowing the links 518 with the function of expanding or contracting in the longitudinal direction, so that the links can conform to such bent vascular tissues.

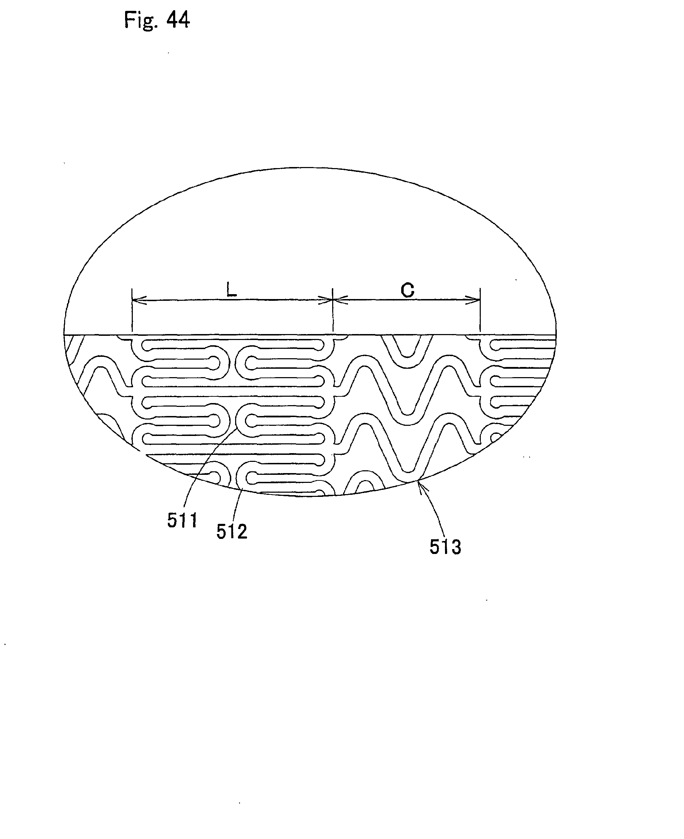

Fig. 44 is a diagram which shows the relationship between the width of the band parts 512 formed by the continuous connection of the basic cells 511, i. e., the length L o the basic cells 511 in the longitudinal direction, and the length C of the link parts 513 in the longitudinal direction. In order to allow the stent to conform to bent vascular tissues, a longer length of the link parts 513 is desirable, since this makes it possible to increase the flexibility; however, since the radial force is not generated by the link parts alone, a shorter length is desirable if only the radial force is considered. As a result of these considerations, it is desirable that the length C o the link parts 513 be such that 0.3 × L ≤C ≤ 2 × L. In cases where C is smaller than 0.3 × L, sufficient flexibility cannot be obtained, so that the ability to conform to bent vascular tissues drops. On the other hand, in cases where C is greater than 2 × L, the problems of the weakening of the radial force of the link parts 513 and the reduction of the expanded diameter of the link parts become more conspicuous.

The stents described above, and especially the main struts 514 and sub-struts 515, can be constructed from a metal or polymer material that can ensure the abovementioned radial force. Examples of metal materials that can be used include stainless steel (as represented by SUS316) and super-elastic metals such as Ni-Ti alloys. In cases where such materials are used, the pattern of the stent can generally be worked using a YAG laser while controlling the position of the worked stent by means of a computer. Furthermore, examples of polymer materials that can be used include thermoplastic polymers, thermo-setting polymers, biodegradable polymers and the like. In order to function as a stent, it is desirable that the material used be a material with a bending elastic modulus of 1 GPa or greater. A material with a bending elastic modulus of 2 GPa or greater is even more desirable; a higher bending elastic modulus makes it possible to reduce the width and thickness of the struts of the stent. Furthermore, in the case of stents, there may be instances in which degradation and absorption of the stent are desired following placement in the body or after a fixed period of time has elapsed. In such cases, biodegradable polymer materials can be used. Concrete examples of biodegradable polymers that can be used as stents include polylactic acids, polyglycolic acids, poly(ε-caprolactones) and the like. In cases where such polymer materials are used as stent raw materials, burning may occur in the laser-irradiate portions, or portions where melted material is piled up may be generated in the vicinity of the laser-irradiated portions, if working is performed using a YAG laser or carbon dioxide gas laser. Accordingly, it is most ideal to use an excimer laser.

In cases where stents are worked using these materials, the diameter of the tubular member constituting the raw material differs according to whether the stent is a stent of the type that is expanded by a balloon or a stent of the type that is self-expanding. Specifically, if the stent is a stent that is expanded by a balloon, a tubular member with a diameter which is equal to or greater than the folded diameter of the balloon on which the stent is mounted, but smaller than the desired stent diameter, is generally used as the raw material. On the other hand, if the stent is a stent of the self-expanding type, a tubular member with a diameter that is close to the desired stent diameter may be worked as the raw material.

Generally, stents are used to expand constricted parts or blocked parts of vascular tissues in the body. However, in an application that differs from such purposes, a treatment to block perforations using a stent in which a thin film cover is formed on the outer surface of the stent may be performed as an emergency measure in cases where such perforations have bee formed in vascular tissues, e. g., in cases where perforations have been formed in blood vessels. In such cases as well, a thin-film polymer material may be applied to the outer surface of the present stent and used. Examples of thin-film polymer materials that can be used include Teflon type resins, silicone resins and the like; materials with a large elongation can be used.

Furthermore, in order to allow the use of these stents under X-ray imaging, it is ordinarily desirable that the stents have an X-ray impermeable marker that can be used to grasp the position of the stent. Examples of X-ray impermeable markers that can be used include gold, platinum, platinum-rhodium alloys and the like. It is desirable that such an X-ray impermeable marker be present on both end portions of the stent. In regard to the method used to apply the marker, the application of a marker by means of plating van be used in the case of stents make of metal materials, besides fastening using physical methods.

Furthermore, drugs used to prevent re-constriction or suppress the formation of thrombi, as well as therapeutic genes, may be added or applied as surface treatments to the abovementioned stents.

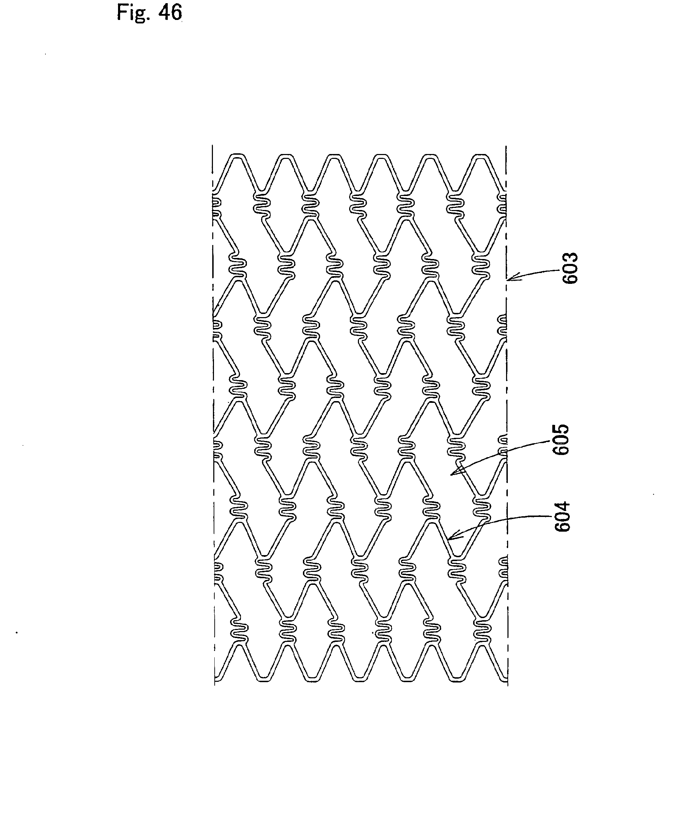

Figs. 45 through 50 show a stent of the third invention of the present application. Fig. 46 is a development view of an embodiment (Embodiment 1) of the stent 603 of the present invention. The stent 603 is a stent which is formed as a substantially tubular body, and which can be expanded outward in the radial direction of this substantially tubular body. Furthermore, this is a stent in which a plurality of patterns 605 forming more or less polygonal shapes surrounded by struts 604 that are linear elements are lined up in the circumferential direction and axial direction. In regard to the term "more or less polygonal shape", this polygonal shape includes triangular shapes, lozenge shapes, oblong shapes, parallelogram shapes, pentagonal shapes and any other polygonal shapes. Furthermore, this term includes shapes in which the corners are not sharp corners, but rather have a curved rounding.

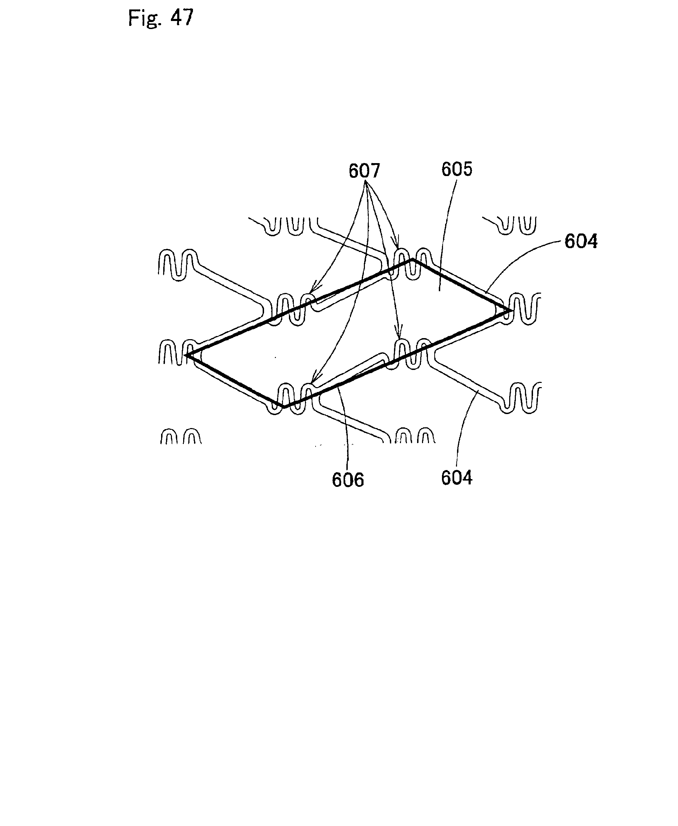

The abovementioned polygonal shape patterns 605 have an original linear peripheral length. This original linear peripheral length is the length indicated by the thick lines 606 in Fig. 47, and indicates the so-called linear minimum peripheral length of the polygonal shape patterns without taking into account the bent total length of the local folded portions 607 that are capable of elongating deformation (described later).

Furthermore, in the abovementioned polygonal shape patterns 605, the stent 603 of the present embodiment has 4 local folded portions 607 capable of elongating deformation per single polygonal shape pattern, so that when the polygonal shape patterns 605 are pushed open from the inside toward the outside by the expansion of the balloon of a stent delivery catheter or the like, the peripheral length following the pushing open of these shape patterns is increased to a value that is 1.3 to 2.0 times the original peripheral length.

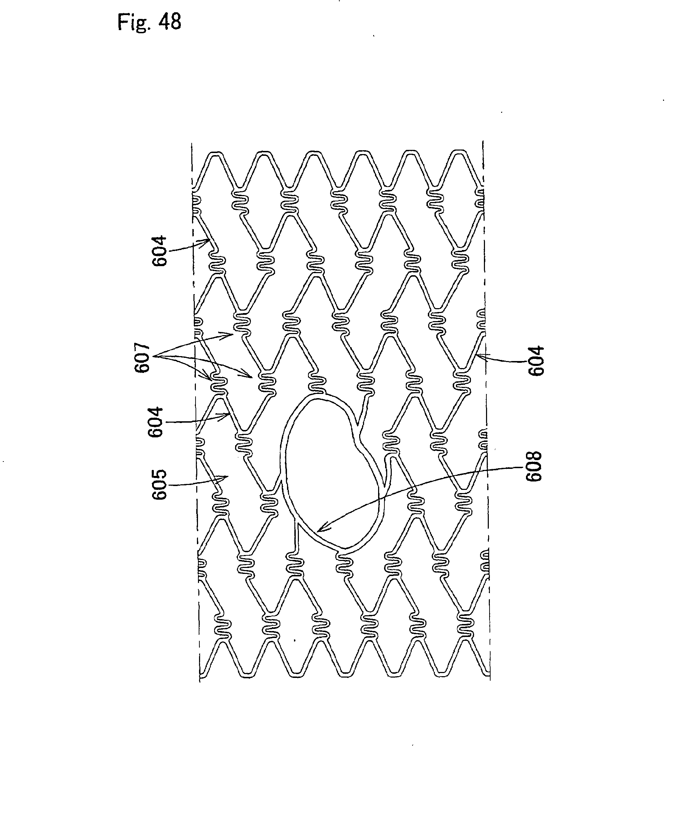

In the present embodiment, the shape of the local folded portions 607 that are capable of elongating deformation is a shape corresponding to two periods of a sine wave. However, as was described above, as long as the shape is a shape which is such that the peripheral length following the pushing open of the shape patterns can be increased to a value that is 1.3 to 2.0 times the original peripheral length 606 by pushing open the local folded portions from the inside toward the outside by means of the expansion of the balloon of a stent delivery catheter or the like in the abovementioned polygonal shape patterns 605, this shape of the local folded portions may be any desired shape. Meanwhile, the "peripheral length following the pushing open of the polygonal shape pat terns" refers to the peripheral length of the pattern formed by the expansion of one of the original linear peripheral lengths as indicated by 608 in Fig. 48.

Here, the reason that the peripheral length 608 following the pushing open of the polygonal shape patterns that is obtained by pushing open these polygonal shape patterns by the expansion of the balloon of a stent delivery catheter or the like is set at 1.3 times to 2.0 times the original linear peripheral length 606 is as follows: specifically, in cases where the original linear peripheral length 606 of the abovementioned polygonal shape patterns 605 is selected in the range described below and the number of local folded portions 607 (capable of elongating deformation) per single polygonal shape pattern 605 is set at 3 or greater in order to obtain a high radial force and satisfy the requirement for superior scaffold properties, an opening part area/peripheral length sufficient to allow access to side branches can be obtained if the peripheral length 608 that is obtained following pushing open by means of the expansion of the balloon of a stent delivery catheter or the like is increased to a value in the range of 1.3 times to 2.0 times the original linear peripheral length 606.

It is desirable that the number of local folded portions 607 capable of elongating deformation per one polygonal shape pattern part 605 be 3 or greater. However, it is even more desirable that this number be the same as the number of sides of the polygonal shape. The reason for this is as follows: namely, in order to make the expansion operation, radial force, and flexibility uniform throughout the entire stent 603, it is best to imitate the structure of the respective sides of the polygonal shapes as closely as possible. In this embodiment, since the parallelogram shapes have four sides, the number of local folded portions 607 that are capable of elongating deformation is 4.

' Furthermore, in order to obtain a high radial force and satisfy the requirement for superior scaffold properties, it is desirable that the original peripheral length 606 o the abovementioned polygonal shape patterns 605 be 6.0 mm to 12.0 mm, and a length of 8.0 mm to 10.0 mm is even more desirable.

For the same reasons, it is desirable that the original area of the opening parts of the abovementioned polygonal shape patterns 605 be 2.0 mm² to 9.0 mm² following the expansion of the stent, and an area of 3.0 mm² to 6.25 mm² is even more desirable.

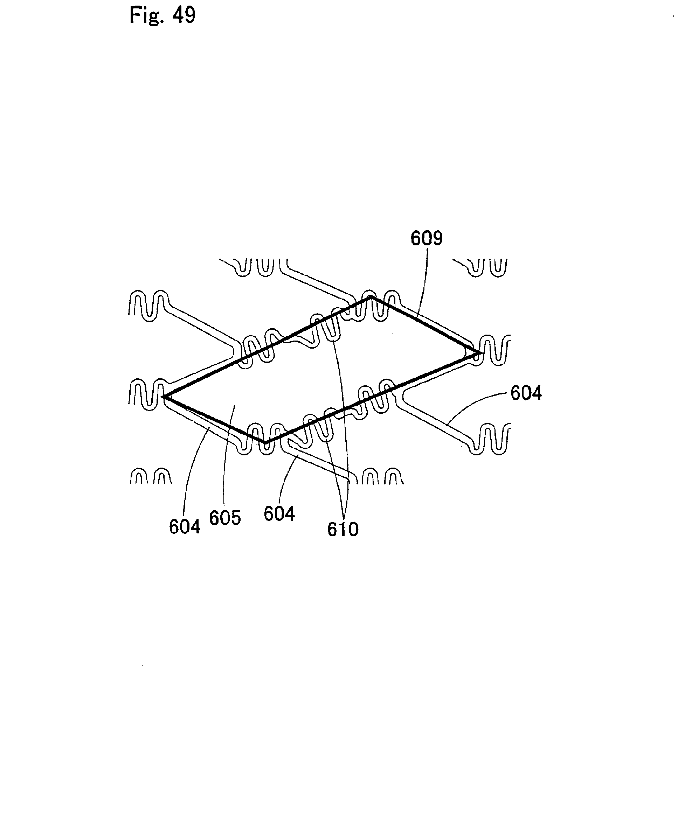

Fig. 49 shows another embodiment in which the folded portions that are capable of elongating deformation have a different construction from that in the preceding embodiment. Here, the construction of the folded portions that are capable of elongating deformation merely differs from that in the preceding embodiment; the abovementioned polygonal shape patterns 605 are shown.

Specifically, the stent 603 of the present embodiment is a stent which is formed as a substantially tubular member, and which can be expanded outward in the radial direction of this substantially tubular member. In this stent, a plurality of patterns 605 which have a more or less polygonal shape surrounded by struts 604 constituting linear elements are lined up in the circumferential direction and axial direction. Furthermore, in regard to the term "more or less polygonal shape", this polygonal shape includes triangular shapes, lozenge shapes, oblong shapes, parallelogram shapes, pentagonal shapes and any other polygonal shapes. Furthermore, this term includes shapes in which the corners are not sharp corners, but rather have a curved rounding.

The abovementioned polygonal shape patterns 605 have an original linear peripheral length. This original linear peripheral length is the length indicated by the thick lines 609 in Fig. 49, and indicates the so-called linear minimum peripheral length of the polygonal shape patterns without taking into account the bent total length of the local folded portions 610 that are capable of elongating deformation (described later).

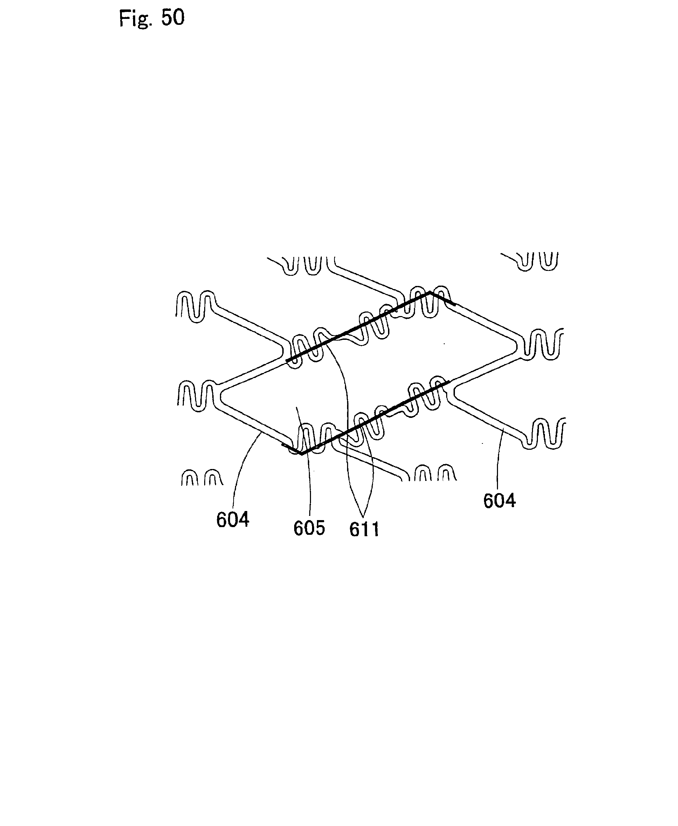

Furthermore, in the abovementioned polygonal shape patterns 605, the stent 603 of the present embodiment has a relatively large number of local folded portions 610 capable of elongating deformation, so that when the polygonal shape patterns 605 are pushed open from the inside toward the outside by the expansion of the balloon of a stent delivery catheter or the like, the peripheral length 608 following the pushing open of these shape patterns is increased to a value that is 1.3 to 2.0 times the original peripheral length 609. These are formed so that the total of the linear lengths of the local folded portions 610 (capable of elongating deformation) in the directions of the sides of the polygonal shapes is 1/3 times to 1 time the original linear peripheral length 609 of the polygonal shape patterns 605. The definition of the linear lengths of the abovementioned local folded portions 610 (capable of elongating deformation) in the directions of the sides of the polygonal shapes is as indicated by the thick lines 611 shown in Fig. 50 (this refers to the total of the thick line portions).

In the present embodiment, the shape of the local folded portions 610 that are capable of elongating deformation is a shape corresponding to two periods of a sine wave. However, as was described above, as long as the shape is a shape which is such that the peripheral length following the pushing open of the shape patterns can be increased to a value that is 1.3 to 2.0 times the original peripheral length 609 by pushing open the local folded portions from the inside toward the outside by means of the expansion of the balloon of a stent delivery catheter or the like in the abovementioned polygonal shape patterns 605, this shape of the local folded portions may be any desired shape. In the present embodiment, the linear length 611 of the local folded portions 610 (that are capable of elongating deformation) in the directions of the sides of the abovementioned polygonal shape patterns 605 is selected so that this length is slightly greater than 1/2 the original linear peripheral length 609 of the abovementioned polygonal shape patterns; however, as long as this value is in the range of 1/3 times to 1 time the original linear peripheral length, any multiple may be used. Furthermore, the abovementioned folded portions 610 that are capable of elongating deformation may be continuously connected on the periphery of the abovementioned polygonal shapes, or a plurality of discontinuous portions may be used.

Here, the reason that the linear length 611 of the abovementioned local folded portions 610 that are capable of elongating deformation is set at 1/3 times to 1 time the original linear peripheral length 609 of the abovementioned polygonal shape patterns 605 is as follows: namely, assuming as a prerequisite the fact that the original peripheral length 609 of the abovementioned polygonal shape pattern parts 605 is selected in the range described below in order to obtain a high radial force and satisfy the requirement for superior scaffold properties, and that the peripheral length 608 following pushing open that is obtained by pushing open the polygonal shape pattern parts by the expansion of the balloon of a sent delivery catheter or the like is 1.3 times to 2.0 times the abovementioned original linear peripheral length 609, then an opening part area/peripheral length that is sufficient to allow access to side branches can be obtained following the pushing open of the polygonal shape pattern parts if the original linear length 611 of the local folded portions 610 capable of elongating deformation (per single polygonal shape pattern part 605) in the directions of the sides of the abovementioned polygonal shape is in the range of 1/3 times to 1 time the original linear peripheral length 609 of the abovementioned polygonal shape patterns 605.

Furthermore, the portions of the abovementioned folded portions 610 capable of elongating deformation that bulge out in the axial direction (differing from the directions of the sides of the polygonal shapes) are not formed as large portions. The reason for this is as follows: namely, in cases where the stent is contracted and pre-mounted on the balloon of a stent delivery catheter, these bulging portions overlap with other local folded portions (capable of elongating deformation) of polygonal shapes that are adjacent in the circumferential direction, so that pre-mounting cannot be accomplished while maintaining a sufficiently small external diameter (low profile). In this sense as well, the range indicated by the abovementioned numerical values is important.

Furthermore, in order to obtain a high radial force and satisfy the requirement for superior scaffold properties, it is desirable that the original peripheral length 609 of the abovementioned polygonal shape patterns 605 be 6.0 mm to 12.0 mm, and a length of 8.0 mm to 10.0 mm is even more desirable.

For the same reasons, it is desirable that the original area of the opening parts of the abovementioned polygonal shape patterns 605 be 2.0 mm² to 9.0 mm² following the expansion of the stent, and an area of 3.0 mm² to 6.25 mm² is even more desirable.

The stents disclosed in the two embodiments described above can maintain a high radial force, and at the same time have superior scaffold properties, as a result of having a plurality of polygonal shape patterns 605 in the circumferential direction and axial direction. Furthermore, as a result of three or more local portions 607 capable of elongating deformation being installed in the respective polygonal shape patterns in the embodiment shown in Figs. 46 through 48, so that the peripheral length 608 following pushing open can be increased to a value that is 1.3 times to 2.0 times the abovementioned original linear peripheral length 606 or 609 by pushing open the abovementioned polygonal shape pattern parts 605 from the inside toward the outside by means of the expansion of the balloon of a stent delivery catheter or the like, and as a result of the linear length 611 of the local portions 610 capable of elongating deformation being set at 1/3 times to 1 time the original linear peripheral length 609 of the polygonal shape patterns 605 in the embodiment shown in Figs. 49 and 50, the peripheral length of the abovementioned polygonal shape patterns 605 can be greatly deformed by expanding the balloon of another stent delivery catheter disposed in a side branch via the polygonal shape patterns; accordingly, access to branched blood vessels and Y stenting are possible.

### INDUSTRIAL APPLICABILITY

As was described above, the stent of the first invention of the present application is flexible in the axial direction, and there is no contraction in the axial length of the stent at the time of expansion. The resistance to forces that tend to cause contraction of the blood vessel is extremely large, and the struts of the stent can be uniformly expanded. Furthermore, the problem of both end portions of the stent warping to a larger diameter than the central portion at the time of expansion does not occur.

Furthermore, in the case of the stent of the second invention, the stent can be uniformly expanded at the time of expansion of the stent, and while excessive expansion can be suppressed, the size of the basic cells that form the stent can be reduced to a very small size, so that a stent can be provided in which the bulging of endothelial cells of vascular tissues into the inside of the stent is suppressed, thus reducing re-constriction of the vascular tissues.

Furthermore, the stent of the third invention provides a closed type stent which eliminates the disadvantages of a closed type stent while maintaining the advantages of such a stent, and which has only the advantages of an open type stent while being free of the disadvantages of such an open type stent, i. e., a closed type stent which allows Y stenting in branched blood vessels while maintaining a high radial force and superior scaffold properties.

## Claims

1. A stent which is formed as a substantially tubular body, and which can be expanded outward in the radial direction of this substantially tubular body, **characterized in that**:
the stent 101 comprises circumferentially extendable substantially wave-form constituent elements 102 and axially extendable substantially wave-form constituent elements 103;
a plurality of the circumferentially extendable substantially wave-form constituent elements 102 are disposed substantially in the circumferential direction of the stent 101 without being directly connected to each other;
a plurality of said axially extendable substantially wave-form constituent elements 103 are disposed substantially in the circumferential direction of the stent 101 without being directly connected to each other; and
these constituent elements 102 and 103 are arranged alternately in periodic series in the axial direction of the stent.

2. A stent which is formed as a substantially tubular body, and which can be expanded outward in the radial direction of this substantially tubular body, **characterized in that**:
said stent 101 comprises circumferentially extendable substantially wave-form constituent elements 102 and axially extendable substantially wave-form constituent elements 103;
a connecting part 127 on one end of said circumferentially extendable substantially wave-form constituent element 102 is connected with a connecting part 131 on one end of said axially extendable substantially wave-form constituent element 103, and a connecting part 139 on the remaining end of said axially extendable substantially wave-form constituent element 103 is connected with a connecting part 121 on the opposite end from the connecting part 127 of another said circumferentially extendable substantially wave-form constituent element 102, so that said circumferentially extendable substantially wave-form constituent elements 102 and the axially extendable substantially wave-form constituent elements 103 are connected periodically; and
furthermore, a connecting part 123 on a peak or valley protruding part of said circumferentially extendable substantially wave-form constituent element 102 is connected with a connecting part 131 on one end of said axially extendable substantially wave-form constituent element 103, and a connecting part 139 on the remaining end of the axially extendable substantially wave-form constituent element 103 is connected with a connecting part 125 on the peak or valley protruding part present on the opposite side from said connecting part 123 of another said circumferentially extendable substantially wave-form constituent elements 102, whereby said circumferentially extendable substantially wave-form constituent elements 102 and the axially extendable substantially wave-form constituent elements 103 are formed in periodic series.

3. The stent according to claim 1 or claim 2, wherein said circumferentially extendable substantially wave-form constituent elements 102 have a substantially wave-form shape in which the direction of progression of the wave is the circumferential direction of the stent, and in which the combined number of peak and valley vertices is 2 or greater, and said axially extendable substantially wave-form constituent elements 103 have a substantially wave-form shape in which the direction of progression of the wave is the axial direction of the stent, and in which the combined number of peak and valley vertices is 1 or greater.

4. The stent according to claim 3, wherein said circumferentially extendable substantially wave-form constituent elements 102 have a substantially wave-form shape which has peak and valley vertices at the end portions thereof, and in which the combined number of peak and valley vertices including those at the end portions is 4, and said axially extendable substantially wave-form constituent elements 103 have a substantially wave-form shape in which the direction of progression of the wave is the axial direction of the stent, and in which the combined number of peak and valley vertices is 4.

5. A stent which is formed as a substantially tubular body, and which can be expanded outward in the radial direction of this substantially tubular body, **characterized in that**:
said stent 10 comprises circumferentially extendable substantially wave-form constituent elements 102 and axially extendable substantially wave-form constituent elements 103;
connecting parts 127 on said substantially wave-form constituent elements 102 are connected with connecting parts 131 on said substantially wave-form constituent elements 103, and connecting parts 139 on said substantially wave-form constituent elements 103 are connected with connecting parts 121 on said substantially wave-form constituent elements 102, so that said substantially wave-form constituent elements 102 and said substantially wave-form constituent elements 103 are arranged in periodic series;
furthermore, connecting parts 123 on said substantially wave-form constituent elements 102 are connected with connecting parts 131 on said substantially wave-form constituent elements 103, and connecting parts 125 on said substantially wave-form constituent elements 102 are connected with connecting parts 139 on said substantially wave-form constituent elements 103, so that said substantially wave-form constituent elements 102 and substantially wave-form constituent elements 103 are arranged in periodic series; and
all of the connecting parts 121, 123, 125, 127 on said substantially wave-form constituent elements 102 are connected to one of the connecting parts 131 and 139 on said substantially wave-form constituent elements 103, and all of the connecting parts 131, 139 on said substantially wave-form constituent elements 103 are connected to one of the connecting parts 121, 123, 125 and 127 on said substantially wave-form constituent elements 102.

6. The stent according to any of claims 1 through 5, wherein circumferential elements 104, that are formed by said circumferentially extendable substantially wave-form constituent elements 102 disposed in the circumferential direction without being directly connected to each other, are disposed with a specified angular shift relative to adjacent circumferential elements 104, and said circumferential elements are connected to each other via said axially extendable substantially wave-form constituent elements 103.

7. The stent according to any of claims 1 through 6, wherein only the axially opposite ends of the stent is formed by arranging a plurality of said circumferentially extendable substantially wave-form constituent elements 102 that are directly connected to each other, along the circumferential direction of the stent.

8. The stent according to claim 7, wherein said circumferentially extendable substantially wave-form constituent elements 102 that constitute the axially opposite ends of the stent satisfy one or more of the following conditions when compared to said circumferentially extendable substantially wave-form constituent elements 102 that constitute other portions of the stent than the axially opposite ends of the stent:
(1) The length in the axial direction of the stent is shorter;
(2) The width of the struts is greater; and
(3) The thickness of the struts is greater.

9. The stent according to claim 7, wherein at least either said circumferentially extendable substantially wave-form constituent elements 102 or said axially extendable substantially wave-form constituent elements 103 satisfy one or more of the following conditions as the positions of these elements shift from the central portion to the axially opposite ends of the stent:
(1) The length in the axial direction of the stent becomes shorter in steps;
(2) The width of the struts becomes greater in steps; and
(3) The thickness of the struts becomes greater.

10. The stent according to any of claims 1 through 6, **characterized in that** the circumferential elements 104, which are constituted by said circumferentially extendable substantially wave-form constituent elements 102 disposed substantially in the circumferential direction of the stent without being directly connected to each other, are provided at the rate of 5 or more per 10 mm of the length of the stent in the axial direction.

11. A stent which is formed as a substantially tubular body, and which can be expanded outward in the radial direction of this substantially tubular body, **characterized in that**:
said stent 201 comprises circumferentially extendable substantially wave-form constituent elements 202, circumferentially extendable substantially wave-form constituent elements 203 and axially extendable substantially wave-form constituent elements 204;
a plurality of said circumferentially extendable substantially wave-form constituent elements 202 are disposed substantially in the circumferential direction of the stent without being directly connected to each other, a plurality of said circumferentially extendable substantially wave-form constituent elements 203 are disposed substantially in the circumferential direction of the stent without being directly connected to each other, a plurality of said axially extendable substantially wave-form constituent elements 204 are disposed substantially in the circumferential direction of the stent without being directly connected to each other, and these elements are arranged alternately in periodic series in the axial direction of the stent.

12. A stent which is formed as a substantially tubular body, and which can be expanded outward in the radial direction of this substantially tubular body, **characterized in that**:
said stent 201 comprises circumferentially extendable substantially wave-form constituent elements 202, circumferentially extendable substantially wave-form constituent elements 203 and axially extendable substantially wave-form constituent elements 204;
the stent 201 further comprises circumferential elements 205 which are formed by disposing a plurality of said substantially wave-form constituent elements 202 that are not directly connected to each other, in the circumferential direction of the stent, circumferential elements 206 which are formed by disposing a plurality of said substantially wave-form constituent elements 203 that are not directly connected to each other, in the circumferential direction of the stent, and circumferential elements 207 that are formed by disposing a plurality of said substantially wave-form constituent elements 204 that are not directly connected to each other, in the circumferential direction of the stent; and
the circumferential elements 205, 207, 206, 207 are arranged in periodic series **in that** order in the axial direction of the stent.

13. A stent which is formed as a substantially tubular body, and which can be expanded outward in the radial direction of this substantially tubular body, **characterized in that**:
said stent comprises circumferentially extendable substantially wave-form constituent elements 202, circumferentially extendable substantially wave-form constituent elements 203, and axially extendable substantially wave-form constituent elements 204;
a connecting part 227 on one end of said circumferentially extendable substantially wave-form constituent element 202 is connected with a connecting part 241 on one end of said axially extendable substantially wave-form constituent element 204, a connecting part 249 on the remaining end of said axially extendable substantially wave-form constituent element 204 is connected with a connecting part 233 on a peak or valley protruding part of said circumferentially extendable substantially wave-form constituent element 203, a connecting part 235 on a peak or valley protruding part present on the opposite side from said connecting part 233 of said circumferentially extendable substantially wave-form constituent element 203 is connected with the connecting part 241 on one end of another circumferentially extendable substantially wave-form constituent element 204, and the connecting part 249 on the remaining end of said axially extendable substantially wave-form constituent element 204 is connected with a connecting part 221 on the opposite end from said connecting part 227 of another circumferentially extendable substantially wave-form constituent element 202, so that said circumferentially extendable substantially wave-form constituent elements 202, said circumferentially extendable substantially wave-form constituent elements 203 and said axially extendable substantially wave-form constituent elements 204 are arranged in periodic series; .
furthermore, a connecting part 223 on a peak or valley protruding part of said circumferentially extendable substantially wave-form constituent element 202 is connected with the connecting part 241 on one end of the axially extendable substantially wave-form constituent elements 204, the connecting part 249 on the remaining end of said axially extendable substantially wave-form constituent element 204 is connected with a connecting part 237 on one end of said circumferentially extendable substantially wave-form constituent element 203, a connecting part 231 on the opposite end from said connecting part 237 of said circumferentially extendable substantially wave-form constituent element 203 is connected with the connecting part 241 on one end of another axially extendable substantially wave-form constituent'element 204, and the connecting part 249 on the remaining end of said axially extendable substantially wave-form constituent element 204 is connected with a connecting part 225 on a peak or valley protruding part on the opposite side from said connecting part 223 of another circumferentially extendable substantially wave-form constituent elements 202, so that said circumferentially extendable substantially wave-form constituent elements 202, said circumferentially extendable substantially wave-form constituent elements 203 and said axially extendable substantially wave-form constituent elements 204 are arranged in periodic series; and
whereby a stent is formed in which said circumferentially extendable substantially wave-form constituent elements 202, said circumferentially extendable substantially wave-form constituent elements 203 and said axially extendable substantially wave-form constituent elements 204 are arranged in periodic series.

14. The stent according to any of claims 11 through 13, wherein said circumferentially extendable substantially wave-form constituent elements 202 and substantially wave-form constituent elements 203 have a substantially wave-form shape in which the direction of progression of the wave is the circumferential direction of the stent, and in which the combined number of peak and valley vertices is 2 or greater, and said axially extendable substantially wave-form constituent elements 204 have a substantially wave-form shape in which the direction of progression of the wave is the axial direction of the stent, and in which the combined number of peak and valley vertices is 1 or greater.

15. The stent according to claim 14, wherein said circumferentially extendable substantially wave-form constituent elements 202 and substantially wave-form constituent elements 203 have a substantially wave-form shape which has peak and valley vertices on the end portions thereof, and in which the combined number of peak and valley vertices including the end portions is 4, and said axially extendable substantially wave-form constituent elements 204 have a substantially wave-form shape in which the direction of progression of the wave is the axial direction of the stent, and in which the combined number of peak and valley vertices is 4.

16. The stent according to any of claims 11 through 15, wherein said circumferentially extendable substantially wave-form constituent elements 202 and substantially wave-form constituent elements 203 have shapes that show linear symmetry with respect to each other.

17. A stent which is formed as a substantially tubular body, and which can be expanded outward in the radial direction of this substantially tubular body, **characterized in that**:
said stent 201 comprises circumferentially extendable substantially wave-form constituent elements 202 and substantially wave-form constituent elements 203, and axially extendable substantially wave-form constituent elements 204;
said substantially wave-form constituent elements 202 are constituted by connecting parts 221, 223, 225, 227 and linear parts 222, 224, 226;
said substantially wave-form constituent elements 203 are constituted by connecting parts 231, 233, 235, 237 and linear parts 232, 234, 236;
said substantially wave-form constituent elements 204 are constituted by connecting parts 241, 249, bent parts 242, 244, 246, 248, and linear parts 243, 245, 247;
the connecting parts 227 of said substantially wave-form constituent elements 202 are connected with the connecting parts 241 of said substantially wave-form constituent elements 204, the connecting parts 249 of said substantially wave-form constituent elements 204 are connected with the connecting parts 233 of said substantially wave-form constituent elements 203, the connecting parts 235 of said substantially wave-form constituent elements 203 is connected with the connecting pats 241 of said substantially wave-form constituent elements 204, and the connecting parts 249 of said substantially wave-form constituent elements 204 are connected with the connecting parts 221 of said substantially wave-form constituent elements 202, so that said substantially wave-form constituent elements 202, said substantially wave-form constituent elements 203 and said substantially wave-form constituent elements 204 are arranged in periodic series;
furthermore, the connecting parts 223 of said substantially wave-form constituent elements 202 are connected with the connecting parts 241 of said substantially wave-form constituent elements 204, the connecting parts 249 of said substantially wave-form constituent elements 204 are connected with the connecting parts 237 of said substantially wave-form constituent elements 203, the connecting parts 231 of said substantially wave-form constituent elements 203 are connected with the connecting parts 241 of said substantially wave-form constituent elements 204, and the connecting parts 249 of said substantially wave-form constituent elements 204 are connected with the connecting parts 225 of said substantially wave-form constituent elements 202, so that said substantially wave-form constituent elements 202, said substantially wave-form constituent elements 203 and said substantially wave-form constituent elements 204 are arranged in periodic series; and
all of the connecting parts 221, 223, 225, 227 of said substantially wave-form constituent elements 202 are connected to either the connecting parts 241 or 249 of said substantially wave-form constituent elements 204, all of the connecting parts 231, 233, 235, 237 of said substantially wave-form constituent elements 203 are connected to either the connecting parts 241 or 249 of said substantially wave-form constituent elements 204, and all of the connecting parts 241, 249 of said substantially wave-form constituent elements 204 are connected to one of the connecting parts 221, 223, 225, 227, 231. 233, 235 and 237 of said substantially wave-form constituent elements 202 or said substantially wave-form constituent elements 203.

18. The stent according to any of claims 11 through 17, **characterized in that** only the axially opposite ends of the stent are formed by disposing along the circumference of the stent a plurality of said circumferentially extendable substantially wave-form constituent elements 202 and/or substantially wave-form constituent elements 203 which are directly connected to each other.

19. The stent according to claim 18, wherein the circumferentially extendable substantially wave-form constituent elements 202 and/or substantially wave-form constituent elements 203 which constitute the axially opposite ends of the stent satisfy one or more of the following conditions compared to the circumferentially extendable substantially wave-form constituent elements 202 and/or substantially wave-form constituent elements 203 which constitute the portions of the stent other than the axially opposite ends of the stent:
(1) The length in the axial direction of the stent is shorter.
(2) The width of the struts is greater.
(3) The thickness of the struts is greater.

20. The stent according to claim 18, wherein one or more types of elements selected from said circumferentially extendable substantially wave-form constituent elements 202 and substantially wave-form constituent elements 203 and said axially extendable substantially wave-form constituent elements 204 satisfy one or more of the following conditions as the positions of these elements shift from the central portion to the end portions with respect to the axial direction of the stent:
(1) The length in the axial direction of the stent becomes shorter in steps.
(2) The width of the struts becomes greater in steps.
(2) The thickness of the struts becomes greater.

21. The stent according to any of claims 11 through 20, wherein circumferential elements 205 or circumferential elements 206, which are formed by said circumferentially extendable substantially wave-form constituent elements 202 or substantially wave-form constituent elements 203 being disposed substantially in the circumferential direction of the stent without being directly connected to each other, are provided at the rate of 5 or more per 100 mm of length of the stent.

22. A stent which is formed as a substantially tubular body and which can be expanded in the radial direction from the inside of this substantially tubular body, **characterized in that** the basic elements that constitute said stent 301, 303 or 305 have a substantially parallelogram shape.

23. The stent according to claim 22, wherein the substantially parallelogram-shaped elements 302, 304, 306 are combined together in an alternately oriented fashion.

24. The stent according to claim 22, wherein the substantially parallelogram-shaped elements 302, 304, 306 are combined together in an alternately oriented fashion, the respective sides of the substantially parallelogram-shaped elements 302, 304, 306 are substantially parallel to the axial direction of the stent prior to the expansion of the stent, and the respective sides of the substantially parallelogram-shaped elements 302, 304, 306 form an angle with respect to the axial direction of the stent following the expansion of the stent.

25. The stent according to any of claims 22 through 24, wherein the substantially parallelogram-shaped elements 304, 306 are constructed from substantially linear struts 321, 331 and substantially wave-form struts 322, 332.

26. The stent according to claim 25, wherein substantially parallelogram-shaped with different areas are disposed in the axial direction of the stent.

27. The stent according to claim 25 or claim 26, wherein different strut widths and/or strut thicknesses are combined in the axial direction of the stent.

28. A stent which is formed as a substantially tubular body, and which can be expanded outward in the radial direction of this substantially tubular body, **characterized in that**:
said stent 401, 402 comprises annular first substantially wave-form elements 411, 451 that can be expanded in the radial direction, link part elements 413, 453 that can be extended in the axial direction, and branch-form elements 412, 452 that extend from the first substantially wave-form elements 411, 451;
one end of the link part element 413, 453 is connected to the first substantially wave-form element 411, 451, the other end of the link part element 413, 453 is connected to one end of the branch-form element 412, 452, and the other end of the branch-form element 412, 452 is connected to the first substantially wave-form element 411, 451.

29. A stent which is formed as a substantially tubular body, and which can be expanded outward in the radial direction of this substantially tubular body, **characterized in that** said stent 403 comprises annular first substantially wave-form elements 481 that can be expanded in the radial direction, link part elements 483 that can be extended in the axial direction, branch-form elements 482 that extend from said first substantially wave-form elements 481, and substantially N-shaped elements 485.

30. The stent according to claim 28 or claim 29, wherein the opposite end portions of the stent comprise annular second substantially wave-form elements 414, 454, 484 that can be expanded in the radial direction.

31. The stent according to any of claims 28 through 30, wherein the stent is uniformly expanded into approximately the same shape except for the opposite end portions thereof.

32. A stent 501, 502 or 503 used for placement in vascular tissues inside body cavities, which is formed as a substantially tubular body, and which can be expanded outward in the radial direction of this substantially tubular body, **characterized in that** said stent has a structure that prevents excessive expansion to a diameter greater than the desired diameter.

33. The stent according to claim 32,
wherein the basic cells 511 that constitute the stent 501, 502 or 503 comprise main struts which are disposed with the longitudinal direction of said struts oriented in the axial direction of the stent when the stent is not expanded, and sub-struts 515 which are folded between the main struts 515 and which support the main struts 514 in the circumferential direction when the stent is expanded;
the main struts 514 and sub-struts 515 form annular substantially polygonal shapes comprising three or more sides at the time of expansion;
a plurality of these basic cells 511 are connected in the circumferential direction to form band parts 512; and
a plurality of these band parts 512 are connected in the longitudinal direction via link parts 513.

34. The stent according to claim 32 or 33, which satisfies the relationships π × D = 0.5 × A × sin θ × B and 60° s θ < 90°, where A is the overall length of the sub-struts 515 in one basic cell 511 folded between the main struts 514, B is the number of basic cells 511 within one band part 512 formed by the series of a plurality of basic cells 511 in the circumferential direction, and D is the desired expanded diameter of the stent.

35. The stent according to claim 34, which satisfies the relationship L ≤ A < 2 × L, where L is the length in the longitudinal direction of the main struts 514 within the basic cells 511 when the stent is not expanded.

36. The stent according to any of claims 32 through 35, which satisfies the relationship 0.5 × W ≤ T ≤ 3 × W, where W is the width of the wire material that constitutes the main struts 514 and sub-struts 515, and T is the thickness of this wire material.

37. The stent according to any of claims 32 through 36, wherein the shape of the basic cells 511 at the time of expansion of the stent is substantially triangular, substantially quadrangular, or substantially trapezoidal.

38. The stent according to any of claims 32 through 37, wherein the link parts 513 that mutually connect the band parts 512 formed by the series of a plurality of the basic cells 511 in the circumferential direction have a structure that can be extended and contracted in the longitudinal direction.

39. The stent according to any of claims 32 through 38, which satisfies the relationship 0.3 × L ≤ C ≤ 2L, where C is the length of the link parts 513 in the axial direction of the stent when the stent is not expanded, and L is the length in the longitudinal direction of the main struts 514 when the stent is not expanded.

40. The stent according to any of claims 32 through 39, wherein the main struts 514 and sub-struts 515 are made from one or more materials selected from among stainless steel, super-elastic metals, polymer materials with a bending elastic modulus of 1 GPa or greater, and biodegradable polymer materials.

41. The stent according to any of claims 32 through 40, wherein a tubular thin polymer film is formed on the outer circumferential surface of the stent.

42. The stent according to any of claims 32 through 41, which has an X-ray-impermeable marker that allows confirmation of the position of the stent in X-ray imaging.

43. A stent 604 which is formed as a substantially tubular body, and which can be expanded outward in the radial direction of the substantially tubular body, and in which a plurality of patterns 605 having a substantially polygonal shape surrounded by struts 604 constituting linear elements are arranged in the circumferential direction and axial direction,
said stent being **characterized in that** said polygonal shape patterns 605 have a linear peripheral length 606, 609 (original linear peripheral length), and these polygonal shape patterns 605 have three or more local folded portions 607, 610 per polygonal shape pattern 605, which are capable of elongating deformation so that the peripheral length 608 following expansion by pushing is extended to 1.3 to 2.0 times the original linear peripheral length 606, 609.

44. The stent according to claim 43, wherein the number of local folded parts 607, 610 per polygonal shape pattern 605 that are capable of elongating deformation is the same as the number of sides of said polygons.

45. A stent 603 which is formed as a substantially tubular body, and which can be expanded outward in the radial direction of the substantially tubular body, and in which a plurality of patterns 605 having a substantially polygonal shape surrounded by struts 604 constituting linear elements are arranged in the circumferential direction and axial direction, said stent being **characterized in that**:
said polygonal shape patterns 605 have a linear peripheral length 606, 609 (original linear peripheral length);
said polygonal shape patterns 605 have parts 607, 610 that are capable of elongating deformation so that the peripheral length of the polygonal shape patterns 605 can be expanded to 1.3 times to 2.0 times said linear peripheral length 606, 609 when expanded by pushing from the inside toward the outside; and
the total of the linear lengths of these local folded parts that are capable of elongating deformation 607, 610 in the side direction of said polygonal shapes is from 1/3 to 1 time the original linear peripheral length 606, 609 of said polygonal shape patterns 605.

46. The stent according to any of claims 43 through 45, **characterized in that** the original linear peripheral length 606, 609 of said polygonal shape patterns is in the range of 6.0 mm to 12.0 mm, and the area of the opening parts surrounded by the peripheral length 608 after being pushed open following expansion of the stent is in the range of 2.0 mm² to 9.0 mm².
